# EUROPEAN PATENT SPECIFICATION

(11) **EP 3 638 243 B1**
(45) Date of publication and mention of the grant of the patent: **07.08.2024**
(21) Application number: 18816685.4
(22) Date of filing: 13.06.2018
(51) Int. Cl.: A61K 31/519, A61K 45/06, C07K 14/495, A61K 38/17, A61P 35/00

(54) **METHODS FOR TREATING MYELOPROLIFERATIVE NEOPLASM-ASSOCIATED MYELOFIBROSIS AND ANEMIA**
VERFAHREN ZUR BEHANDLUNG VON MIT MYELOPROLIFERATIVEM NEOPLASMA ASSOZIIERTER MYELOFIBROSE UND ANÄMIE
MÉTHODES DE TRAITEMENT DE LA MYÉLOFIBROSE ASSOCIÉE À UN NÉOPLASME MYÉLOPROLIFÉRATIF ET DE L'ANÉMIE

(30) Priority: 14.06.2017 US 201762519725 P; 01.06.2018 US 201862679210 P
(43) Date of publication of application: 22.04.2020
(73) Proprietor: Celgene Corporation, Princeton, NJ 08543 (US)
(72) Inventor: LAADEM, Abderrahmane, Belle Mead NJ 08502 (US); GALE, Robert, Los Angeles CA 90077 (US)
(74) Representative: Jones Day
(86) International application number: PCT/US2018/037213
(87) International publication number: WO 2018/231905

(56) References cited:
- WO-A1-2016/183280
- WO-A1-2017/091706
- WO-A1-2018/022762
- US-A1- 2013 243 743
- US-A1- 2017 037 100
- ANONYMOUS: "A phase 2, multicenter, open-label study of the safety and efficacy of luspatercept in subjects with myeloproliferative neoplasm (MPN)-associated myelofibrosis and anemia with or without RBC transfusion dependence. | Journal of Clinical Oncology", 1 January 2018 (2018-01-01), XP055739720, Retrieved from the Internet <URL:https://ascopubs.org/doi/10.1200/JCO.2018.36.15_suppl.TPS7083> [retrieved on 20201014]
- ANONYMOUS: "History of Changes for Study: NCT03194542", 20 June 2017 (2017-06-20), XP055739721, Retrieved from the Internet <URL:https://clinicaltrials.gov/ct2/history/NCT03194542?V_1=View#StudyPageTop> [retrieved on 20201014]
- KEOHANE C ET AL: "Ruxolitinib for myelofibrosis", CLINICAL INVESTIGATION, FUTURE SCIENCE, UK, vol. 2, no. 10, 30 September 2012 (2012-09-30), pages 1023 - 1031, XP009519059, ISSN: 2041-6792, DOI: 10.4155/CLI.12.94
- ARCHANA AGARWAL ET AL: "Bone marrow fibrosis in primary myelofibrosis: pathogenic mechanisms and the role of TGF-[beta]", STEM CELL INVESTIGATION, 1 January 2016 (2016-01-01), China, pages 5 - 5, XP055738249, Retrieved from the Internet <URL:https://www.ncbi.nlm.nih.gov/pmc/articles/PMC4923632/pdf/sci-03-2016.02.03.pdf> DOI: 10.3978/j.issn.2306-9759.2016.02.03
- R. N. V. S. SURAGANI ET AL: "Modified activin receptor IIB ligand trap mitigates ineffective erythropoiesis and disease complications in murine ?-thalassemia", BLOOD, vol. 123, no. 25, 2 May 2014 (2014-05-02), US, pages 3864 - 3872, XP055423087, ISSN: 0006-4971, DOI: 10.1182/blood-2013-06-511238
- RAJASEKHAR N V S SURAGANI ET AL: "Transforming growth factor-&bgr; superfamily ligand trap ACE-536 corrects anemia by promoting late-stage erythropoiesis", NATURE MEDICINE, vol. 20, no. 4, 23 March 2014 (2014-03-23), New York, pages 408 - 414, XP055410209, ISSN: 1078-8956, DOI: 10.1038/nm.3512

## Description

### 1. FIELD

The patent generally relates to methods for treating anemia in a subject having myeloproliferative neoplasm-associated myelofibrosis comprising administering to the subject an ActRIIB signaling inhibitor.

### 2. BACKGROUND

Anemia is a decrease in number of red blood cells or less than the normal quantity of hemoglobin in the blood. Anemia can also be caused by decreased oxygen-binding ability of the hemoglobin. Anemia is the most common disorder of the blood. Anemia can be caused by ineffective erythropoiesis. Ineffective erythropoiesis is present if active erythropoiesis takes place but mature red blood cells fail to develop at the proper rate. Progenitor cells undergo apoptosis before the stage of mature red blood cells is reached.

Myeloproliferative neoplasm- (MPN-) associated myelofibrosis is an umbrella term that is used to describe a clonal myeloid neoplasm that can develop directly (primary myelofibrosis (also known as agnogenic myeloid metaplasia, chronic idiopathic myelofibrosis, myelosclerosis with myeloid metaplasia, and idiopathic myelofibrosis)) or evolve from other myeloid neoplasms (polycythemia vera and essential thrombocythemia), and are characterized by bone marrow dysfunction and fibrosis, extramedullary hematopoiesis, and an increased inflammatory state. MPN-associated myelofibrosis results in excessive fibrous tissue formation in the bone marrow, which can lead to severe anemia, fatigue, weakness, and enlarged spleen and liver. Current treatments for MPN-associated myelofibrosis include hydroxyurea, splenectomy, and allogeneic hematopoietic stem cell transplantation. However, hydroxyurea treatment may worsen anemia and there is concern of its mutagenic potential with long-term use, splenectomy has a short media duration of response, may shorten postsplenectomy survival, and has high postoperative morbidity, and allogeneic hematopoietic stem cell transplantation can lead to leukemic transformation (Sochacki et al., 2016, Therapeutic approaches in myelofibrosis and myelodysplastic/myeloproliferative overlap syndromes, Onco Targets Ther, 2016(9):2273-286). Thus, there remains a need for therapeutics for the treatment of MPN-associated myelofibrosis.

Two related type II receptors, ActRIIA and ActRIIB, have been identified as the type II receptors for activins (Mathews and Vale, 1991, Cell 65:973-982; Attisano et al., 1992, Cell 68: 97-108). Besides activins, ActRIIA and ActRIIB can biochemically interact with several other TGF-beta family proteins, including BMP7, Nodal, GDF8, and GDF11 (Yamashita et al., 1995, J. Cell Biol. 130:217-226; Lee and McPherron, 2001, Proc. Natl. Acad. Sci. 98:9306-9311; Yeo and Whitman, 2001, Mol. Cell 7: 949-957; Oh et al., 2002, Genes Dev. 16:2749-54). ALK4 is the primary type I receptor for activins, particularly for activin A, and ALK-7 may serve as a receptor for activins as well, particularly for activin B. ActRIIB signaling inhibitors have been described for treating ineffective erythropoiesis and myelodysplastic syndromes (see, e.g., International Patent Application Publication Nos. WO 2013/059347, WO 2011/020045, and WO 2016/090077).

Keohane C et al.: "Ruxolitinib for myelofibrosis", Clinical Investigation, Future Science, UK, vol. 2, no. 10, 30 September 2012 (2012-09-30), pages 1023-1031, is directed to the use of ruxolitinib in the treatment of myelofibrosis. Anemia and thrombocytopenia are identified as side-effects of the treatment.

A Agarwal et al.: "Bone marrow fibrosis in primary myelofibrosis: pathogenic mechanisms and the role of TGF-beta", Stem cell investigation, 1 January 2016 (2016-01-01), pages 5-5, indicates that inhibition of the TGF-beta pathway in conjunction with other therapies might prove useful in the reversal of bone marrow fibrosis in myeloproliferative neoplasm-associated myelofibrosis.

R. N. V. S. Suragani et al.: "Modified activin receptor IIB ligand trap mitigates ineffective erythropoiesis and disease complications in murine beta-thalassemia", BLOOD, vol. 123, no. 25, 2 May 2014 (2014-05-02), pages 3864-3872, discloses the use of modified activin receptor type IIB (ActRIIB) ligand trap (RAP-536) in a beta-thalassemia murine model. Treatment alleviated anemia by promoting differentiation of late-stage erythroid precursors and reducing hemolysis.

### 3. SUMMARY

Provided herein is an ActRIIB signaling inhibitor for use in a method for treating anemia in a subject having myeloproliferative neoplasm-associated myelofibrosis, wherein the subject has previously been treated with ruxolitinib, wherein the method comprises administering to the subject a pharmaceutically effective amount of the ActRIIB signaling inhibitor, wherein the ActRIIB signaling inhibitor is a fusion protein comprising: (a) a fragment of the extracellular domain of ActRIIB, wherein the fragment comprises the amino acid sequence of SEQ ID NO: 9; (b) a linker; and (c) an Fc of an IgG. Further aspects and embodiments of the invention are provided in the appended claims.

Reference to an ActRIIB signaling inhibitor in connection with the present invention should be understood as reference to the ActRIIB signaling inhibitor as defined above.

Methods of treatments of a human or animal body are not claimed. Should a method of treatment be mentioned in the context of the present invention, it should be understood as an ActRIIB signaling inhibitor for use in such a method.

In specific embodiments, the myeloproliferative neoplasm-associated myelofibrosis is primary myelofibrosis. In specific embodiments, the myeloproliferative neoplasm-associated myelofibrosis is polycythemia vera myelofibrosis. In more specific embodiments, the polycythemia vera myelofibrosis is associated with, or caused by, a *JAK2* mutation, *e.g*., a *JAK2* V617F mutation or *JAK2* exon 12 mutation, or a thrombopoietin receptor (MPL) mutation. In specific embodiments, the myeloproliferative neoplasm-associated myelofibrosis is post-polycythemia vera myelofibrosis. In more specific embodiments, the post-polycythemia vera myelofibrosis is associated with, or caused by, a *JAK2* mutation, *e.g*., a *JAK2* V617F mutation, or a thrombopoietin receptor (MPL) mutation. In specific embodiments, the myeloproliferative neoplasm-associated myelofibrosis is essential thrombocytopenia or post-essential thrombocythemia myelofibrosis. In more specific embodiments, the essential thrombocytopenia or post-essential thrombocythemia myelofibrosis is associated with, or caused by, a *JAK2* mutation, *e.g., a JAK2* V617F mutation, or a thrombopoietin receptor (MPL) mutation. In more specific embodiments, the essential thrombocytopenia or post-essential thrombocythemia myelofibrosis includes reticulin fibrosis or trichrome fibrosis. In specific embodiments, the subject is red blood cell transfusion-independent. In specific embodiments, the subject is red blood cell transfusion-independent if the subject has received no (0) red blood cell units during a period of time of 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to administration of the ActRIIB signaling inhibitor to the subject. In specific embodiments, the subject is red blood cell transfusion-dependent. In specific embodiments, the subject is red blood cell transfusion dependent if the subject has received an average red blood cell transfusion frequency of 2 to 4 red blood cell units per 28 days during a period of time of at least 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to administration of the ActRIIB signaling inhibitor to the subject. In specific embodiments, the pharmaceutically effective amount is from 0.3 mg/kg to 2.0 mg.kg, *e.g.,* 0.33 mg/kg, 0.45 mg/kg, 0.6 mg/kg, 0.8 mg/kg, 1 mg/kg, 1.33 mg/kg, 1.75 mg/kg, or 2.0 mg/kg. In specific embodiments, the pharmaceutically effective amount is 1 mg/kg. In specific embodiments, the ActRIIB signaling inhibitor is administered to the subject once every 21 days, once every 28 days, or once every 48 days. In specific embodiments, the ActRIIB signaling inhibitor is administered to the subject once every 21 days. In specific embodiments, the ActRIIB signaling inhibitor is administered to the subject intravenously or subcutaneously. In specific embodiments, the pharmaceutically effective amount is 1 mg/kg and the ActRIIB signaling inhibitor is administered to the subject subcutaneously once every 21 days. In specific embodiments, the ActRIIB signaling inhibitor is administered to the subject subcutaneously. In specific embodiments, the ActRIIB signaling inhibitor is a humanized fusion-protein consisting of the extracellular domain of ActRIIB comprising the amino acid sequence of SEQ ID NO:9 and the human IgG1 Fc domain. In specific embodiments, the ActRIIB signaling inhibitor is a fusion-protein comprising the above extracellular domain of ActRIIB and the human IgG1 Fc domain. Known ActRIIB signaling inhibitor is a polypeptide comprising an amino acid sequence selected from the group consisting of: (a) 90% identical to SEQ ID NO.4; (b) 95% identical to SEQ ID NO:4; (c) 98% identical to SEQ ID NO:4; (d) SEQ ID NO:4; (e) 90% identical to SEQ ID NO:7; (f) 95% identical to SEQ ID NO:7; (g) 98% identical to SEQ ID NO:7; (h) SEQ ID NO:7; (i) 90% identical to SEQ ID NO:8; (j) 95% identical to SEQ ID NO:8; (k) 98% identical to SEQ ID NO:8; (1) SEQ ID NO:8; (m) 90% identical to SEQ ID NO:11; (n) 95% identical to SEQ ID NO:11; (o) 98% identical to SEQ ID NO:1 1; and (p) SEQ ID NO:11. Known ActRIIB signaling inhibitor is a polypeptide comprising an amino acid sequence selected from the group consisting of: (a) 90% identical to SEQ ID NO:11; (b) 95% identical to SEQ ID NO:11; (c) 98% identical to SEQ ID NO:11; and (d) SEQ ID NO:11. In specific embodiments, the ActRIIB signaling inhibitor is a polypeptide comprising the amino acid sequence of SEQ ID NO:11. In specific embodiments, the ActRIIB signaling inhibitor is a polypeptide comprising an amino acid sequence consisting of the amino acid sequence of SEQ ID NO:11. In specific embodiments, the ActRIIB signaling inhibitor is a polypeptide consisting of the amino acid sequence set forth in SEQ ID NO:11. In specific embodiments, the subject is a human.

The treatment in accordance with the invention reduces or palliates one or more symptoms of said myeloproliferative neoplasm-associated myelofibrosis in said subject. In specific embodiments, the symptom is one or more of fatigue, night sweats, itchiness, abdominal discomfort, pain under the ribs on the left side, early satiety, or bone pain. In specific embodiments, the myeloproliferative neoplasm-associated myelofibrosis is primary myelofibrosis. In specific embodiments, the myeloproliferative neoplasm-associated myelofibrosis is polycythemia vera myelofibrosis. In more specific embodiments, the polycythemia vera myelofibrosis is associated with, or caused by, a *JAK2* mutation, *e*.*g*., a *JAK2* V617F mutation or *JAK2* exon 12 mutation, or a thrombopoietin receptor (MPL) mutation. In specific embodiments, the myeloproliferative neoplasm-associated myelofibrosis is post-polycythemia vera myelofibrosis. In more specific embodiments, the post-polycythemia vera myelofibrosis is associated with, or caused by, a *JAK2* mutation, *e.g.,* a *JAK2* V617F mutation, or a thrombopoietin receptor (MPL) mutation. In specific embodiments, the myeloproliferative neoplasm-associated myelofibrosis is essential thrombocytopenia or post-essential thrombocythemia myelofibrosis. In more specific embodiments, the essential thrombocytopenia or post-essential thrombocythemia myelofibrosis is associated with, or caused by, *a JAK2* mutation, *e.g.,* a *JAK2* V617F mutation, or a thrombopoietin receptor (MPL) mutation. In more specific embodiments, the essential thrombocytopenia or post-essential thrombocythemia myelofibrosis includes reticulin fibrosis or trichrome fibrosis. In specific embodiments, the subject is red blood cell transfusion-independent. In specific embodiments, the subject is red blood cell transfusion-independent if the subject has received no (0) red blood cell units during a period of time of 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to administration of the ActRIIB signaling inhibitor to the subject. In specific embodiments, the subject is red blood cell transfusion-dependent. In specific embodiments, the subject is red blood cell transfusion dependent if the subject has received an average red blood cell transfusion frequency of 2 to 4 red blood cell units per 28 days during a period of time of at least 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to administration of the ActRIIB signaling inhibitor to the subject. In specific embodiments, a pharmaceutically effective amount of the ActRIIB signaling inhibitor is from 0.3 mg/kg to 2.0 mg.kg, *e*.*g*., 0.33 mg/kg, 0.45 mg/kg, 0.6 mg/kg, 0.8 mg/kg, 1 mg/kg, 1.33 mg/kg, 1.75 mg/kg, or 2.0 mg/kg. In specific embodiments, the pharmaceutically effective amount is 1 mg/kg. In specific embodiments, the ActRIIB signaling inhibitor is administered to the subject once every 21 days, once every 28 days, or once every 48 days. In specific embodiments, the ActRIIB signaling inhibitor is administered to the subject once every 21 days. In specific embodiments, the ActRIIB signaling inhibitor is administered to the subject intravenously or subcutaneously. In specific embodiments, the ActRIIB signaling inhibitor is administered to the subject subcutaneously. In specific embodiments, the pharmaceutically effective amount is 1 mg/kg and the ActRIIB signaling inhibitor is administered to the subject subcutaneously once every 21 days. In specific embodiments, the ActRIIB signaling inhibitor is a humanized fusion-protein consisting of the extracellular domain of ActRIIB comprising the amino acid sequence of SEQ ID NO:9 and the human IgG1 Fc domain. In specific embodiments, the ActRIIB signaling inhibitor is a fusion-protein comprising the above extracellular domain of ActRIIB and the human IgG1 Fc domain. Known ActRIIB signaling inhibitor is a polypeptide comprising an amino acid sequence selected from the group consisting of: (a) 90% identical to SEQ ID NO:4; (b) 95% identical to SEQ ID NO:4; (c) 98% identical to SEQ ID NO:4; (d) SEQ ID NO:4; (e) 90% identical to SEQ ID NO:7; (f) 95% identical to SEQ ID NO:7; (g) 98% identical to SEQ ID NO:7; (h) SEQ ID NO:7; (i) 90% identical to SEQ ID NO:8; (j) 95% identical to SEQ ID NO:8; (k) 98% identical to SEQ ID NO:8; (1) SEQ ID NO:8; (m) 90% identical to SEQ ID NO:11; (n) 95% identical to SEQ ID NO:11; (o) 98% identical to SEQ ID NO:11; and (p) SEQ ID NO:11. Known ActRIIB signaling inhibitor is a polypeptide comprising an amino acid sequence selected from the group consisting of: (a) 90% identical to SEQ ID NO:11; (b) 95% identical to SEQ ID NO:11; (c) 98% identical to SEQ ID NO:11; and (d) SEQ ID NO:11. In specific embodiments, the ActRIIB signaling inhibitor is a polypeptide comprising the amino acid sequence of SEQ ID NO:11. In specific embodiments, the ActRIIB signaling inhibitor is a polypeptide comprising an amino acid sequence consisting of the amino acid sequence of SEQ ID NO:11. In specific embodiments, the ActRIIB signaling inhibitor is a polypeptide consisting of the amino acid sequence set forth in SEQ ID NO:11. In specific embodiments, the subject is a human.

In one embodiment the method comprises administering to the subject a dose of 0.33 mg/kg, 0.45 mg/kg, 0.6 mg/kg, 0.8 mg/kg, 1 mg/kg, 1.33 mg/kg, 1.75 mg/kg, or 2.0 mg/kg of a polypeptide comprising the amino acid sequence of SEQ ID NO:11, wherein the polypeptide is administered to the subject subcutaneously once every 21 days. In specific embodiments, the myeloproliferative neoplasm-associated myelofibrosis is polycythemia vera myelofibrosis. In more specific embodiments, the polycythemia vera myelofibrosis is associated with, or caused by, a *JAK2* mutation, *e.g.,* a *JAK2* V617F mutation or *JAK2* exon 12 mutation, or a thrombopoietin receptor (MPL) mutation. In specific embodiments, the myeloproliferative neoplasm-associated myelofibrosis is post-polycythemia vera myelofibrosis. In more specific embodiments, the post-polycythemia vera myelofibrosis is associated with, or caused by, a *JAK2* mutation, *e.g.,* a *JAK2* V617F mutation, or a thrombopoietin receptor (MPL) mutation. In specific embodiments, the myeloproliferative neoplasm-associated myelofibrosis is essential thrombocytopenia or post-essential thrombocythemia myelofibrosis. In more specific embodiments, the essential thrombocytopenia or post-essential thrombocythemia myelofibrosis is associated with, or caused by, a *JAK2* mutation, *e.g.,* a *JAK2* V617F mutation, or a thrombopoietin receptor (MPL) mutation. In more specific embodiments, the essential thrombocytopenia or post-essential thrombocythemia myelofibrosis includes reticulin fibrosis or trichrome fibrosis. In specific embodiments, the subject is red blood cell transfusion-independent. In specific embodiments, the subject is red blood cell transfusion-independent if the subject has received no (0) red blood cell units during a period of time 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to administration of the ActRIIB signaling inhibitor to the subject. In specific embodiments, the subject is red blood cell transfusion-dependent. In specific embodiments, the subject is red blood cell transfusion dependent if the subject has received an average red blood cell transfusion frequency of 2 to 4 red blood cell units per 28 days during a period of time of at least 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to administration of the ActRIIB signaling inhibitor to the subject. In specific embodiments, the subject is a human.

In one embodiment, the method comprises administering to the subject a dose of 0.33 mg/kg, 0.45 mg/kg, 0.6 mg/kg, 0.8 mg/kg, 1 mg/kg, 1.33 mg/kg, 1.75 mg/kg, or 2.0 mg/kg of a polypeptide comprising an amino acid sequence consisting of the amino acid sequence of SEQ ID NO:11, wherein the polypeptide is administered to the subject subcutaneously once every 21 days. In specific embodiments, the dose is 1 mg/kg. In specific embodiments, the myeloproliferative neoplasm-associated myelofibrosis is polycythemia vera myelofibrosis. In more specific embodiments, the polycythemia vera myelofibrosis is associated with, or caused by, a *JAK2* mutation, *e.g.,* a *JAK2* V617F mutation or *JAK2* exon 12 mutation, or a thrombopoietin receptor (MPL) mutation. In specific embodiments, the myeloproliferative neoplasm-associated myelofibrosis is post-polycythemia vera myelofibrosis. In more specific embodiments, the psot-polycythemia vera myelofibrosis is associated with, or caused by, a *JAK2* mutation, *e.g.,* a *JAK2* V617F mutation, or a thrombopoietin receptor (MPL) mutation. In specific embodiments, the myeloproliferative neoplasm-associated myelofibrosis is essential thrombocytopenia or post-essential thrombocythemia myelofibrosis. In more specific embodiments, the essential thrombocytopenia or post-essential thrombocythemia myelofibrosis is associated with, or caused by, *a JAK2* mutation, *e.g.,* a *JAK2* V617F mutation, or a thrombopoietin receptor (MPL) mutation. In more specific embodiments, the essential thrombocytopenia or post-essential thrombocythemia myelofibrosis includes reticulin fibrosis or trichrome fibrosis. In specific embodiments, the subject is red blood cell transfusion-independent. In specific embodiments, the subject is red blood cell transfusion-independent if the subject has received no (0) red blood cell units during a period of time 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to administration of the ActRIIB signaling inhibitor to the subject. In specific embodiments, the subject is red blood cell transfusion-dependent. In specific embodiments, the subject is red blood cell transfusion dependent if the subject has received an average red blood cell transfusion frequency of 2 to 4 red blood cell units per 28 days during a period of time of at least 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to administration of the ActRIIB signaling inhibitor to the subject. In specific embodiments, the subject is a human.

In one embodiment, the method comprises administering to the subject a dose of 0.33 mg/kg, 0.45 mg/kg, 0.6 mg/kg, 0.8 mg/kg, 1 mg/kg, 1.33 mg/kg, 1.75 mg/kg, or 2.0 mg/kg of a polypeptide consisting of the amino acid sequence of SEQ ID NO:11, wherein the polypeptide is administered to the subject subcutaneously once every 21 days. In specific embodiments, the dose is 1 mg/kg. In specific embodiments, the myeloproliferative neoplasm-associated myelofibrosis is polycythemia vera myelofibrosis. In more specific embodiments, the polycythemia vera myelofibrosis is associated with, or caused by, a *JAK2* mutation, *e.g.,* a *JAK2* V617F mutation or *JAK2* exon 12 mutation, or a thrombopoietin receptor (MPL) mutation. In specific embodiments, the myeloproliferative neoplasm-associated myelofibrosis is post-polycythemia vera myelofibrosis. In more specific embodiments, the post-polycythemia vera myelofibrosis is associated with, or caused by, a *JAK2* mutation, *e.g.,* a *JAK2* V617F mutation, or a thrombopoietin receptor (MPL) mutation. In specific embodiments, the myeloproliferative neoplasm-associated myelofibrosis is essential thrombocytopenia or post-essential thrombocythemia myelofibrosis. In more specific embodiments, the essential thrombocytopenia or post-essential thrombocythemia myelofibrosis is associated with, or caused by, a *JAK2* mutation, *e.g.,* a *JAK2* V617F mutation, or a thrombopoietin receptor (MPL) mutation. In more specific embodiments, the essential thrombocytopenia or post-essential thrombocythemia myelofibrosis includes reticulin fibrosis or trichrome fibrosis. In specific embodiments, the subject is red blood cell transfusion-independent. In specific embodiments, the subject is red blood cell transfusion-independent if the subject has received no (0) red blood cell units during a period of time 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to administration of the ActRIIB signaling inhibitor to the subject. In specific embodiments, the subject is red blood cell transfusion-dependent. In specific embodiments, the subject is red blood cell transfusion dependent if the subject has received an average red blood cell transfusion frequency of 2 to 4 red blood cell units per 28 days during a period of time of at least 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to administration of the ActRIIB signaling inhibitor to the subject. In specific embodiments, the subject is a human.

### 4. TERMINOLOGY AND ABBREVIATIONS

As used herein, "ActRII" refers to activin receptor type II. As used herein, "ActRIIA" refers to activin receptor type IIA. See, for example, Mathews and Vale, 1991, Cell 65:973-982. GenBank^{™} accession number NM_001278579.1 provides an exemplary human ActRIIA nucleic acid sequence. GenBank^{™} accession number NP_001265508.1 provides an exemplary human ActRIIA amino acid sequence. As used herein, "ActRIIB" refers to activin receptor type IIB. See, for example, Attisano et al., 1992, Cell 68: 97-108. GenBank^{™} accession number NM 001106.3 provides an exemplary human ActRIIB nucleic acid sequence. GenBank^{™} accession number NP_001097.2 provides an exemplary human ActRIIB amino acid sequence.

As used herein, "Hb" and "Hgb" refer to hemoglobin.

As used herein, "mg/kg", in the context of a dose of an ActRIIB signaling inhibitor, refers to milligrams of the ActRIIB signaling inhibitor per kilogram of the weight of the subject to whom the ActRIIB signaling inhibitor is to be administered.

As used herein, "MF" refers to myelofibrosis.

As used herein "PMF" refers to primary myelofibrosis.

As used herein, "post-PV MF" refers to post-polycythemia vera myelofibrosis.

As used herein, "post-ET MF" refers to post-essential thrombocythemia myelofibrosis.

As used herein, "MPN" refers to myeloproliferative neoplasm.

As used herein, "RBC" refers to red blood cell.

As used herein, "RBC-TI" refers to red blood cell transfusion independent.

### 4.1 BRIEF DESCRIPTION OF THE DRAWINGS

FIG. 1: Mechanism of Action of Luspatercept.
FIG. 2: Study Design.

### 5. DETAILED DESCRIPTION

### 5.1 OVERVIEW

Provided herein is an ActRIIB signaling inhibitor for use in a method for treating anemia in a subject having myeloproliferative neoplasm-associated myelofibrosis, wherein the subject has previously been treated with ruxolitinib, wherein the method comprises administering to the subject a pharmaceutically effective amount of the ActRIIB signaling inhibitor, wherein the ActRIIB signaling inhibitor is a fusion protein comprising: (a) a fragment of the extracellular domain of ActRIIB, wherein the fragment comprises the amino acid sequence of SEQ ID NO: 9; (b) a linker; and (c) an Fc of an IgG. Further aspects and embodiments of the invention are provided in the appended claims.

In specific embodiments, the myeloproliferative neoplasm-associated myelofibrosis is primary myelofibrosis. In specific embodiments, the myeloproliferative neoplasm-associated myelofibrosis is polycythemia vera myelofibrosis. In more specific embodiments, the polycythemia vera myelofibrosis is associated with, or caused by, a *JAK2* mutation, *e.g.,* a *JAK2* V617F mutation or *JAK2* exon 12 mutation, or a thrombopoietin receptor (MPL) mutation. In specific embodiments, the myeloproliferative neoplasm-associated myelofibrosis is post-polycythemia vera myelofibrosis. In more specific embodiments, the post-polycythemia vera myelofibrosis is associated with, or caused by, *a JAK2* mutation, *e.g*., a *JAK2* V617F mutation, or a thrombopoietin receptor (MPL) mutation. In specific embodiments, the myeloproliferative neoplasm-associated myelofibrosis is essential thrombocytopenia or post-essential thrombocythemia myelofibrosis. In more specific embodiments, the essential thrombocytopenia or post-essential thrombocythemia myelofibrosis is associated with, or caused by, a *JAK2* mutation, *e.g.,* a *JAK2* V617F mutation, or a thrombopoietin receptor (MPL) mutation. In more specific embodiments, the essential thrombocytopenia or post-essential thrombocythemia myelofibrosis includes reticulin fibrosis or trichrome fibrosis. In specific embodiments, the subject is red blood cell transfusion-independent. In specific embodiments, the subject is red blood cell transfusion-independent if the subject has received no (0) red blood cell units during a period of time of 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to administration of the ActRIIB signaling inhibitor to the subject. In specific embodiments, the subject is red blood cell transfusion-dependent. In specific embodiments, the subject is red blood cell transfusion dependent if the subject has received an average red blood cell transfusion frequency of 2 to 4 red blood cell units per 28 days during a period of time of at least 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to administration of the ActRIIB signaling inhibitor to the subject. In specific embodiments, the pharmaceutically effective amount is from 0.3 mg/kg to 2.0 mg.kg, *e*.*g*., 0.33 mg/kg, 0.45 mg/kg, 0.6 mg/kg, 0.8 mg/kg, 1 mg/kg, 1.33 mg/kg, 1.75 mg/kg, or 2.0 mg/kg. In specific embodiments, the ActRIIB signaling inhibitor is administered to the subject once every 21 days, once every 28 days, or once every 48 days. In specific embodiments, the ActRIIB signaling inhibitor is administered to the subject once every 21 days. In specific embodiments, the ActRIIB signaling inhibitor is administered to the subject intravenously or subcutaneously. In specific embodiments, the ActRIIB signaling inhibitor is administered to the subject subcutaneously. In specific embodiments, the ActRIIB signaling inhibitor is a humanized fusion-protein consisting of the extracellular domain of ActRIIB comprising the amino acid sequence of SEQ ID NO:9 and the human IgG1 Fc domain. Known ActRIIB signaling inhibitor is a polypeptide comprising an amino acid sequence selected from the group consisting of: (a) 90% identical to SEQ ID NO:4; (b) 95% identical to SEQ ID NO:4; (c) 98% identical to SEQ ID NO:4; (d) SEQ ID NO:4; (e) 90% identical to SEQ ID NO:7; (f) 95% identical to SEQ ID NO:7; (g) 98% identical to SEQ ID NO:7; (h) SEQ ID NO:7; (i) 90% identical to SEQ ID NO:8; (j) 95% identical to SEQ ID NO:8; (k) 98% identical to SEQ ID NO:8; (1) SEQ ID NO:8; (m) 90% identical to SEQ ID NO:11; (n) 95% identical to SEQ ID NO:11; (o) 98% identical to SEQ ID NO:11; and (p) SEQ ID NO:11. In specific embodiments, the ActRIIB signaling inhibitor is a polypeptide comprising an amino acid sequence selected from the group consisting of: (a) 90% identical to SEQ ID NO:11; (b) 95% identical to SEQ ID NO:11; (c) 98% identical to SEQ ID NO:11; and (d) SEQ ID NO:11. In specific embodiments, the ActRIIB signaling inhibitor is a polypeptide comprising the amino acid sequence of SEQ ID NO:1 1. In specific embodiments, the ActRIIB signaling inhibitor is a polypeptide comprising an amino acid sequence consisting of the amino acid sequence of SEQ ID NO:11. In specific embodiments, the ActRIIB signaling inhibitor is a polypeptide consisting of the amino acid sequence set forth in SEQ ID NO:11. In specific embodiments, the subject is a human.

The treatment in accordance with the invention reduces or palliates one or more symptoms of said myeloproliferative neoplasm-associated myelofibrosis in said subject. In specific embodiments, the symptom is one or more of fatigue, night sweats, itchiness, abdominal discomfort, pain under the ribs on the left side, early satiety, or bone pain. In specific embodiments, the myeloproliferative neoplasm-associated myelofibrosis is primary myelofibrosis. In specific embodiments, the myeloproliferative neoplasm-associated myelofibrosis is polycythemia vera myelofibrosis. In more specific embodiments, the polycythemia vera myelofibrosis is associated with, or caused by, a *JAK2* mutation, *e.g*., a *JAK2* V617F mutation or *JAK2* exon 12 mutation, or a thrombopoietin receptor (MPL) mutation. In specific embodiments, the myeloproliferative neoplasm-associated myelofibrosis is post-polycythemia vera myelofibrosis. In more specific embodiments, the post-polycythemia vera myelofibrosis is associated with, or caused by, a *JAK2* mutation, *e.g.,* a *JAK2* V617F mutation, or a thrombopoietin receptor (MPL) mutation. In specific embodiments, the myeloproliferative neoplasm-associated myelofibrosis is essential thrombocytopenia or post-essential thrombocythemia myelofibrosis. In more specific embodiments, the essential thrombocytopenia or post-essential thrombocythemia myelofibrosis is associated with, or caused by, *a JAK2* mutation, *e.g.,* a *JAK2* V617F mutation, or a thrombopoietin receptor (MPL) mutation. In more specific embodiments, the essential thrombocytopenia or post-essential thrombocythemia myelofibrosis includes reticulin fibrosis or trichrome fibrosis. In specific embodiments, the subject is red blood cell transfusion-independent. In specific embodiments, the subject is red blood cell transfusion-independent if the subject has received no (0) red blood cell units during a period of time of 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to administration of the ActRIIB signaling inhibitor to the subject. In specific embodiments, the subject is red blood cell transfusion-dependent. In specific embodiments, the subject is red blood cell transfusion dependent if the subject has received an average red blood cell transfusion frequency of 2 to 4 red blood cell units per 28 days during a period of time of at least 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to administration of the ActRIIB signaling inhibitor to the subject. In specific embodiments, the pharmaceutically effective amount is from 0.3 mg/kg to 2.0 mg.kg, *e.g.,* 0.33 mg/kg, 0.45 mg/kg, 0.6 mg/kg, 0.8 mg/kg, 1 mg/kg, 1.33 mg/kg, 1.75 mg/kg, or 2.0 mg/kg. In specific embodiments, the ActRIIB signaling inhibitor is administered to the subject once every 21 days, once every 28 days, or once every 48 days. In specific embodiments, the ActRIIB signaling inhibitor is administered to the subject once every 21 days. In specific embodiments, the ActRIIB signaling inhibitor is administered to the subject intravenously or subcutaneously. In specific embodiments, the ActRIIB signaling inhibitor is administered to the subject subcutaneously. In specific embodiments, the ActRIIB signaling inhibitor is a humanized fusion-protein consisting of the extracellular domain of ActRIIB comprising the amino acid sequence of SEQ ID NO:9 and the human IgG1 Fc domain. Known ActRIIB signaling inhibitor is a polypeptide comprising an amino acid sequence selected from the group consisting of: (a) 90% identical to SEQ ID NO:4; (b) 95% identical to SEQ ID NO:4; (c) 98% identical to SEQ ID NO:4, (d) SEQ ID NO:4; (e) 90% identical to SEQ ID NO:7; (f) 95% identical to SEQ ID NO:7; (g) 98% identical to SEQ ID NO:7; (h) SEQ ID NO:7; (i) 90% identical to SEQ ID NO:8; (j) 95% identical to SEQ ID NO:8; (k) 98% identical to SEQ ID NO:8; (1) SEQ ID NO:8; (m) 90% identical to SEQ ID NO:11; (n) 95% identical to SEQ ID NO:11; (o) 98% identical to SEQ ID NO:11; and (p) SEQ ID NO:11. Known ActRIIB signaling inhibitor is a polypeptide comprising an amino acid sequence selected from the group consisting of: (a) 90% identical to SEQ ID NO:11; (b) 95% identical to SEQ ID NO:11; (c) 98% identical to SEQ ID NO:11; and (d) SEQ ID NO:11. In specific embodiments, the ActRIIB signaling inhibitor is a polypeptide comprising the amino acid sequence of SEQ ID NO:11. In specific embodiments, the ActRIIB signaling inhibitor is a polypeptide comprising an amino acid sequence consisting of the amino acid sequence of SEQ ID NO:11. In specific embodiments, the ActRIIB signaling inhibitor is a polypeptide consisting of the amino acid sequence set forth in SEQ ID NO:11. In specific embodiments, the subject is a human.

In one embodiment the method comprises administering to the subject a dose of 0.33 mg/kg, 0.45 mg/kg, 0.6 mg/kg, 0.8 mg/kg, 1 mg/kg, 1.33 mg/kg, 1.75 mg/kg, or 2.0 mg/kg of a polypeptide consisting of the amino acid sequence of SEQ ID NO:11, wherein the polypeptide is administered to the subject subcutaneously once every 21 days. In specific embodiments, the myeloproliferative neoplasm-associated myelofibrosis is polycythemia vera myelofibrosis. In more specific embodiments, the polycythemia vera myelofibrosis is associated with, or caused by, a *JAK2* mutation, *e.g*., a *JAK2* V617F mutation or *JAK2* exon 12 mutation, or a thrombopoietin receptor (MPL) mutation. In specific embodiments, the myeloproliferative neoplasm-associated myelofibrosis is post-polycythemia vera myelofibrosis. In more specific embodiments, the post-polycythemia vera myelofibrosis is associated with, or caused by, a *JAK2* mutation, *e.g.,* a *JAK2* V617F mutation, or a thrombopoietin receptor (MPL) mutation. In specific embodiments, the myeloproliferative neoplasm-associated myelofibrosis is essential thrombocytopenia or post-essential thrombocythemia myelofibrosis. In more specific embodiments, the essential thrombocytopenia or post-essential thrombocythemia myelofibrosis is associated with, or caused by, a *JAK2* mutation, *e.g.,* a *JAK2* V617F mutation, or a thrombopoietin receptor (MPL) mutation. In more specific embodiments, the essential thrombocytopenia or post-essential thrombocythemia myelofibrosis includes reticulin fibrosis or trichrome fibrosis. In specific embodiments, the subject is red blood cell transfusion-independent. In specific embodiments, the subject is red blood cell transfusion-independent if the subject has received no (0) red blood cell units during a period of time 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to administration of the ActRIIB signaling inhibitor to the subject. In specific embodiments, the subject is red blood cell transfusion-dependent. In specific embodiments, the subject is red blood cell transfusion dependent if the subject has received an average red blood cell transfusion frequency of 2 to 4 red blood cell units per days during a period of time of at least 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to administration of the ActRIIB signaling inhibitor to the subject. In specific embodiments, the subject is a human.

### 5.2 METHODS OF TREATMENT

Disclosed herein is a method for treating myeloproliferative neoplasm-associated myelofibrosis in a subject in need thereof, wherein the method comprises administering to the subject a pharmaceutically effective amount of an ActRIIB signaling inhibitor. In specific cases the myeloproliferative neoplasm-associated myelofibrosis is primary myelofibrosis. In specific cases the myeloproliferative neoplasm-associated myelofibrosis is polycythemia vera myelofibrosis. In more specific cases the polycythemia vera myelofibrosis is associated with, or caused by, a *JAK2* mutation, *e.g*., a *JAK2* V617F mutation or *JAK2* exon 12 mutation, or a thrombopoietin receptor (MPL) mutation. In specific cases the myeloproliferative neoplasm-associated myelofibrosis is post-polycythemia vera myelofibrosis. In more specific cases the post-polycythemia vera myelofibrosis is associated with, or caused by, a *JAK2* mutation, *e.g.,* a *JAK2* V617F mutation, or a thrombopoietin receptor (MPL) mutation. In specific cases, the myeloproliferative neoplasm-associated myelofibrosis is essential thrombocytopenia or post-essential thrombocythemia myelofibrosis. In more specific cases the essential thrombocytopenia or post-essential thrombocythemia myelofibrosis is associated with, or caused by, a *JAK2* mutation, *e.g.,* a *JAK2* V617F mutation, or a thrombopoietin receptor (MPL) mutation. In more specific cases the essential thrombocytopenia or post-essential thrombocythemia myelofibrosis includes reticulin fibrosis or trichrome fibrosis.

In specific cases the subject is a subject described in Section 5.3. In specific cases the subject is a human. In specific cases the subject has anemia. In specific cases the subject is red blood cell transfusion-independent. In specific cases the subject is red blood cell transfusion-independent if the subject has received no (0) red blood cell units during a period of time of 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to administration of the ActRIIB signaling inhibitor to the subject. In specific cases the subject is red blood cell transfusion-dependent. In specific cases the subject is red blood cell transfusion dependent if the subject has received an average red blood cell transfusion frequency of 2 to 4 red blood cell units per 28 days during a period of time of at least 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to administration of the ActRIIB signaling inhibitor to the subject.

In specific cases the ActRIIB signaling inhibitor is administered to the subject as part of a composition. In specific cases the composition is a composition described in Section 5.5.

In specific cases, the pharmaceutically effective dose is a dose described in Section 5.6. In specific cases, the pharmaceutically effective amount is from 0.3 mg/kg to 2.0 mg.kg, *e.g.,* 0.33 mg/kg, 0.45 mg/kg, 0.6 mg/kg, 0.8 mg/kg, 1 mg/kg, 1.33 mg/kg, 1.75 mg/kg, or 2.0 mg/kg of the ActRIIB signaling inhibitor. In specific cases, the ActRIIB signaling inhibitor is administered to the subject at a frequency described in Section 5.6. In specific cases the ActRIIB signaling inhibitor is administered to the subject once every 21 days, once every 28 days, or once every 48 days. In preferred cases the ActRIIB signaling inhibitor is administered to the subject once every 21 days.

In specific cases, the ActRIIB signaling inhibitor is administered to the subject via a route of administration described in Section 5.6. In specific cases the ActRIIB signaling inhibitor is administered to the subject intravenously or subcutaneously. In preferred cases the ActRIIB signaling inhibitor is administered to the subject subcutaneously.

In specific cases the ActRIIB signaling inhibitor is an ActRIIB signaling inhibitor described in Section 5.4. In specific cases the ActRIIB signaling inhibitor is a polypeptide comprising the amino acid sequence of SEQ ID NO:11. In specific cases the ActRIIB signaling inhibitor is a polypeptide comprising an amino acid sequence consisting of the amino acid sequence of SEQ ID NO:11. In specific cases the ActRIIB signaling inhibitor is a polypeptide consisting of the amino acid sequence set forth in SEQ ID NO:11. In a preferred case, the ActRIIB signaling inhibitor is luspatercept. In specific cases, the subject is a human.

Also disclosed herein is a method for treating myeloproliferative neoplasm-associated myelofibrosis in a subject in need thereof, wherein the method comprises administering to the subject a pharmaceutically effective amount of an ActRIIB signaling inhibitor, and wherein said treating reduces or palliates one or more symptoms of said myeloproliferative neoplasm-associated myelofibrosis in said subject. In specific cases the symptom is one or more of fatigue, night sweats, itchiness, abdominal discomfort, pain under the ribs on the left side, early satiety, or bone pain. In specific cases the myeloproliferative neoplasm-associated myelofibrosis is primary myelofibrosis. In specific cases the myeloproliferative neoplasm-associated myelofibrosis is polycythemia vera myelofibrosis. In more specific cases the polycythemia vera myelofibrosis is associated with, or caused by, a *JAK2* mutation, *e*.*g*., a *JAK2* V617F mutation or *JAK2* exon 12 mutation, or a thrombopoietin receptor (MPL) mutation. In specific cases, the myeloproliferative neoplasm-associated myelofibrosis is post-polycythemia vera myelofibrosis. In more specific cases the post-polycythemia vera myelofibrosis is associated with, or caused by, a *JAK2* mutation, *e*.*g*., a *JAK2* V617F mutation, or a thrombopoietin receptor (MPL) mutation. In specific cases the myeloproliferative neoplasm-associated myelofibrosis is essential thrombocytopenia or post-essential thrombocythemia myelofibrosis. In more specific cases, the essential thrombocytopenia or post-essential thrombocythemia myelofibrosis is associated with, or caused by, a *JAK2* mutation, *e.g.,* a *JAK2* V617F mutation, or a thrombopoietin receptor (MPL) mutation. In more specific cases, the essential thrombocytopenia or post-essential thrombocythemia myelofibrosis includes reticulin fibrosis or trichrome fibrosis.

In specific cases the subject is a subject described in Section 5.3. In specific cases, the subject is a human. In specific cases, the subject has anemia. In specific cases the subject is red blood cell transfusion-independent. In specific cases the subject is red blood cell transfusion-independent if the subject has received no (0) red blood cell units during a period of time of 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to administration of the ActRIIB signaling inhibitor to the subject. In specific cases the subject is red blood cell transfusion-dependent. In specific cases the subject is red blood cell transfusion dependent if the subject has received an average red blood cell transfusion frequency of 2 to 4 red blood cell units per 28 days during a period of time of at least 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to administration of the ActRIIB signaling inhibitor to the subject.

In specific cases the ActRIIB signaling inhibitor is administered to the subject as part of a composition. In specific cases the composition is a composition described in Section 5.5.

In specific cases the pharmaceutically effective dose is a dose described in Section 5.6. In specific cases the pharmaceutically effective amount is from 0.3 mg/kg to 2.0 mg.kg, *e.g.,* 0.33 mg/kg, 0.45 mg/kg, 0.6 mg/kg, 0.8 mg/kg, 1 mg/kg, 1.33 mg/kg, 1.75 mg/kg, or 2.0 mg/kg of the ActRIIB signaling inhibitor. In specific cases the ActRIIB signaling inhibitor is administered to the subject at a frequency described in Section 56. In specific cases the ActRIIB signaling inhibitor is administered to the subject once every 21 days, once every 28 days, or once every 48 days. In preferred cases the ActRIIB signaling inhibitor is administered to the subject once every 21 days.

In specific cases the ActRIIB signaling inhibitor is administered to the subject via a route of administration described in Section 5.6. In specific embodiments, the ActRIIB signaling inhibitor is administered to the subject intravenously or subcutaneously. In preferred cases the ActRIIB signaling inhibitor is administered to the subject subcutaneously.

In specific cases the ActRIIB signaling inhibitor is an ActRIIB signaling inhibitor described in Section 5.4. In specific cases the ActRIIB signaling inhibitor is a polypeptide comprising the amino acid sequence of SEQ ID NO:11. In specific cases the ActRIIB signaling inhibitor is a polypeptide comprising an amino acid sequence consisting of the amino acid sequence of SEQ ID NO:11. In specific cases the ActRIIB signaling inhibitor is a polypeptide consisting of the amino acid sequence set forth in SEQ ID NO:11. In a preferred case, the ActRIIB signaling inhibitor is luspatercept. In specific cases the subject is a human.

Provided herein is a method for treating anemia in a subject in need thereof, comprising administering to the subject a pharmaceutically effective amount of an ActRIIB signaling inhibitor, wherein the subject has myeloproliferative neoplasm-associated myelofibrosis as defined in the appended claims. In specific embodiments, the myeloproliferative neoplasm-associated myelofibrosis is primary myelofibrosis. In specific embodiments, the myeloproliferative neoplasm-associated myelofibrosis is polycythemia vera myelofibrosis. In more specific embodiments, the polycythemia vera myelofibrosis is associated with, or caused by, a *JAK2* mutation, *e.g*., a *JAK2* V617F mutation or *JAK2* exon 12 mutation, or a thrombopoietin receptor (MPL) mutation. In specific embodiments, the myeloproliferative neoplasm-associated myelofibrosis is post-polycythemia vera myelofibrosis. In more specific embodiments, the post-polycythemia vera myelofibrosis is associated with, or caused by, a *JAK2* mutation, *e*.*g*., a *JAK2* V617F mutation, or a thrombopoietin receptor (MPL) mutation. In specific embodiments, the myeloproliferative neoplasm-associated myelofibrosis is essential thrombocytopenia or post-essential thrombocythemia myelofibrosis. In more specific embodiments, the essential thrombocytopenia or post-essential thrombocythemia myelofibrosis is associated with, or caused by, a *JAK2* mutation, *e.g.,* a *JAK2* V617F mutation, or a thrombopoietin receptor (MPL) mutation. In more specific embodiments, the essential thrombocytopenia or post-essential thrombocythemia myelofibrosis includes reticulin fibrosis or trichrome fibrosis.

In specific embodiments, the subject is a subject described in Section 5.3. In specific embodiments, the subject is a human. In specific embodiments, the subject is red blood cell transfusion-independent. In specific embodiments, the subject is red blood cell transfusion-independent if the subject has received no (0) red blood cell units during a period of time of 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to administration of the ActRIIB signaling inhibitor to the subject. In specific embodiments, the subject is red blood cell transfusion-dependent. In specific embodiments, the subject is red blood cell transfusion dependent if the subject has received an average red blood cell transfusion frequency of 2 to 4 red blood cell units per 28 days during a period of time of at least 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to administration of the ActRIIB signaling inhibitor to the subject.

In specific embodiments, the ActRIIB signaling inhibitor is administered to the subject as part of a composition. In specific embodiments, the composition is a composition described in Section 5.5.

In specific embodiments, the pharmaceutically effective dose is a dose described in Section 5.6. In specific embodiments, the pharmaceutically effective amount is from 0.3 mg/kg to 2.0 mg.kg, *e.g.,* 0.33 mg/kg, 0.45 mg/kg, 0.6 mg/kg, 0.8 mg/kg, 1 mg/kg, 1.33 mg/kg, 1.75 mg/kg, or 2.0 mg/kg of the ActRIIB signaling inhibitor. In specific embodiments, the ActRIIB signaling inhibitor is administered to the subject at a frequency described in Section 5.6. In specific embodiments, the ActRIIB signaling inhibitor is administered to the subject once every 21 days, once every 28 days, or once every 48 days. In preferred embodiments, the ActRIIB signaling inhibitor is administered to the subject once every 21 days.

In specific embodiments, the ActRIIB signaling inhibitor is administered to the subject via a route of administration described in Section 5.6. In specific embodiments, the ActRIIB signaling inhibitor is administered to the subject intravenously or subcutaneously. In preferred embodiments, the ActRIIB signaling inhibitor is administered to the subject subcutaneously.

In specific embodiments, the ActRIIB signaling inhibitor is an ActRIIB signaling inhibitor described in Section 5.4. In specific embodiments, the ActRIIB signaling inhibitor is a polypeptide comprising the amino acid sequence of SEQ ID NO:11. In specific embodiments, the ActRIIB signaling inhibitor is a polypeptide comprising an amino acid sequence consisting of the amino acid sequence of SEQ ID NO:11. In specific embodiments, the ActRIIB signaling inhibitor is a polypeptide consisting of the amino acid sequence set forth in SEQ ID NO:11. In a preferred embodiment, the ActRIIB signaling inhibitor is luspatercept. In specific embodiments, the subject is a human.

In one embodiment, the method comprises administering to the subject a dose of 0.33 mg/kg, 0.45 mg/kg, 0.6 mg/kg, 0.8 mg/kg, 1 mg/kg, 1.33 mg/kg, 1.75 mg/kg, or 2.0 mg/kg of a polypeptide comprising the amino acid sequence of SEQ ID NO:11, wherein the polypeptide is administered to the subject subcutaneously once every 21 days. In specific embodiments, the myeloproliferative neoplasm-associated myelofibrosis is primary myelofibrosis. In specific embodiments, the myeloproliferative neoplasm-associated myelofibrosis is polycythemia vera myelofibrosis. In more specific embodiments, the polycythemia vera myelofibrosis is associated with, or caused by, a *JAK2* mutation, *e.g*., a *JAK2* V617F mutation or *JAK2* exon 12 mutation, or a thrombopoietin receptor (MPL) mutation. In specific embodiments, the myeloproliferative neoplasm-associated myelofibrosis is post-polycythemia vera myelofibrosis. In more specific embodiments, the post-polycythemia vera myelofibrosis is associated with, or caused by, a *JAK2* mutation, *e.g.,* a *JAK2* V617F mutation, or a thrombopoietin receptor (MPL) mutation. In specific embodiments, the myeloproliferative neoplasm-associated myelofibrosis is essential thrombocytopenia or post-essential thrombocythemia myelofibrosis. In more specific embodiments, the essential thrombocytopenia or post-essential thrombocythemia myelofibrosis is associated with, or caused by, *a JAK2* mutation, *e.g.,* a *JAK2* V617F mutation, or a thrombopoietin receptor (MPL) mutation. In more specific embodiments, the essential thrombocytopenia or post-essential thrombocythemia myelofibrosis includes reticulin fibrosis or trichrome fibrosis.

In specific embodiments, the subject is a subject described in Section 5.3. In specific embodiments, the subject is red blood cell transfusion-independent. In specific embodiments, the subject is red blood cell transfusion-independent if the subject has received no (0) red blood cell units during a period of time of 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to administration of the ActRIIB signaling inhibitor to the subject. In specific embodiments, the subject is red blood cell transfusion-dependent. In specific embodiments, the subject is red blood cell transfusion dependent if the subject has received an average red blood cell transfusion frequency of 2 to 4 red blood cell units per 28 days during a period of time of at least 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to administration of the ActRIIB signaling inhibitor to the subject.

In specific embodiments, the ActRIIB signaling inhibitor is administered to the subject as part of a composition. In specific embodiments, the composition is a composition described in Section 5.5.

In one embodiment, the method comprises administering to the subject a dose of 0.33 mg/kg, 0.45 mg/kg, 0.6 mg/kg, 0.8 mg/kg, 1 mg/kg, 1.33 mg/kg, 1.75 mg/kg, or 2.0 mg/kg of a polypeptide comprising an amino acid sequence consisting of the amino acid sequence of SEQ ID NO:11, wherein the polypeptide is administered to the subject subcutaneously once every 21 days. In specific embodiments, the myeloproliferative neoplasm-associated myelofibrosis is primary myelofibrosis. In specific embodiments, the myeloproliferative neoplasm-associated myelofibrosis is polycythemia vera myelofibrosis. In more specific embodiments, the polycythemia vera myelofibrosis is associated with, or caused by, a *JAK2* mutation, *e.g.,* a *JAK2* V617F mutation or *JAK2* exon 12 mutation, or a thrombopoietin receptor (MPL) mutation. In specific embodiments, the myeloproliferative neoplasm-associated myelofibrosis is post-polycythemia vera myelofibrosis. In more specific embodiments, the post-polycythemia vera myelofibrosis is associated with, or caused by, a *JAK2* mutation, *e.g.,* a *JAK2* V617F mutation, or a thrombopoietin receptor (MPL) mutation. In specific embodiments, the myeloproliferative neoplasm-associated myelofibrosis is essential thrombocytopenia or post-essential thrombocythemia myelofibrosis. In more specific embodiments, the essential thrombocytopenia or post-essential thrombocythemia myelofibrosis is associated with, or caused by, a *JAK2* mutation, *e.g.,* a *JAK2* V617F mutation, or a thrombopoietin receptor (MPL) mutation. In more specific embodiments, the essential thrombocytopenia or post-essential thrombocythemia myelofibrosis includes reticulin fibrosis or trichrome fibrosis.

In specific embodiments, the subject is a subject described in Section 5.3. In specific embodiments, the subject is red blood cell transfusion-independent. In specific embodiments, the subject is red blood cell transfusion-independent if the subject has received no (0) red blood cell units during a period of time of 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to administration of the ActRIIB signaling inhibitor to the subject. In specific embodiments, the subject is red blood cell transfusion-dependent. In specific embodiments, the subject is red blood cell transfusion dependent if the subject has received an average red blood cell transfusion frequency of 2 to 4 red blood cell units per 28 days during a period of time of at least 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to administration of the ActRIIB signaling inhibitor to the subject.

In specific embodiments, the ActRIIB signaling inhibitor is administered to the subject as part of a composition. In specific embodiments, the composition is a composition described in Section 5.5.

In one embodiment, the method comprises administering to the subject a dose of 0.33 mg/kg, 0.45 mg/kg, 0.6 mg/kg, 0.8 mg/kg, 1 mg/kg, 1.33 mg/kg, 1.75 mg/kg, or 2.0 mg/kg of a polypeptide consisting of the amino acid sequence of SEQ ID NO:11, wherein the polypeptide is administered to the subject subcutaneously once every 21 days. In specific embodiments, the myeloproliferative neoplasm-associated myelofibrosis is primary myelofibrosis. In specific embodiments, the myeloproliferative neoplasm-associated myelofibrosis is polycythemia vera myelofibrosis. In more specific embodiments, the polycythemia vera myelofibrosis is associated with, or caused by, a *JAK2* mutation, *e*.*g*., a *JAK2* V617F mutation or *JAK2* exon 12 mutation, or a thrombopoietin receptor (MPL) mutation. In specific embodiments, the myeloproliferative neoplasm-associated myelofibrosis is post-polycythemia vera myelofibrosis. In more specific embodiments, the post-polycythemia vera myelofibrosis is associated with, or caused by, a *JAK2* mutation, *e.g.,* a *JAK2* V617F mutation, or a thrombopoietin receptor (MPL) mutation. In specific embodiments, the myeloproliferative neoplasm-associated myelofibrosis is essential thrombocytopenia or post-essential thrombocythemia myelofibrosis. In more specific embodiments, the essential thrombocytopenia or post-essential thrombocythemia myelofibrosis is associated with, or caused by, a *JAK2* mutation, *e.g.,* a *JAK2* V617F mutation, or a thrombopoietin receptor (MPL) mutation. In more specific embodiments, the essential thrombocytopenia or post-essential thrombocythemia myelofibrosis includes reticulin fibrosis or trichrome fibrosis.

In specific embodiments, the subject is a subject described in Section 5.3. In specific embodiments, the subject is red blood cell transfusion-independent. In specific embodiments, the subject is red blood cell transfusion-independent if the subject has received no (0) red blood cell units during a period of time of 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to administration of the ActRIIB signaling inhibitor to the subject. In specific embodiments, the subject is red blood cell transfusion-dependent. In specific embodiments, the subject is red blood cell transfusion dependent if the subject has received an average red blood cell transfusion frequency of 2 to 4 red blood cell units per 28 days during a period of time of at least 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to administration of the ActRllB signaling inhibitor to the subject.

In specific embodiments, the ActRIIB signaling inhibitor is administered to the subject as part of a composition. In specific embodiments, the composition is a composition described in Section 5.5.

### 5.3 PATIENT POPULATION

The subjects treated in accordance with the methods described herein can be any mammals such as rodents and primates, and in a preferred embodiment, humans. In certain embodiments, the subject is a human. In certain embodiments, the methods described herein can be used to treat myeloproliferative neoplasm-associated myelofibrosis and/or anemia in any mammals, such as rodents and primates, and in a preferred embodiment, in human subjects.

In certain embodiments, the subject treated in accordance with the methods described here can be of any age. In certain embodiments, the subject treated in accordance with the methods described herein is less than 18 years old. In a specific embodiment, the subject treated in accordance with the methods described herein is less than 13 years old. In another specific embodiment, the subject treated in accordance with the methods described herein is less than 12, less than 11, less than 10, less than 9, less than 8, less than 7, less than 6, or less than 5 years old. In another specific embodiment, the subject treated in accordance with the methods described herein is 1-3 years old, 3-5 years old, 5-7 years old, 7-9 years old, 9-11 years old, 11-13 years old, 13-15 years old, 15-20 years old, 20-25 years old, 25-30 years old, or greater than 30 years old. In another specific embodiment, the subject treated in accordance with the methods described herein is 30-35 years old, 35-40 years old, 40-45 years old, 45-50 years old, 50-55 years old, 55-60 years old, or greater than 60 years old. In another specific embodiment, the subject treated in accordance with the methods described herein is 60-65 years old, 65-70 years old, 70-75 years old, 75-80 years old, or greater than 80 years old.

The subject treated according to the methods provided herein has myeloproliferative neoplasm-associated myelofibrosis. In certain embodiments, the myeloproliferative neoplasm-associated myelofibrosis is primary myelofibrosis. In specific embodiments, the myeloproliferative neoplasm-associated myelofibrosis is polycythemia vera myelofibrosis. In more specific embodiments, the polycythemia vera myelofibrosis is associated with, or caused by, a *JAK2* mutation, *e.g.,* a *JAK2* V617F mutation or *JAK2* exon 12 mutation, or a thrombopoietin receptor (MPL) mutation. In specific embodiments, the myeloproliferative neoplasm-associated myelofibrosis is post-polycythemia vera myelofibrosis. In more specific embodiments, the post-polycythemia vera myelofibrosis is associated with, or caused by, a *JAK2* mutation, *e.g.,* a *JAK2* V617F mutation, or a thrombopoietin receptor (MPL) mutation. In specific embodiments, the myeloproliferative neoplasm-associated myelofibrosis is essential thrombocytopenia or post-essential thrombocythemia myelofibrosis. In more specific embodiments, the essential thrombocytopenia or post-essential thrombocythemia myelofibrosis is associated with, or caused by, a *JAK2* mutation, *e.g.,* a *JAK2* V617F mutation, or a thrombopoietin receptor (MPL) mutation. In more specific embodiments, the essential thrombocytopenia or post-essential thrombocythemia myelofibrosis includes reticulin fibrosis or trichrome fibrosis.

The subject treated according to the methods provided herein has myeloproliferative neoplasm-associated myelofibrosis and anemia. In certain embodiments, the myeloproliferative neoplasm-associated myelofibrosis is primary myelofibrosis. In specific embodiments, the myeloproliferative neoplasm-associated myelofibrosis is polycythemia vera myelofibrosis. In more specific embodiments, the polycythemia vera myelofibrosis is associated with, or caused by, a *JAK2* mutation, *e.g.,* a *JAK2* V617F mutation or *JAK2* exon 12 mutation, or a thrombopoietin receptor (MPL) mutation. In specific embodiments, the myeloproliferative neoplasm-associated myelofibrosis is post-polycythemia vera myelofibrosis. In more specific embodiments, the post-polycythemia vera myelofibrosis is associated with, or caused by, a *JAK2* mutation, *e.g.,* a *JAK2* V617F mutation, or a thrombopoietin receptor (MPL) mutation. In specific embodiments, the myeloproliferative neoplasm-associated myelofibrosis is essential thrombocytopenia or post-essential thrombocythemia myelofibrosis. In more specific embodiments, the essential thrombocytopenia or post-essential thrombocythemia myelofibrosis is associated with, or caused by, a *JAK2* mutation, *e.g.,* a *JAK2* V617F mutation, or a thrombopoietin receptor (MPL) mutation. In more specific embodiments, the essential thrombocytopenia or post-essential thrombocythemia myelofibrosis includes reticulin fibrosis or trichrome fibrosis.

The subject treated according to the methods provided herein has previously been treated with ruxolitinib. In a specific embodiment, a subject treated according to the methods provided herein has been on a stable dose of ruxolitinib for at least 112 days immediately prior to treatment.

In certain embodiments, a subject treated according to the methods provided herein is red blood cell transfusion-independent. In specific embodiments, a subject is red blood cell transfusion-independent if the subject has received no (0) red blood cell units during a period of time of 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to a first administration of the ActRIIB signaling inhibitor to the subject.

In certain embodiments, a subject treated according to the methods provided herein is red blood cell transfusion-dependent. In specific embodiments, a subject is red blood cell transfusion-dependent if the subject has received an average red blood cell transfusion frequency of 2 to 4 red blood cell units per 28 days during a period of time of at least 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to a first administration of the ActRIIB signaling inhibitor to the subject.

### 5.4 INHIBITORS OF ACTRIIB SIGNALING

As used herein, the term "ActRIIB" refers to a family of activin receptor type IIB (ActRIIB) proteins from any species and variants derived from such ActRIIB proteins by mutagenesis or other modification. Reference to ActRIIB herein is understood to be a reference to any one of the currently identified forms of the receptor. Members of the ActRIIB family are generally transmembrane proteins, composed of a ligand-binding extracellular domain with a cysteine-rich region, a transmembrane domain, and a cytoplasmic domain with predicted serine/threonine kinase activity.

ActRIIB signaling inhibitors include, without limitation, activin-binding soluble ActRIIB polypeptides; antibodies that bind to activin (particularly the activin A or B subunits, also referred to as βA or βB) and disrupt ActRIIB binding; antibodies that bind to ActRIIB and disrupt activin binding; non-antibody proteins selected for activin or ActRIIB binding; and randomized peptides selected for activin or ActRIIB binding, which can be conjugated to an Fc domain.

Two or more different proteins (or other moieties) with activin or ActRIIB binding activity, especially activin binders that block the type I (e.g., a soluble type I activin receptor) and type II (e.g., a soluble type II activin receptor) binding sites, respectively, may be linked together to create a bifunctional or multifunctional binding molecule that inhibits ActRIIB and thus can be used in the compositions and methods described herein include. cases Activin-ActRIIB signaling axis antagonists that inhibit ActRIIB include nucleic acid aptamers, small molecules and other agents are used in the compositions and methods described herein include.

### 5.4.1 ActRIIB Signaling Inhibitors Comprising ActRIIB Polypeptides

As used herein, the term "ActRIIB polypeptide" refers to polypeptides comprising any naturally occurring polypeptide of an ActRIIB family member as well as any variants thereof (including mutants, fragments, fusions, and peptidomimetic forms) that retain a useful activity. For example, ActRIIB polypeptides include polypeptides derived from the sequence of any known ActRIIB receptor having a sequence at least about 80% identical to the sequence of an ActRIIB polypeptide, and optionally at least 85%, 90%, 95%, 96%, 97%, 98%, 99% or greater identity. For example, an ActRIIB polypeptide may bind to and inhibit the function of an ActRIIB protein and/or activin. An example of an ActRIIB polypeptide includes the human ActRIIB precursor polypeptide (SEQ ID NO:3 or SEQ ID NO: 14). With respect to the ActRIIB precursor polypeptide whose amino acid sequence is depicted as SEQ ID NO:3 or SEQ ID NO: 14 (i.e., the human ActRIIB precursor polypeptide), the signal peptide of the ActRIIB precursor polypeptide is located at amino acids 1 to 18; the extracellular domain is located at amino acids 19 to 134 and the potential N-linked glycosylation sites are located at amino acid positions 42 and 65. The nucleic acid sequence encoding the human ActRIIB precursor polypeptide of SEQ ID NO:3 is disclosed as SEQ ID NO:6 (SEQ ID NO:6 provides an alanine at the codon corresponding to amino acid position 64, but could be readily modified by one of skill in the art using methods known in the art to provide an arginine at the codon corresponding to amino acid position 64 instead). *See* Table 2 for a description of the sequences. The ActRIIB signaling inhibitor of the invention is definied in the claims.

The numbering of amino acids for all of the ActRIIB-related polypeptides described herein is based on the amino acid numbering for SEQ ID NO:3 and SEQ ID NO: 14 (which only differ in the amino acid expressed at position 64), unless specifically designated otherwise. For example, if an ActRIIB polypeptide is described as having a substitution/mutation at amino acid position 79, then it is to be understood that position 79 refers to the 79th amino acid in SEQ ID NO:3 or SEQ ID NO:14, from which the ActRIIB polypeptide is derived. Likewise, if an ActRIIB polypeptide is described as having an alanine or an arginine at amino acid position 64, then it is to be understood that position 64 refers to the 64th amino acid in SEQ ID NO:3 or SEQ ID NO:14, from which the ActRIIB polypeptide is derived.

The inhibitors of ActRIIB signaling used in the compositions and methods described herein comprise polypeptides comprising an activin-binding domain of ActRIIB. In some cases the activin-binding domains of ActRIIB comprise the extracellular domain of ActRIIB, or a portion thereof. In specific cases the extracellular domain or portion thereof of ActRIIB is soluble. Illustrative modified forms of ActRIIB polypeptides are disclosed in U.S. Patent Application Publication Nos. 20090005308 and 20100068215. Illustrative modified forms of ActRIIB polypeptides are also disclosed in International Patent Application Publication Nos. WO 2008/097541 and WO 2010/019261

In specific cases the ActRIIB polypeptides used in the compositions and methods described herein are soluble ActRIIB polypeptides. The term "soluble ActRIIB polypeptide" generally refers to polypeptides comprising an extracellular domain of an ActRIIB protein, including any naturally occurring extracellular domain of an ActRIIB protein as well as any variants thereof (including mutants, fragments and peptidomimetic forms). Soluble ActRIIB polypeptides can bind to activin; however, the wild type ActRIIB protein does not exhibit significant selectivity in binding to activin versus GDF8/11. In certain cases altered forms of ActRIIB with different binding properties can be used in the methods provided herein. Such altered forms are disclosed, *e.g*., in international patent application publication Nos. WO 2006/012627 and WO 2010/019261. Native or altered ActRIIB proteins may be given added specificity for activin by coupling them with a second, activin-selective binding agent. Exemplary soluble ActRIIB polypeptides include the extracellular domain of a human ActRIIB polypeptide (e.g., SEQ ID NOs: 4, 5, 9, 12, 13, 15, 16, 17, 18, 19, 22, 23, 28, and 29).

An Fc fusion protein having the ActRIIB extracellular sequence disclosed by Hilden et al. (Blood, 1994, 83(8):2163-70), which has an alanine at the position corresponding to amino acid 64 of the ActRIIB precursor amino acid sequence, i.e., SEQ ID NO:3 (herein referred to as "A64"), has been demonstrated to possess a relatively low affinity for activin and GDF-11. By contrast, an Fc fusion protein with an arginine at position 64 of the ActRIIB precursor amino acid sequence (herein referred to as "R64") has an affinity for activin and GDF-11 in the low nanomolar to high picomolar range (see, e.g., U.S. Patent Application Publication No. 20100068215). See, also, International Publication No. WO 2010/019261.

An ActRIIB precursor amino acid sequence with an arginine at position 64 is presented in SEQ ID NO:14. As such, in certain embodiments, the ActRIIB polypeptides used in accordance with the compositions and methods described herein may comprise either (i) an alanine at the position corresponding to amino acid 64 of the ActRIIB precursor amino acid sequence, i.e., SEQ ID NO:3; or (ii) an arginine at position 64 of the ActRIIB precursor amino acid sequence, i.e., SEQ ID NO:14. In other embodiments, the ActRIIB polypeptides used in accordance with the compositions and methods described herein may comprise an amino acid that is not alanine or arginine at the position corresponding to amino acid 64 of the ActRIIB precursor amino acid sequence, i.e., SEQ ID NO:3 or SEQ ID NO:14.

It has been shown that a deletion of the proline knot at the C-terminus of the extracellular domain of ActRIIB reduces the affinity of the receptor for activin (see, e.g., Attisano et al., Cell, 1992, 68(1):97-108). An ActRIIB-Fc fusion protein containing amino acids 20-119 of SEQ ID NO:14 (i.e., SEQ ID NO:18), "ActRIIB(20-119)-Fc" has reduced binding to GDF-11 and activin relative to an ActRIIB-Fc fusion protein containing amino acids 20-134 of SEQ ID NO:14 (i.e., SEQ ID NO:17), "ActRIIB(20-134)-Fc", which includes the proline knot region and the complete juxtamembrane domain. However, an ActRIIB-Fc fusion protein containing amino acids 20-129 of SEQ ID NO:14, "ActRIIB(20-129)-Fc" retains similar but somewhat reduced activity relative to the non-truncated extracellular domain of ActRIIB, even though the proline knot region is disrupted. Thus, ActRIIB polypeptides comprising extracellular domains that stop at amino acid 134, 133, 132, 131, 130 and 129 of SEQ ID NO:14 (or SEQ ID NO:3) are all expected to be active, but constructs stopping at amino acid 134 or 133 may be most active. Similarly, mutations at any of residues 129-134 are not expected to alter ligand binding affinity by large margins, as indicated by the fact that mutations of P129 and P130 of SEQ ID NO:14 do not substantially decrease ligand binding. Therefore, the ActRIIB polypeptides used in accordance with the methods and compositions described herein may end as early as amino acid 109 (i.e., the final cysteine) of SEQ ID NO: 14 (or SEQ ID NO:3), however, forms ending at or between amino acid positions 109 and 119 of SEQ ID NO:14 (or SEQ ID NO:3) are expected to have reduced ligand binding ability.

Amino acid 29 of SEQ ID NO:3 and SEQ ID NO:14 represents the initial cysteine in the ActRIIB precursor sequence. It is expected that an ActRIIB polypeptide beginning at amino acid 29 of the N-terminus of SEQ ID NO:3 or SEQ ID NO:14, or before these amino acid positions, will retain ligand binding activity. An alanine to asparagine mutation at position 24 of SEQ ID NO:3 or SEQ ID NO:14 introduces an N-linked glycosylation sequence without substantially affecting ligand binding. This confirms that mutations in the region between the signal cleavage peptide and the cysteine cross-linked region, corresponding to amino acids 20-29 of SEQ ID NO:3 or SEQ ID NO:14, are well tolerated. In particular, ActRIIB polypeptides beginning at amino acid position 20, 21, 22, 23 and 24 of SEQ ID NO:3 or SEQ ID NO:14 will retain activity, and ActRIIB polypeptides beginning at amino acid positions 25, 26, 27, 28 and 29 of SEQ ID NO:3 or SEQ ID NO:14 are also expected to retain activity. An ActRIIB polypeptide beginning at amino acid position 22, 23, 24 or 25 of SEQ ID NO:3 or SEQ ID NO: 14 will have the most activity.

Taken together, the active portions (i.e., ActRIIB polypeptides) of the ActRIIB precursor protein (i.e., SEQ ID NO:3 or SEQ ID NO:14) to be used in accordance with the methods and compositions described herein will generally comprise amino acids 29-109 of SEQ ID NO:3 or SEQ ID NO:14, and such ActRIIB polypeptides may, for example, begin at a residue corresponding to any one of amino acids 19-29 of SEQ ID NO:3 or SEQ ID NO:14 and end at a position corresponding to any one of amino acids 109-134 of SEQ ID NO:3 or SEQ ID NO:14. Specific examples of ActRIIB polypeptides encompassed herein include those that begin at an amino acid position from 19-29, 20-29 or 21-29 of SEQ ID NO:3 or SEQ ID NO:14 and end at an amino acid position from 119-134, 119-133 or 129-134, 129-133 of SEQ ID NO:3 or SEQ ID NO:14. Other specific examples of ActRIIB polypeptides encompassed herein include those that begin at an amino acid position from 20-24 (or 21-24, or 22-25) of SEQ ID NO:3 or SEQ ID NO: 14 and end at an amino acid position from 109-134 (or 109-133), 119-134 (or 119-133) or 129-134 (or 129-133) of SEQ ID NO:3 or SEQ ID NO:14. Variant ActRIIB polypeptides falling within these ranges are also contemplated, particularly those having at least 80%, 85%, 90%, 91%, 92%, 93%, 94%, 95%, 96%, 97%, 98%, or 99% sequence identity or sequence homology to the corresponding portion of SEQ ID NO:3 or SEQ ID NO:14.

In certain cases, the inhibitors of ActRIIB signaling used in the compositions and methods described herein comprise a truncated form of an extracellular domain of ActRIIB. The truncation can be at the carboxy terminus and/or the amino terminus of the ActRIIB polypeptide. In certain cases, the truncation can be 1, 2, 3, 4, 5, 6, 7, 8, 9, 10, 11, 12, 13, 14, 15, 16, 17, 18, 19, 20, 21, 22, 23, 24, or 25 amino acids long relative to the mature ActRIIB polypeptide extracellular domain. In certain cases, the truncation can be 1, 2, 3, 4, 5, 6, 7, 8, 9, 10, 11, 12, 13, 14, 15, 16, 17, 18, 19, 20, 21, 22, 23, 24, or 25 N-terminal amino acids of the mature ActRIIB polypeptide extracellular domain. In certain cases, the truncation can be 1, 2, 3, 4, 5, 6, 7, 8, 9, 10, 11, 12, 13, 14, 15, 16, 17, 18, 19, 20, 21, 22, 23, 24, or 25 C-terminal amino acids of the mature ActRIIB polypeptide extracellular domain. For example, truncated forms of ActRIIB include polypeptides with amino acids 20-119; 20-128; 20-129; 20-130; 20-131; 20-132; 20-133; 20-134; 20-131; 21-131; 22-131; 23-131; 24-131; and 25-131, wherein the amino acid positions refer to the amino acid positions in SEQ ID NO:3 or SEQ ID NO:14.

Additional exemplary truncated forms of ActRIIB include (i) polypeptides beginning at amino acids at any of amino acids 21-29 of SEQ ID NO:3 or SEQ ID NO:14 (optionally beginning at 22-25 of SEQ ID NO: 3 or SEQ ID NO:14) and ending at any of amino acids 109-134 of SEQ ID NO:3 or SEQ ID NO:14; (ii) polypeptides beginning at any of amino acids 20-29 of SEQ ID NO:3 or SEQ ID NO:14 (optionally beginning at 22-25 of SEQ ID NO:3 or SEQ ID NO:14) and ending at any of amino acids 109-133 of SEQ ID NO: 3 or SEQ ID NO:14; (iii) polypeptides beginning at any of amino acids 20-24 of SEQ ID NO:3 or SEQ ID NO:14 (optionally beginning at 22-25 of SEQ ID NO:3 or SEQ ID NO:14) and ending at any of amino acids 109-133 of SEQ ID NO:3 or SEQ ID NO:14; (iv) polypeptides beginning at any of amino acids 21-24 of SEQ ID NO: 3 or SEQ ID NO:14 and ending at any of amino acids 109-134 of SEQ ID NO: 3 or SEQ ID NO:14; (v) polypeptides beginning at any of amino acids 20-24 of SEQ ID NO:3 or SEQ ID NO:14 and ending at any of amino acids 118-133 of SEQ ID NO:3 or SEQ ID NO:14; (vi) polypeptides beginning at any of amino acids 21-24 of SEQ ID NO:3 or SEQ ID NO:14 and ending at any of amino acids 118-134 of SEQ ID NO:3 or SEQ ID NO:14; (vii) polypeptides beginning at any of amino acids 20-24 of SEQ ID NO:3 or SEQ ID NO:14 and ending at any of amino acids 128-133 of SEQ ID NO:3 or SEQ ID NO:14; (viii) polypeptides beginning at any of amino acids 20-24 of SEQ ID NO:3 or SEQ ID NO:14 and ending at any of amino acids 128-133 of SEQ ID NO:3 or SEQ ID NO:14, (ix) polypeptides beginning at any of amino acids 21-29 of SEQ ID NO:3 or SEQ ID NO:14 and ending at any of amino acids 118-134 of SEQ ID NO:3 or SEQ ID NO:14; (x) polypeptides beginning at any of amino acids 20-29 of SEQ ID NO:3 or SEQ ID NO:14 and ending at any of amino acids 118-133 of SEQ ID NO:3 or SEQ ID NO:14; (xi) polypeptides beginning at any of amino acids 21-29 of SEQ ID NO:3 or SEQ ID NO:14 and ending at any of amino acids 128-134 of SEQ ID NO:3 or SEQ ID NO:14; and (xii) polypeptides beginning at any of amino acids 20-29 of SEQ ID NO:3 or SEQ ID NO:14 and ending at any of amino acids 128-133 of SEQ ID NO:3 or SEQ ID NO:14. In a specific case, an ActRIIB polypeptides comprises, consists essentially of, or consists of, an amino acid sequence beginning at amino acid position 25 of SEQ ID NO:3 or SEQ ID NO:14 and ending at amino acid position 131 of SEQ ID NO:3 or SEQ ID NO:14. In another specific case, an ActRIIB polypeptide consists of, or consists essentially of, the amino acid sequence of SEQ ID NO:4, 18, 23, 26, 27, 29, 30, 31, 32, or 33.

Any of the ActRIIB polypeptides used in the compositions and methods described herein may be produced as a homodimer. Any of the ActRIIB polypeptides used in the compositions and methods described herein may be formulated as a fusion protein having a heterologous portion that comprises a constant region from an IgG heavy chain, such as an Fc domain. Any of the ActRIIB polypeptides used in the compositions and methods described herein may comprise an acidic amino acid at the position corresponding to position 79 of SEQ ID NO:3 or SEQ ID NO:14, optionally in combination with one or more additional amino acid substitutions, deletions or insertions relative to SEQ ID NO:3 or SEQ ID NO:14.

In specific cases, the inhibitors of ActRIIB signaling used in the compositions and methods described herein comprise an extracellular domain of ActRIIB with one or more amino acid substitutions/mutations. Such an amino acid substitution/mutation can be, for example, an exchange from the leucine at amino acid position 79 of SEQ ID NO:3 or SEQ ID NO:14 to an acidic amino acid, such as aspartic acid or glutamic acid. For example, position L79 of SEQ ID NO: 3 or SEQ ID NO:14 may be altered in ActRIIB extracellular domain polypeptides to confer altered activin-myostatin (GDF-11) binding properties. L79A and L79P mutations reduce GDF-11 binding to a greater extent than activin binding. L79E and L79D mutations retain GDF-11 binding, while demonstrating greatly reduced activin binding.

In certain cases, the inhibitors of ActRIIB signaling used in the compositions and methods described herein comprise a truncated form of an ActRIIB extracellular domain that also carries an amino acid substitution, e.g., an exchange from the leucine at amino acid position 79 of SEQ ID NO: 3 or SEQ ID NO:14 to an acidic amino acid, such as aspartic acid or glutamic acid. According to the claims. the truncated form of an extracellular domain of ActRIIB polypeptide that also carries an amino acid substitution used in the compositions and methods described herein is SEQ ID NO:9. Forms of ActRIIB that are truncated and/or carry one or more amino acid substitutions can be linked to an Fc domain of an antibody as discussed above.

Functionally active fragments of ActRIIB polypeptides can be obtained, for example, by screening polypeptides recombinantly produced from the corresponding fragment of the nucleic acid encoding an ActRIIB polypeptide. In addition, fragments can be chemically synthesized using techniques known in the art such as conventional Merrifield solid phase f-Moc or t-Boc chemistry. The fragments can be produced (recombinantly or by chemical synthesis) and tested to identify those peptidyl fragments that can function as antagonists (inhibitors) of ActRIIB protein or signaling mediated by activin.

In addition, functionally active variants of ActRIIB polypeptides can be obtained, for example, by screening libraries of modified polypeptides recombinantly produced from the corresponding mutagenized nucleic acids encoding an ActRIIB polypeptide. The variants can be produced and tested to identify those that can function as antagonists (inhibitors) of ActRIIB protein or signaling mediated by activin. In certain cases, a functional variant of the ActRIIB polypeptides comprises an amino acid sequence that is at least 75% identical to an amino acid sequence selected from SEQ ID NO:4, 5, 9, 12, 13, 15, 16, 17, 18, 19, 22, 23, 28, and 29. In certain cases, the functional variant has an amino acid sequence at least 80%, 85%, 90%, 95%, 96%, 97%, 98%, or 99% identical to an amino acid sequence selected from SEQ ID NO:4, 5, 9, 12, 13, 15, 16, 17, 18, 19, 22, 23, 28, and 29.

Functional variants may be generated, for example, by modifying the structure of an ActRIIB polypeptide for such purposes as enhancing therapeutic efficacy, or stability (e.g., ex vivo shelf life and resistance to proteolytic degradation in vivo). Such modified ActRIIB polypeptides when selected to retain activin binding, are considered functional equivalents of the naturally-occurring ActRIIB polypeptides. Modified ActRIIB polypeptides can also be produced, for instance, by amino acid substitution, deletion, or addition. For instance, it is reasonable to expect that an isolated replacement of a leucine with an isoleucine or valine, an aspartate with a glutamate, a threonine with a serine, or a similar replacement of an amino acid with a structurally related amino acid (e.g., conservative mutations) will not have a major effect on the biological activity of the resulting molecule. Conservative replacements are those that take place within a family of amino acids that are related in their side chains. Whether a change in the amino acid sequence of an ActRIIB polypeptide results in a functional homolog can be readily determined by assessing the ability of the variant ActRIIB polypeptide to produce a response in cells in a fashion similar to the wild-type ActRIIB polypeptide.

ActRIIB polypeptide mutants, particularly sets of combinatorial mutants of an ActRIIB polypeptide, as well as truncation mutants, pools of combinatorial mutants are especially useful for identifying functional variant sequences can be used in the methods and compositions described herein. The purpose of screening such combinatorial libraries may be to generate, for example, ActRIIB polypeptide variants which can act as either agonists or antagonist, or alternatively, which possess novel activities all together.

It has been demonstrated that the ligand binding pocket of ActRIIB is defined by residues Y31, N33, N35, L38 through T41, E47, E50, Q53 through K55, L57, H58, Y60, S62, K74, W78 through N83, Y85, R87, A92, and E94 through F 101 of SEQ ID NO:3 or SEQ ID NO: 14. At these positions, it is expected that conservative mutations will be tolerated, although a K74A mutation is well-tolerated, as are R40A, K55A, F82A and mutations at position L79. R40 is a K in Xenopus, indicating that basic amino acids at this position will be tolerated. Q53 is R in bovine ActRIIB and K in Xenopus ActRIIB, and therefore amino acids including R, K, Q, N and H will be tolerated at this position. Thus, a general formula for an ActRIIB polypeptide for use in the methods and compositions described herein is one that comprises amino acids 29-109 of SEQ ID NO:3 or SEQ ID NO: 14, but optionally beginning at an amino acid position ranging from 20-24 or 22-25 of SEQ ID NO:3 or SEQ ID NO: 14 and ending at an amino acid position ranging from 129-134 of SEQ ID NO:3 or SEQ ID NO:14, and comprising no more than 1, 2, 5, or 15 conservative amino acid changes in the ligand binding pocket, and zero, one or more non-conservative alterations at amino acid positions 40, 53, 55, 74, 79 and/or 82 of SEQ ID NO:3 or SEQ ID NO:14 in the ligand binding pocket. Such an ActRIIB polypeptide may retain greater than 80%, 90%, 95% or 99% sequence identity or sequence homology to the sequence of amino acids 29-109 of SEQ ID NO:3 or SEQ ID NO:14. Sites outside the binding pocket, at which variability may be particularly well tolerated, include the amino and carboxy termini of the extracellular domain of ActRIIB, and positions 42-46 and 65-73. An asparagine to alanine alteration at position 65 of SEQ ID NO:3 or SEQ ID NO:14 (N65A) actually improves ligand binding in the A64 background, and is thus expected to have no detrimental effect on ligand binding in the R64 background. This change probably eliminates glycosylation at N65 in the A64 background, thus demonstrating that a significant change in this region is likely to be tolerated. While an R64A change is poorly tolerated, R64K is well-tolerated, and thus another basic residue, such as H may be tolerated at position 64.

As a specific example of an ActRIIB polypeptide with a mutation in the ligand binding domain, the positively-charged amino acid residue Asp (D80) of the ligand-binding domain of ActRIIB can be mutated to a different amino acid residue such that the variant ActRIIB polypeptide preferentially binds to GDF8, but not activin. In a specific case, the D80 residue is changed to an amino acid residue selected from the group consisting of: an uncharged amino acid residue, a negative amino acid residue, and a hydrophobic amino acid residue. As a further specific example, the hydrophobic residue L79 can be altered to the acidic amino acids aspartic acid or glutamic acid to greatly reduce activin binding while retaining GDF11 binding. As will be recognized by one of skill in the art, most of the described mutations, variants or modifications may be made at the nucleic acid level or, in some cases, by post translational modification or chemical synthesis. Such techniques are well known in the art.

In specific cases, the inhibitors of ActRIIB signaling used in the compositions and methods described herein comprise a conjugate/fusion protein comprising an extracellular domain (e.g., an activin-binding domain) of an ActRIIB receptor linked to an Fc portion of an antibody. Such conjugate/fusion proteins may comprise any of the ActRIIB polypeptides disclosed herein (e.g., any of SEQ ID NOs:17, 18, 23, 26, 27, 29, 30, 31, 32, 33), any ActRIIB polypeptides known in the art, or any ActRIIB polypeptides generated using methods known in the art and/or provided herein.

In certain cases, the extracellular domain is linked to an Fc portion of an antibody via a linker, e.g., a peptide linker. Exemplary linkers include short polypeptide sequences such as 2-10, 2-5, 2-4, 2-3 amino acid residues (e.g., glycine residues), such as, for example, a Gly-Gly-Gly linker. In a specific case, the linker comprises the amino acid sequence Gly-Gly-Gly (GGG). In another specific case, the linker comprises the amino acid sequence Thr-Gly-Gly-Gly (TGGG). Optionally, the Fc domain has one or more mutations at residues such as Asp-265, lysine 322, and Asn-434. In certain cases, the mutant Fc domain having one or more of these mutations (e.g., an Asp-265 mutation) has a reduced ability to bind to the Fcy receptor relative to a wild-type Fc domain. In other cases, the mutant Fc domain having one or more of these mutations (e.g., an Asn-434 mutation) has an increased ability to bind to the MHC class I- related Fc-receptor (FcRN) relative to a wild-type Fc domain. Exemplary fusion proteins comprising a soluble extracellular domain of ActRIIB fused to an Fc domain are set forth in SEQ ID NOs:20, 21, 24, 25, or 34.

In a specific case, the ActRIIB signaling inhibitors used in the compositions and methods described herein comprise the extracellular domain of ActRIIB, or a portion thereof, linked to an Fc portion of an antibody, wherein said ActRIIB signaling inhibitor comprises an amino acid sequence that is at least 75% identical to an amino acid sequence selected from SEQ ID NOs:20, 21, 24, 25, 34. In another specific case, the ActRIIB signaling inhibitors used in the compositions and methods described herein comprise the extracellular domain of ActRIIB, or a portion thereof, linked to an Fc portion of an antibody, wherein said ActRIIB signaling inhibitor comprises an amino acid sequence that is at least 80%, 85%, 90%, 95%, 96%, 97%, 98%, or 99% identical to an amino acid sequence selected from SEQ ID NOs:20, 21, 24, 25, and 34.

In a specific case, the ActRIIB signaling inhibitor to be used in the compositions and methods described herein is a fusion protein between the extracellular domain of the human ActRIIB receptor and the Fc portion of IgG1. In another specific case, the ActRIIB signaling inhibitor to be used in the compositions and methods described herein is a fusion protein between a truncated extracellular domain of the human ActRIIB receptor and the Fc portion of IgG1. In another specific case, the ActRIIB signaling inhibitor to be used in the compositions and methods described herein is a fusion protein between a truncated extracellular domain of the human ActRIIB receptor and the Fc portion of IgG1, wherein the truncated extracellular domain of the human ActRIIB receptor possesses an amino acid substitution at the amino acid position corresponding to amino acid 79 of SEQ ID NO:3 or SEQ ID NO:14. In one embodiment, the amino acid substitution at the amino acid position corresponding to amino acid 79 of SEQ ID NO:3 or SEQ ID NO:14 is substitution of Leucine for Aspartic Acid (i.e., an L79D mutation).

In a specific embodiment, the ActRIIB signaling inhibitor to be used in the compositions and methods described herein is SEQ ID NO:10 or 11, which represents a fusion protein between the extracellular domain of the human ActRIIB receptor and the Fc portion of IgG1, wherein said ActRIIB extracellular domain comprises amino acids 25-131 of SEQ ID NO:14 with an L79D mutation. The nucleic acid sequence encoding the ActRIIB-Fc fusion protein of SEQ ID NO:10 is presented in SEQ ID NO:31.

In another specific case, the ActRIIB signaling inhibitor to be used in the compositions and methods described herein is SEQ ID NO:20 or 21, which represents a fusion protein between the extracellular domain of the human ActRIIB receptor and the Fc portion of IgG1, wherein said ActRIIB extracellular domain comprises amino acids 25-131 of SEQ ID NO:3 with an L79D mutation.

Asparagine-linked glycosylation recognition sites generally comprise a tripeptide sequence, asparagine-X-threonine (or asparagine-X-serine) (where "X" is any amino acid) which is specifically recognized by appropriate cellular glycosylation enzymes. The alteration may also be made by the addition of, or substitution by, one or more serine or threonine residues to the sequence of the wild-type ActRIIB polypeptide (for O-linked glycosylation sites). A variety of amino acid substitutions or deletions at one or both of the first or third amino acid positions of a glycosylation recognition site (and/or amino acid deletion at the second position) results in non-glycosylation at the modified tripeptide sequence. Another means of increasing the number of carbohydrate moieties on an ActRIIB polypeptide is by chemical or enzymatic coupling of glycosides to the ActRIIB polypeptide. Depending on the coupling mode used, the sugar(s) may be attached to (a) arginine and histidine; (b) free carboxyl groups; (c) free sulfhydryl groups such as those of cysteine; (d) free hydroxyl groups such as those of serine, threonine, or hydroxyproline; (e) aromatic residues such as those of phenylalanine, tyrosine, or tryptophan, or (f) the amide group of glutamine. These methods are described in International Patent Application No. WO 87/05330 published Sep. 11, 1987, and in Aplin and Wriston (1981) CRC Crit. Rev. Biochem., pp. 259-306. Removal of one or more carbohydrate moieties present on an ActRIIB polypeptide may be accomplished chemically and/or enzymatically. Chemical deglycosylation may involve, for example, exposure of the ActRIIB polypeptide to the compound trifluoromethanesulfonic acid, or an equivalent compound. This treatment results in the cleavage of most or all sugars except the linking sugar (N-acetylglucosamine or N-acetylgalactosamine), while leaving the amino acid sequence intact. Chemical deglycosylation is further described by Hakimuddin et al. (1987) Arch. Biochem. Biophys. 259:52 and by Edge et al. (1981) Anal. Biochem. 118:131. Enzymatic cleavage of carbohydrate moieties on ActRIIB polypeptides can be achieved by the use of a variety of endo- and exo-glycosidases as described by Thotakura et al. (1987) Meth. Enzymol. 138:350. The sequence of an ActRIIB polypeptide may be subsequent, as appropriate, depending on the type of expression system used, as mammalian, yeast, insect and plant cells may all introduce differing glycosylation patterns that can be affected by the amino acid sequence of the peptide. In general, ActRIIB proteins for use in humans will be expressed in a mammalian cell line that provides proper glycosylation, such as HEK293 or CHO cell lines, although other expression systems, such as other mammalian expression cell lines, yeast cell lines with engineered glycosylation enzymes and insect cells, are expected to be useful as well.

In specific cases, mutated ActRIIB polypeptides comprising the addition of a further N-linked glycosylation site (N-X-S/T) that increases the serum half-life of an ActRIIB-Fc fusion protein, relative to the ActRIIB(R64)-Fc form can be used in the methods and compositions described herein. In a specific case, introduction of an asparagine at position 24 of SEQ ID NO:3 or SEQ ID NO:14 (A24N) results in the creation of an NXT sequence that confers a longer half-life. Other NX(T/S) sequences can be found at 42-44 (NQS) and 65-67 (NSS), although the latter may not be efficiently glycosylated with the R at position 64 (i.e., in R64 polypeptides). N-X-S/T sequences may be generally introduced at positions outside the ligand binding pocket of ActRIIB, which is detailed above. Particularly suitable sites for the introduction of non-endogenous N-X-S/T sequences include amino acids 20-29, 20-24, 22-25, 109-134, 120-134 or 129-134 of SEQ ID NO:3 or SEQ ID NO: 14. N-X-S/T sequences may also be introduced into the linker between the ActRIIB sequence and the Fc or other fusion component. Such a site may be introduced with minimal effort by introducing an N in the correct position with respect to a pre-existing S or T, or by introducing an S or T at a position corresponding to a pre-existing N. Thus, desirable alterations that would create an N-linked glycosylation site are: A24N, R64N, S67N (possibly combined with an N65A alteration), E106N, R112N, G120N, E123N, P129N, A132N, R112S and R112T (with all amino acid positions corresponding to the positions they can be found in SEQ ID NO:3 or SEQ ID NO:14). Any S that is predicted to be glycosylated may be altered to a T without creating an immunogenic site, because of the protection afforded by the glycosylation. Likewise, any T that is predicted to be glycosylated may be altered to an S. Thus the alterations S67T and S44T are encompassed herein. Likewise, in an A24N variant, an S26T alteration may be used. Accordingly, an ActRIIB polypeptide may include one or more additional, non-endogenous N-linked glycosylation consensus sequences.

A variety of screening assays may be used to evaluate ActRIIB polypeptide variants. For example, an ActRIIB polypeptide variant may be screened for ability to bind to an ActRIIB ligand, to prevent binding of an ActRIIB ligand to an ActRIIB polypeptide or to interfere with signaling caused by an ActRIIB ligand. The activity of an ActRIIB polypeptide or its variants may also be tested in a cell-based or in vivo assay.

Combinatorially-derived variants can be generated which have a selective or generally increased potency relative to a naturally occurring ActRIIB polypeptide. Likewise, mutagenesis can give rise to variants which have intracellular half-lives dramatically different than the corresponding wild-type ActRIIB polypeptide. For example, the altered protein can be rendered either more stable or less stable to proteolytic degradation or other cellular processes which result in destruction of, or otherwise inactivation of a native ActRIIB polypeptide. Such variants, and the genes which encode them, can be utilized to alter ActRIIB polypeptide levels by modulating the half-life of the ActRIIB polypeptides. For instance, a short half-life can give rise to more transient biological effects and can allow tighter control of recombinant ActRIIB polypeptide levels within the subject. In an Fc fusion protein, mutations may be made in the linker (if any) and/or the Fc portion to alter the half-life of the protein.

A combinatorial library may be produced by way of a degenerate library of genes encoding a library of polypeptides which each include at least a portion of potential ActRIIB polypeptide sequences. For instance, a mixture of synthetic oligonucleotides can be enzymatically ligated into gene sequences such that the degenerate set of potential ActRIIB polypeptide nucleotide sequences are expressible as individual polypeptides, or alternatively, as a set of larger fusion proteins (e.g., for phage display).

There are many ways by which the library of potential homologs can be generated from a degenerate oligonucleotide sequence. Chemical synthesis of a degenerate gene sequence can be carried out in an automatic DNA synthesizer, and the synthetic genes then be ligated into an appropriate vector for expression. The synthesis of degenerate oligonucleotides is well known in the art (see for example, Narang, S A (1983) Tetrahedron 39:3; Itakura et al., (1981) Recombinant DNA, Proc. 3rd Cleveland Sympos. Macromolecules, ed. AG Walton, Amsterdam: Elsevier pp 273-289; Itakura et al., (1984) Annu. Rev. Biochem. 53:323; Itakura et al., (1984) Science 198:1056; Ike et al., (1983) Nucleic Acid Res. 11:477). Such techniques have been employed in the directed evolution of other proteins (see, for example, Scott et al., (1990) Science 249:386-390; Roberts et al., (1992) PNAS USA 89:2429-2433; Devlin et al., (1990) Science 249: 404-406; Cwirla et al., (1990) PNAS USA 87: 6378-6382; as well as U.S. Pat. Nos. 5,223,409, 5,198,346, and 5,096,815).

Alternatively, other forms of mutagenesis can be utilized to generate a combinatorial library. For example, ActRIIB polypeptide variants can be generated and isolated from a library by screening using, for example, alanine scanning mutagenesis and the like (Ruf et al., (1994) Biochemistry 33:1565-1572; Wang et al., (1994) J. Biol. Chem. 269:3095-3099; Balint et al., (1993) Gene 137:109-118; Grodberg et al., (1993) Eur. J. Biochem. 218:597-601; Nagashima et al., (1993) J. Biol. Chem. 268:2888-2892; Lowman et al., (1991) Biochemistry 30:10832-10838; and Cunningham et al., (1989) Science 244:1081-1085), by linker scanning mutagenesis (Gustin et al., (1993) Virology 193:653-660; Brown et al., (1992) Mol. Cell Biol. 12:2644-2652; McKnight et al., (1982) Science 232:316); by saturation mutagenesis (Meyers et al., (1986) Science 232:613); by PCR mutagenesis (Leung et al., (1989) Method Cell Mol Biol 1:11-19); or by random mutagenesis, including chemical mutagenesis, etc. (Miller et al., (1992) A Short Course in Bacterial Genetics, CSHL Press, Cold Spring Harbor, N.Y.; and Greener et al., (1994) Strategies in Mol Biol 7:32-34). Linker scanning mutagenesis, particularly in a combinatorial setting, is an attractive method for identifying truncated (bioactive) forms of ActRIIB polypeptides.

A wide range of techniques are known in the art for screening gene products of combinatorial libraries made by point mutations and truncations, and, for that matter, for screening cDNA libraries for gene products having a certain property. Such techniques will be generally adaptable for rapid screening of the gene libraries generated by the combinatorial mutagenesis of ActRIIB polypeptides. The most widely used techniques for screening large gene libraries typically comprises cloning the gene library into replicable expression vectors, transforming appropriate cells with the resulting library of vectors, and expressing the combinatorial genes under conditions in which detection of a desired activity facilitates relatively easy isolation of the vector encoding the gene whose product was detected. Preferred assays include activin binding assays and activin-mediated cell signaling assays.

In certain embodiments, ActRIIB polypeptides used in the methods and compositions described herein may further comprise post-translational modifications in addition to any that are naturally present in the ActRIIB polypeptides. Such modifications include, but are not limited to, acetylation, carboxylation, glycosylation, phosphorylation, lipidation, and acylation. As a result, the modified ActRIIB polypeptides may contain non-amino acid elements, such as polyethylene glycols, lipids, poly- or mono-saccharide, and phosphates. Effects of such non-amino acid elements on the functionality of an ActRIIB polypeptide may be tested by any method known to the skilled artisan. When an ActRIIB polypeptide is produced in cells by cleaving a nascent form of the ActRIIB polypeptide, post-translational processing may also be important for correct folding and/or function of the protein. Different cells (such as CHO, HeLa, MDCK, 293, W138, NIH-3T3 or HEK293) have specific cellular machinery and characteristic mechanisms for such post-translational activities and may be chosen to ensure the correct modification and processing of the ActRIIB polypeptides.

In certain aspects, functional variants or modified forms of the ActRIIB polypeptides include fusion proteins having at least a portion of the ActRIIB polypeptides and one or more fusion domains. Well known examples of such fusion domains include, but are not limited to, polyhistidine, Glu-Glu, glutathione S transferase (GST), thioredoxin, protein A, protein G, an immunoglobulin heavy chain constant region (Fc), maltose binding protein (MBP), or human serum albumin. A fusion domain may be selected so as to confer a desired property. For example, some fusion domains are particularly useful for isolation of the fusion proteins by affinity chromatography. For the purpose of affinity purification, relevant matrices for affinity chromatography, such as glutathione-, amylase-, and nickel- or cobalt-conjugated resins are used. Many of such matrices are available in "kit" form, such as the Pharmacia GST purification system and the QIAexpressTM system (Qiagen) useful with (HIS6) fusion partners. As another example, a fusion domain may be selected so as to facilitate detection of the ActRIIB polypeptides. Examples of such detection domains include the various fluorescent proteins (e.g., GFP) as well as "epitope tags," which are usually short peptide sequences for which a specific antibody is available. Well known epitope tags for which specific monoclonal antibodies are readily available include FLAG, influenza virus hemagglutinin (HA), and c-myc tags. In some cases, the fusion domains have a protease cleavage site, such as for Factor Xa or Thrombin, which allows the relevant protease to partially digest the fusion proteins and thereby liberate the recombinant proteins there from. The liberated proteins can then be isolated from the fusion domain by subsequent chromatographic separation. In certain preferred cases, an ActRIIB polypeptide is fused with a domain that stabilizes the ActRIIB polypeptide in vivo (a "stabilizer" domain). By "stabilizing" is meant anything that increases serum half life, regardless of whether this is because of decreased destruction, decreased clearance by the kidney, or other pharmacokinetic effect. Fusions with the Fc portion of an immunoglobulin are known to confer desirable pharmacokinetic properties on a wide range of proteins. Likewise, fusions to human serum albumin can confer desirable properties. Other types of fusion domains that may be selected include multimerizing (e.g., dimerizing, tetramerizing) domains and functional domains (that confer an additional biological function, such as further stimulation of bone growth or muscle growth, as desired).

It is understood that different elements of the fusion proteins may be arranged in any manner that is consistent with the desired functionality. For example, an ActRIIB polypeptide may be placed C-terminal to a heterologous domain, or, alternatively, a heterologous domain may be placed C-terminal to an ActRIIB polypeptide. The ActRIIB polypeptide domain and the heterologous domain need not be adjacent in a fusion protein, and additional domains or amino acid sequences may be included C- or N-terminal to either domain or between the domains.

In certain cases, the ActRIIB polypeptides used in the methods and compositions described herein contain one or more modifications that are capable of stabilizing the ActRIIB polypeptides. For example, such modifications enhance the in vitro half life of the ActRIIB polypeptides, enhance circulatory half life of the ActRIIB polypeptides or reduce proteolytic degradation of the ActRIIB polypeptides. Such stabilizing modifications include, but are not limited to, fusion proteins (including, for example, fusion proteins comprising an ActRIIB polypeptide and a stabilizer domain), modifications of a glycosylation site (including, for example, addition of a glycosylation site to an ActRIIB polypeptide), and modifications of carbohydrate moiety (including, for example, removal of carbohydrate moieties from an ActRIIB polypeptide). In the case of fusion proteins, an ActRIIB polypeptide is fused to a stabilizer domain such as an IgG molecule (e.g., an Fc domain). As used herein, the term "stabilizer domain" not only refers to a fusion domain (e.g., Fc) as in the case of fusion proteins, but also includes nonproteinaceous modifications such as a carbohydrate moiety, or nonproteinaceous polymer, such as polyethylene glycol.

In certain cases, the methods and compositions described herein use isolated or purified ActRIIB polypeptides, i.e., ActRIIB polypeptides which are isolated from, or otherwise substantially free of, other proteins can be used with the methods and compositions described herein. ActRIIB polypeptides will generally be produced by expression from recombinant nucleic acids.

In certain aspects, the ActRIIB polypeptides used in the methods and compositions described herein are encoded by isolated and/or recombinant nucleic acids, including fragments, functional variants and fusion proteins disclosed herein. For example, SEQ ID NO:6 encodes the naturally occurring human ActRIIB precursor polypeptide. The subject nucleic acids may be single-stranded or double stranded. Such nucleic acids may be DNA or RNA molecules. These nucleic acids may be used, for example, in methods for making ActRIIB polypeptides or as direct therapeutic agents (e.g., in a gene therapy approach).

In certain aspects, the nucleic acids that can be used to produce ActRIIB polypeptides suitable for use in the methods and compositions described herein are further understood to include nucleic acids that are variants of SEQ ID NO:6 as well as variants of those nucleic acid sequences that encode soluble ActRIIB polypeptides (e.g., nucleic acids that encode SEQ ID NOs: 4, 5, 9, 12, 13, 15, 16, 17, 18, 19, 22, 23, 28, and 29). Variant nucleotide sequences include sequences that differ by one or more nucleotide substitutions, additions or deletions, such as allelic variants.

In certain cases, the isolated or recombinant nucleic acid sequences that can be used to produce ActRIIB polypeptides suitable for use in the methods and compositions described herein are at least 80%, 85%, 90%, 95%, 97%, 98%, 99% or 100% identical to SEQ ID NO:6 or those nucleic acid sequences that encode soluble ActRIIB polypeptides (e.g., nucleic acids that encode SEQ ID NOs: 4, 5, 9, 12, 13, 15, 16, 17, 18, 19, 22, 23, 28, and 29). One of ordinary skill in the art will appreciate that nucleic acid sequences complementary to SEQ ID NO:6 or those nucleic acid sequences that encode soluble ActRIIB polypeptides (e.g., nucleic acids that encode SEQ ID NOs: 4, 5, 9, 12, 13, 15, 16, 17, 18, 19, 22, 23, 28, and 29), and variants of SEQ ID NO:6 or those nucleic acid sequences that encode soluble ActRIIB polypeptides (e.g., nucleic acids that encode SEQ ID NOs: 4, 5, 9, 12, 13, 15, 16, 17, 18, 19, 22, 23, 28, and 29) can be used with the methods and compositions described herein. In further embodiments, the nucleic acid sequences can be isolated, recombinant, and/or fused with a heterologous nucleotide sequence, or in a DNA library.

In other cases, nucleic acids that can be used to produce ActRIIB polypeptides suitable for use in the methods and compositions described herein include nucleotide sequences that hybridize under highly stringent conditions to the nucleotide sequence designated in SEQ ID NO:6 or those nucleic acid sequences that encode soluble ActRIIB polypeptides (e.g., nucleic acids that encode SEQ ID NOs: 4, 5, 9, 12, 13, 15, 16, 17, 18, 19, 22, 23, 28, and 29), complement sequence of SEQ ID NO:6 or those nucleic acid sequences that encode soluble ActRIIB polypeptides (e.g., nucleic acids that encode SEQ ID NOs: 4, 5, 9, 12, 13, 15, 16, 17, 18, 19, 22, 23, 28, and 29), or fragments thereof. One of ordinary skill in the art will understand that appropriate stringency conditions which promote DNA hybridization can be varied. For example, one can perform the hybridization at 6.0 times sodium chloride/sodium citrate (SSC) at about 45° Celsius, followed by a wash of 2.0 times SSC at 50° Celsius. For example, the salt concentration in the wash step can be selected from a low stringency of about 2.0 times SSC at 50° Celsius to a high stringency of about 0.2 times SSC at 50° Celsius. In addition, the temperature in the wash step can be increased from low stringency conditions at room temperature, about 22° Celsius, to high stringency conditions at about 65° Celsius. Both temperature and salt may be varied, or temperature or salt concentration may be held constant while the other variable is changed. In one embodiment, nucleic acids which hybridize under low stringency conditions of 6 times SSC at room temperature followed by a wash at 2 times SSC at room temperature can be used with the methods and compositions described herein.

Isolated nucleic acids which differ from the nucleic acids as set forth in SEQ ID NO:6 or those nucleic acid sequences that encode soluble ActRIIB polypeptides (e.g., nucleic acids that encode SEQ ID NOs: 4, 5, 9, 12, 13, 15, 16, 17, 18, 19, 22, 23, 28, and 29) due to degeneracy in the genetic code can also be used to produce ActRIIB polypeptides suitable for use in the methods and compositions described herein. For example, a number of amino acids are designated by more than one triplet. Codons that specify the same amino acid, or synonyms (for example, CAU and CAC are synonyms for histidine) may result in "silent" mutations which do not affect the amino acid sequence of the protein. However, it is expected that DNA sequence polymorphisms that do lead to changes in the amino acid sequences of the subject proteins will exist among mammalian cells. One skilled in the art will appreciate that these variations in one or more nucleotides (up to about 3-5% of the nucleotides) of the nucleic acids encoding a particular protein may exist among individuals of a given species due to natural allelic variation. Any and all such nucleotide variations and resulting amino acid polymorphisms can be used with the methods and compositions described herein.

In certain cases, the recombinant nucleic acids that can be used to produce ActRIIB polypeptides suitable for use in the methods and compositions described herein may be operably linked to one or more regulatory nucleotide sequences in an expression construct. Regulatory nucleotide sequences will generally be appropriate to the host cell used for expression. Numerous types of appropriate expression vectors and suitable regulatory sequences are known in the art for a variety of host cells. Typically, said one or more regulatory nucleotide sequences may include, but are not limited to, promoter sequences, leader or signal sequences, ribosomal binding sites, transcriptional start and termination sequences, translational start and termination sequences, and enhancer or activator sequences. Constitutive or inducible promoters as known in the art can be used with the methods and compositions described herein. The promoters may be either naturally occurring promoters, or hybrid promoters that combine elements of more than one promoter. An expression construct may be present in a cell on an episome, such as a plasmid, or the expression construct may be inserted in a chromosome. In a preferred embodiment, the expression vector contains a selectable marker gene to allow the selection of transformed host cells. Selectable marker genes are well known in the art and will vary with the host cell used.

In certain aspects, the nucleic acids that can be used to produce ActRIIB polypeptides suitable for use in the methods and compositions described herein are provided in an expression vector comprising a nucleotide sequence encoding an ActRIIB polypeptide and operably linked to at least one regulatory sequence. Regulatory sequences are art-recognized and are selected to direct expression of the ActRIIB polypeptide. Accordingly, the term regulatory sequence includes promoters, enhancers, and other expression control elements. Exemplary regulatory sequences are described in Goeddel; Gene Expression Technology: Methods in Enzymology, Academic Press, San Diego, Calif. (1990). For instance, any of a wide variety of expression control sequences that control the expression of a DNA sequence when operatively linked to it may be used in these vectors to express DNA sequences encoding an ActRIIB polypeptide. Such useful expression control sequences, include, for example, the early and late promoters of SV40, tet promoter, adenovirus or cytomegalovirus immediate early promoter, RSV promoters, the lac system, the trp system, the TAC or TRC system, T7 promoter whose expression is directed by T7 RNA polymerase, the major operator and promoter regions of phage lambda, the control regions for fd coat protein, the promoter for 3-phosphoglycerate kinase or other glycolytic enzymes, the promoters of acid phosphatase, e.g., Pho5, the promoters of the yeast alpha. - mating factors, the polyhedron promoter of the baculovirus system and other sequences known to control the expression of genes of prokaryotic or eukaryotic cells or their viruses, and various combinations thereof. It should be understood that the design of the expression vector may depend on such factors as the choice of the host cell to be transformed and/or the type of protein desired to be expressed. Moreover, the vector's copy number, the ability to control that copy number and the expression of any other protein encoded by the vector, such as antibiotic markers, should also be considered.

A recombinant nucleic acid can be produced by ligating the cloned gene, or a portion thereof, into a vector suitable for expression in either prokaryotic cells, eukaryotic cells (yeast, avian, insect or mammalian), or both. Expression vehicles for production of a recombinant ActRIIB polypeptide include plasmids and other vectors. For instance, suitable vectors include plasmids of the types: pBR322-derived plasmids, pEMBL-derived plasmids, pEX-derived plasmids, pBTac-derived plasmids and pUC-derived plasmids for expression in prokaryotic cells, such as E. coli.

Some mammalian expression vectors contain both prokaryotic sequences to facilitate the propagation of the vector in bacteria, and one or more eukaryotic transcription units that are expressed in eukaryotic cells. The pcDNAI/amp, pcDNAI/neo, pRc/CMV, pSV2gpt, pSV2neo, pSV2-dhfr, pTk2, pRSVneo, pMSG, pSVT7, pko-neo and pHyg derived vectors are examples of mammalian expression vectors suitable for transfection of eukaryotic cells. Some of these vectors are modified with sequences from bacterial plasmids, such as pBR322, to facilitate replication and drug resistance selection in both prokaryotic and eukaryotic cells. Alternatively, derivatives of viruses such as the bovine papilloma virus (BPV-1), or Epstein-Barr virus (pHEBo, pREP-derived and p205) can be used for transient expression of proteins in eukaryotic cells. Examples of other viral (including retroviral) expression systems can be found below in the description of gene therapy delivery systems. The various methods employed in the preparation of the plasmids and in transformation of host organisms are well known in the art. For other suitable expression systems for both prokaryotic and eukaryotic cells, as well as general recombinant procedures, see Molecular Cloning A Laboratory Manual, 3rd Ed., ed. by Sambrook, Fritsch and Maniatis (Cold Spring Harbor Laboratory Press, 2001). In some instances, it may be desirable to express the recombinant polypeptides by the use of a baculovirus expression system. Examples of such baculovirus expression systems include pVL-derived vectors (such as pVL1392, pVL 1393 and pVL941), pAcUW-derived vectors (such as pAcUW1), and pBlueBac-derived vectors (such as the .beta.-gal containing pBlueBac III).

In one embodiment, a vector can be designed for production of the ActRIIB polypeptides used in the methods and compositions described herein in CHO cells, such as a Pcmv-Script vector (Stratagene, La Jolla, Calif.), pcDNA4 vectors (Invitrogen, Carlsbad, Calif.) and pCI-neo vectors (Promega, Madison, Wis.). As will be apparent, the subject gene constructs can be used to cause expression of the subject ActRIIB polypeptides in cells propagated in culture, e.g., to produce proteins, including fusion proteins or variant proteins, for purification.

Host cells transfected with a recombinant gene including a coding sequence (e.g., SEQ ID NO:6 or those nucleic acid sequences that encode soluble ActRIIB polypeptides (e.g., nucleic acids that encode SEQ ID NOs: 4, 5, 9, 12, 13, 15, 16, 17, 18, 19, 22, 23, 28, and 29)) for one or more of the subject ActRIIB polypeptides can be used to produce ActRIIB polypeptides suitable for use in the methods and compositions described herein. The host cell may be any prokaryotic or eukaryotic cell. For example, an ActRIIB polypeptide may be expressed in bacterial cells such as E. coli, insect cells (e.g., using a baculovirus expression system), yeast, or mammalian cells. Other suitable host cells are known to those skilled in the art.

Accordingly, provided herein are methods of producing the ActRIIB polypeptides used in the methods and compositions described herein. For example, a host cell transfected with an expression vector encoding an ActRIIB polypeptide can be cultured under appropriate conditions to allow expression of the ActRIIB polypeptide to occur. The ActRIIB polypeptide may be secreted and isolated from a mixture of cells and medium containing the ActRIIB polypeptide. Alternatively, the ActRIIB polypeptide may be retained cytoplasmically or in a membrane fraction and the cells harvested, lysed and the protein isolated. A cell culture includes host cells, media and other byproducts. Suitable media for cell culture are well known in the art. The subject ActRIIB polypeptides can be isolated from cell culture medium, host cells, or both, using techniques known in the art for purifying proteins, including ion-exchange chromatography, gel filtration chromatography, ultrafiltration, electrophoresis, immunoaffinity purification with antibodies specific for particular epitopes of the ActRIIB polypeptides and affinity purification with an agent that binds to a domain fused to the ActRIIB polypeptide (e.g., a protein A column may be used to purify an ActRIIB-Fc fusion). In a preferred case, the ActRIIB polypeptide is a fusion protein containing a domain which facilitates its purification. In a preferred case, purification is achieved by a series of column chromatography steps, including, for example, three or more of the following, in any order: protein A chromatography, Q sepharose chromatography, phenylsepharose chromatography, size exclusion chromatography, and cation exchange chromatography. The purification could be completed with viral filtration and buffer exchange. As demonstrated herein, ActRIIB -hFc protein was purified to a purity of >98% as determined by size exclusion chromatography and >95% as determined by SDS PAGE. This level of purity was sufficient to achieve desirable effects on bone in mice and an acceptable safety profile in mice, rats and non-human primates.

In another case, a fusion gene coding for a purification leader sequence, such as a poly-(His)/enterokinase cleavage site sequence at the N-terminus of the desired portion of the recombinant ActRIIB polypeptide, can allow purification of the expressed fusion protein by affinity chromatography using a Ni2+ metal resin. The purification leader sequence can then be subsequently removed by treatment with enterokinase to provide the purified ActRIIB polypeptide (e.g., see Hochuli et al., (1987) J. Chromatography 411:177; and Janknecht et al., PNAS USA 88:8972).

Techniques for making fusion genes are well known. Essentially, the joining of various DNA fragments coding for different polypeptide sequences is performed in accordance with conventional techniques, employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed to generate a chimeric gene sequence (see, for example, Current Protocols in Molecular Biology, eds. Ausubel et al., John Wiley & Sons: 1992).

ActRIIB -Fc fusion protein can be expressed in stably transfected CHO-DUKX Bl 1 cells from a pAID4 vector (SV40 ori/enhancer, CMV promoter), using a tissue plasminogen leader sequence of SEQ ID NO:2. The Fc portion can comprise a human IgGl Fc sequence, as shown in SEQ ID NO: 1. In certain cases, upon expression, the protein contained has, on average, between about 1.5 and 2.5 moles of sialic acid per molecule of ActRIIB-Fc fusion protein.

In certain cases, the long serum half-life of an ActRIIB-Fc fusion can be 25-32 days in human subjects. Additionally, the CHO cell expressed material can have a higher affinity for activin B ligand than that reported for an ActRIIB-hFc fusion protein expressed in human 293 cells (del Re et al., J Biol Chem. 2004 Dec 17;279(51):53126-35). Additionally, without being bound by theory, the use of the TPA leader sequence provided greater production than other leader sequences and, unlike ActRIIB-Fc expressed with a native leader, may provide a highly pure N-terminal sequence. Use of the native leader sequence may result in two major species of ActRIIB-Fc, each having a different N-terminal sequence.

In a preferred embodiment, the ActRIIB signaling inhibitor is luspatercept *(see* International Nonproprietary Names for Pharmaceutical Substances (INN), WHO Drug Information, Vol. 27, No. 4, 2013, Proposed INN: List 110-458, 422).

### 5.5 COMPOSITIONS

In certain embodiments, ActRIIB signaling inhibitors are formulated with a pharmaceutically acceptable carrier for use with the methods described herein. For example, an ActRIIB signaling inhibitor can be administered alone or as a component of a pharmaceutical formulation (therapeutic composition). The subject compounds may be formulated for administration in any convenient way for use in human or veterinary medicine.

In a preferred embodiment, the ActRII signaling inhibitor is formulated for subcutaneous administration.

In another preferred embodiment, the ActRIIB signaling inhibitor is packaged in a container as a sterile, preservative-free lyophilized powder or cake. In certain embodiments, the container comprises 25 mg of the ActRIIB signaling inhibitor. In certain embodiments, the container comprising 25 mg of the ActRIIB signaling inhibitor comprises a total of 37.5 mg of protein. In certain embodiments, ActRIIB signaling inhibitor in the container comprising 25 mg of the ActRIIB signaling inhibitor is reconstituted with 0.68 mL of water for injection. In certain embodiments, the container comprises 75 mg of the ActRIIB signaling inhibitor. In certain embodiments, the container comprising 75 mg of the ActRIIB signaling inhibitor comprises a total of 87.5 mg of protein. In certain embodiments, ActRIIB signaling inhibitor in the container comprising 75 mg of the ActRIIB signaling inhibitor is reconstituted with 1.6 mL of water for injection. In certain embodiments, the ActRIIB signaling inhibitor in the container is reconstituted with a volume of water for injection, such that the final concentration of the reconstituted ActRIIB signaling inhibitor in the water for injection is 50 mg/mL with a pH of approximately 6.5. In certain embodiments, the ActRIIB signaling inhibitor is administered to a subject within 10 hours of reconstitution. In certain embodiments, the container comprises the ActRIIB signaling inhibitor at a concentration of 50 mg/mL in a 10 mM citrate buffer-based solution, wherein the 10 mM citrate-buffer based solution comprises 10 mM citrate, pH 6.5, 9% sucrose, and 0.02% polysorbate 80. In certain embodiments, the container is stored at between 2°C and 8°C. In certain embodiments, the container is stored at between 2°C and 8°C for 18 months. In certain embodiments, the container is a 3 mL glass vial with a gray butyl coated stopper. In certain embodiments, the container is a 3 mL glass vial with a gray rubber stopper. In certain embodiments, the rubber stopper is secured in place by a crimped aluminum flip cap with a colored plastic button. In certain embodiments, the 3 mL glass vial comprises 25 mg of the ActRIIB signaling inhibitor and the colored plastic button is red. In certain embodiments, 3 mL glass vial comprises 75 mg of the ActRIIB signaling inhibitor and the colored plastic button is white. In specific embodiments, the ActRIIB signaling inhibitor is a polypeptide comprising the amino acid sequence of SEQ ID NO:11. In specific embodiments, the ActRIIB signaling inhibitor is a polypeptide comprising an amino acid sequence consisting of the amino acid sequence of SEQ ID NO:11. In specific embodiments, the ActRIIB signaling inhibitor is a polypeptide consisting of the amino acid sequence set forth in SEQ ID NO:11.

In a specific embodiment, the ActRIIB signaling inhibitor is packaged in a container as a sterile, preservative-free lyophilized powder or cake. In a specific embodiment, the container comprises 50 mg/mL of ActRIIB signaling inhibitor in 10 mM citrate buffer pH 6.5. In a specific embodiment, the container comprises 56 mg of ActRIIB signaling inhibitor, 0 19 mg of citric acid monohydrate, 3.03 mg of tri-sodium citrate dehydrate, 0.24 mg of polysorbate 80, and 100.80 mg of sucrose. In specific embodiments, the ActRIIB signaling inhibitor is a polypeptide comprising the amino acid sequence of SEQ ID NO:11. In specific embodiments, the ActRIIB signaling inhibitor is a polypeptide comprising an amino acid sequence consisting of the amino acid sequence of SEQ ID NO:11. In specific embodiments, the ActRIIB signaling inhibitor is a polypeptide consisting of the amino acid sequence set forth in SEQ ID NO:11.

In certain embodiments, the therapeutic methods provided herein include administering the composition (comprising an ActRIIB signaling inhibitor) systemically, or locally as an implant or device. When administered, the therapeutic composition for uses provided herein is in a pyrogen-free, physiologically acceptable form. Therapeutically useful agents other than the ActRIIB signaling inhibitors which may also optionally be included in the composition as described above, may be administered simultaneously or sequentially with the subject compounds (e.g., ActRII polypeptides, such as ActRIIA and / or ActRIIB polypeptides).

Typically, ActRIIB signaling inhibitors will be administered parenterally. In a preferred embodiment, the ActRIIB signaling inhibitor will be administered subcutaneously. Pharmaceutical compositions suitable for parenteral administration may comprise one or more ActRIIB polypeptides in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents. Examples of suitable aqueous and nonaqueous carriers which may be employed in the pharmaceutical compositions for use in the methods described herein include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

The compositions described herein may also contain adjuvants, such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption, such as aluminum monostearate and gelatin.

In certain embodiments, the ActRIIB signaling inhibitor is substantially pure in a pharmaceutical composition. Specifically, at most 20%, 10%, 5%, 2.5%, 1%, 0.1%, or at most 0.05% of the compounds in the pharmaceutical composition are compounds other than the ActRIIB signaling inhibitor and the pharmaceutical acceptable carrier.

In certain embodiments, the ActRIIB signaling inhibitor is administered at room temperature to a patient according to a method provided herein.

### 5.6 DOSES AND ROUTES OF ADMINISTRATION

Also provided herein are doses of an ActRIIB signaling inhibitor for administering to a subject in connection with a method of treatment described herein (see, e.g., Section 5.2). In certain embodiments the dose is a pharmaceutically effective dose.

In certain embodiments, the pharmaceutically effective dose of the ActRII signaling inhibitor is a dose sufficient to reduce or palliate one or more symptoms of myeloproliferative neoplasm-associated myelofibrosis. In certain embodiments, the pharmaceutically effective dose of the ActRII signaling inhibitor is a dose sufficient to prevent at least one symptom of myeloproliferative neoplasm-associated myelofibrosis from worsening. Non-limiting examples of myeloproliferative neoplasm-associated myelofibrosis include fatigue, night sweats, itchiness, abdominal discomfort, pain under the ribs on the left side, early satiety, or bone pain.

In certain embodiments, the pharmaceutically effective dose of the ActRII signaling inhibitor is a dose sufficient to reduce or palliate one or more symptoms of anemia caused by or associated with myeloproliferative neoplasm-associated myelofibrosis. In certain embodiments, the pharmaceutically effective dose of the ActRII signaling inhibitor is a dose sufficient to prevent at least one symptom of anemia from worsening. Non-limiting examples of anemia include fatigue, loss of energy, rapid heartbeat, shortness of breath, headaches, difficulty concentrating, dizziness, pale skin, leg cramps, and insomnia.

In certain embodiments, the pharmaceutically effective dose of the ActRII signaling inhibitor is a dose sufficient to reduce or palliate one or more symptoms of anemia and one or more symptoms of myeloproliferative neoplasm-associated myelofibrosis. In certain embodiments, the pharmaceutically effective dose of the ActRII signaling inhibitor is a dose sufficient to prevent at least one symptom of anemia from worsening and at least one symptom of myeloproliferative neoplasm-associated myelofibrosis from worsening. Non-limiting examples of anemia include fatigue, loss of energy, rapid heartbeat, shortness of breath, headaches, difficulty concentrating, dizziness, pale skin, leg cramps, and insomnia. Non-limiting examples of myeloproliferative neoplasm-associated myelofibrosis include fatigue, night sweats, itchiness, abdominal discomfort, pain under the ribs on the left side, early satiety, or bone pain.

In certain embodiments, the ActRIIB signaling inhibitor is dosed at intervals and amounts sufficient to achieve serum concentrations of 0.2 mg/kg or greater, for example, serum levels of 1 mg/kg or 2 mg/kg or greater. Dosing regimens may be designed to reach serum concentrations of between 0.2 and 15 mg/kg, and optionally between 1 and 5 mg/kg. In humans, serum levels of 0.2 mg/kg may be achieved with a single dose of 0.1 mg/kg or greater and serum levels of 1 mg/kg may be achieved with a single dose of 0.3 mg/kg or greater. The observed serum half-life of the molecule is between about 20 and 30 days, substantially longer than most Fc fusion proteins, and thus a sustained effective serum level may be achieved, for example, by dosing with 0.2-0.4 mg/kg on a weekly or biweekly basis, or higher doses may be used with longer intervals between dosings. For example, doses of 1-3 mg/kg might be used on a monthly or bimonthly basis, and the effect on bone may be sufficiently durable that dosing is necessary only once every 3, 4, 5, 6, 9, 12 or more months. Serum levels of the ActRIIB signaling inhibitor can be measured by any means known to the skilled artisan. For example, antibodies against the ActRIIB signaling inhibitor can be used to determine the serum levels of the ActRIIB signaling inhibitor using, e.g., an ELISA. In a specific embodiment, the method provided herein also achieves significant effects on bone density and strength.

In certain embodiments, the dose of the ActRIIB signaling inhibitor is about 0.1 mg/kg, about 0.3 mg/kg, about 0.45 mg/kg, about 0.5 mg/kg, about 0.6 mg/kg, 0.75 mg/kg, about 0.8 mg/kg, about 1.0 mg/kg, about 1.25 mg/kg, about 1.33 mg/kg, about 1.5 mg/kg, about 1.75 mg/kg, about 2.0 mg/kg, or about 2.25 mg/kg. In certain embodiments, the dose of the ActRIIB signaling inhibitor is between 0.1 mg/kg and 2.25 mg/kg. In certain embodiments, the dose of the ActRIIB signaling inhibitor is between 0.1 mg/kg and 1 mg/kg. In certain embodiments, the dose of the ActRIIB signaling inhibitor is between 0.3 mg/kg and 1.25 mg/kg. In certain embodiments, the dose of the ActRIIB signaling inhibitor is between 0.5 mg/kg and 1.5 mg/kg. In certain embodiments, the dose of the ActRIIB signaling inhibitor is between 0.1 mg/kg and 2.0 mg/kg. In certain embodiments, the dose of the ActRIIB signaling inhibitor is between 0.33 mg/kg and 2.0 mg/kg. In certain embodiments, the dose of the ActRIIB signaling inhibitor is between 0.75 mg/kg and 1.0 mg/kg, between 1.0 mg/kg and 1.25 mg/kg, between 1.25 mg/kg and 1.5 mg/kg, between 1.5 mg/kg and 1.75 mg/kg, or between 1.75 mg/kg and 2.0 mg/kg. In certain embodiments, the dose of the ActRIIB signaling inhibitor is a pharmaceutically effective dose. In a specific embodiment, the dose of the ActRIIB signaling inhibitor is 0.33 mg/kg. In a specific embodiment, the dose of the ActRIIB signaling inhibitor is 0.45 mg/kg. In a specific embodiment, the dose of the ActRIIB signaling inhibitor is 0.6 mg/kg. In a specific embodiment, the dose of the ActRIIB signaling inhibitor is 0.8 mg/kg. In a specific embodiment, the dose of the ActRIIB signaling inhibitor is 1.0 mg/kg. In a specific embodiment, the dose of the ActRIIB signaling inhibitor is 1.33 mg/kg. In a specific embodiment, the dose of the ActRIIB signaling inhibitor is 1.75 mg/kg. In a specific embodiment, the dose of the ActRIIB signaling inhibitor is 2.00 mg/kg. When used in conjunction with a dose provided herein (e.g., a dose of an ActRIIB signaling inhibitor or a dose of a second active agent), the word "about" refers to any number within 1, 5 or 10% of the referenced number.

In certain embodiments, the dose of the ActRIIB signaling inhibitor is a pharmaceutically effective dose. In certain embodiments, the pharmaceutically effective dose of the ActRIIB signaling inhibitor is about 0.1 mg/kg, about 0.3 mg/kg, about 0.45 mg/kg, about 0.5 mg/kg, about 0.6 mg/kg, 0.75 mg/kg, about 0.8 mg/kg, about 1.0 mg/kg, about 1.25 mg/kg, about 1.33 mg/kg, about 1.5 mg/kg, about 1.75 mg/kg, about 2.0 mg/kg, or about 2.25 mg/kg. In certain embodiments, the pharmaceutically effective dose of the ActRIIB signaling inhibitor is between 0.1 mg/kg and 2.25 mg/kg. In certain embodiments, the pharmaceutically effective dose of the ActRIIB signaling inhibitor is between 0.1 mg/kg and 1 mg/kg. In certain embodiments, the pharmaceutically effective dose of the ActRIIB signaling inhibitor is between 0.3 mg/kg and 1.25 mg/kg. In certain embodiments, the pharmaceutically effective dose of the ActRIIB signaling inhibitor is between 0.5 mg/kg and 1.5 mg/kg. In certain embodiments, the pharmaceutically effective dose of the ActRIIB signaling inhibitor is between 0.1 mg/kg and 2.0 mg/kg. In certain embodiments, the pharmaceutically effective dose of the ActRIIB signaling inhibitor is between 0.33 mg/kg and 2.0 mg/kg. In certain embodiments, the pharmaceutically effective dose of the ActRIIB signaling inhibitor is between 0.75 mg/kg and 1.0 mg/kg, between 1.0 mg/kg and 1.25 mg/kg, between 1.25 mg/kg and 1.5 mg/kg, between 1.5 mg/kg and 1.75 mg/kg, or between 1.75 mg/kg and 2.0 mg/kg. In a specific embodiment, the dose of the ActRIIB signaling inhibitor is 0.33 mg/kg. In a specific embodiment, the dose of the ActRIIB signaling inhibitor is 0.45 mg/kg. In a specific embodiment, the dose of the ActRIIB signaling inhibitor is 0.6 mg/kg. In a specific embodiment, the dose of the ActRIIB signaling inhibitor is 0.8 mg/kg. In a specific embodiment, the dose of the ActRIIB signaling inhibitor is 1.0 mg/kg. In a specific embodiment, the dose of the ActRIIB signaling inhibitor is 1.33 mg/kg. In a specific embodiment, the dose of the ActRIIB signaling inhibitor is 1.75 mg/kg. In a specific embodiment, the dose of the ActRIIB signaling inhibitor is 2.00 mg/kg.

In certain embodiments, the dose of an ActRIIB signaling inhibitor administered to a subject according to the methods provided herein is sufficient to increase the level of hemoglobin in the subject as compared to the level of hemoglobin in the subject at baseline. In the context of evaluating the increase in the level of hemoglobin in the subject, "baseline" refers to the time immediately prior to the first administration of the ActRIIB signaling inhibitor to the subject. In certain embodiments, the dose of an ActRIIB signaling inhibitor administered to a subject according to the methods provided herein is sufficient to increase the level of hemoglobin in the subject by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100% over any consecutive 84-day period after the subject has been administered a first dose of the ActRIIB signaling inhibitor, wherein the subject has been transfused 0 units of red blood cell units during the consecutive 84-day period, as compared to the level of hemoglobin in the subject at baseline. In certain embodiments, the dose of an ActRIIB signaling inhibitor administered to a subject according to the methods provided herein is sufficient to increase the level of hemoglobin in the subject by at most 5%, at most 10%, at most 15%, at most 20%, at most 25%, at most 30%, at most 35%, at most 40%, at most 45%, at most 50%, at most 55%, at most 60%, at most 65%, at most 70%, at most 75%, at most 80%, at most 85%, at most 90%, at most 95%, or at most 100% over any consecutive 84-day period after the subject has been administered a first dose of the ActRIIB signaling inhibitor, wherein the subject has been transfused 0 units of red blood cell units during the consecutive 84-day period, as compared to the level of hemoglobin in the subject at baseline. In certain embodiments, the dose of an ActRIIB signaling inhibitor administered to a subject according to the methods provided herein is sufficient to increase the level of hemoglobin by at least 1.5 g/dL, at least 1.8 g g/dL, at least 2.0 g/dL, at least 2.2 g/dL, at least 2.4 g/dL, at least 2.6 g/dL, at least 2.8 g/dL, at least 3.0 g/dL, at least 3.2 g/dL, at least 3.4 g/dL, at least 3.6 g/dL, at least 3.8 g/dL, at least 4.0 g/dL, at least 5.0 g/dL, or at least 6.0 g/dL over any consecutive 84-day period after the subject has been administered a first dose of the ActRIIB signaling inhibitor, wherein the subject has been transfused 0 units of red blood cell units during the consecutive 84-day period, as compared to the level of hemoglobin in the subject at baseline. In certain embodiments, the dose of an ActRIIB signaling inhibitor administered to a subject according to the methods provided herein is sufficient to increase the level of hemoglobin by at most 1.5 g/dL, at most 1.8 g g/dL, at most 2.0 g/dL, at most 2.2 g/dL, at most 2.4 g/dL, at most 2.6 g/dL, at most 2.8 g/dL, at most 3.0 g/dL, at most 3.2 g/dL, at most 3.4 g/dL, at most 3.6 g/dL, at most 3.8 g/dL, at most 4.0 g/dL, at most 5.0 g/dL, or at most 6.0 g/dL over any consecutive 84-day period after the subject has been administered a first dose of the ActRIIB signaling inhibitor, wherein the subject has been transfused 0 units of red blood cell units during the consecutive 84-day period, as compared to the level of hemoglobin in the subject at baseline. In certain embodiments, the dose of an ActRIIB signaling inhibitor administered to a subject according to the methods provided herein is sufficient to result in the increase in hemoglobin level in the subject for at least 3, at least 4, at least 5, at least 6, at least 12, at least 18, at least 24 or at least 48 months after administration of the ActRIIB signaling inhibitor. In certain embodiments, the dose of an ActRIIB signaling inhibitor administered to a subject according to the methods provided herein is sufficient to result in the increase in hemoglobin level in the subject indefinitely after administration of the ActRIIB signaling inhibitor. In certain embodiments, the dose of the ActRIIB signaling inhibitor is between 0.33 mg/kg and 2.0 mg/kg. In a specific embodiment, the dose of the ActRIIB signaling inhibitor is 0.33 mg/kg. In a specific embodiment, the dose of the ActRIIB signaling inhibitor is 0.45 mg/kg. In a specific embodiment, the dose of the ActRIIB signaling inhibitor is 0.6 mg/kg. In a specific embodiment, the dose of the ActRIIB signaling inhibitor is 0.8 mg/kg. In a specific embodiment, the dose of the ActRIIB signaling inhibitor is 1.0 mg/kg. In a specific embodiment, the dose of the ActRIIB signaling inhibitor is 1.33 mg/kg. In a specific embodiment, the dose of the ActRIIB signaling inhibitor is 1.75 mg/kg. In a specific embodiment, the dose of the ActRIIB signaling inhibitor is 2.00 mg/kg. In certain embodiments, the ActRIIB signaling inhibitor is an ActRIIB signaling inhibitor described in Section 5.4. In specific embodiments, the ActRIIB signaling inhibitor is a polypeptide comprising the amino acid sequence of SEQ ID NO:11. In specific embodiments, the ActRIIB signaling inhibitor is a polypeptide comprising an amino acid sequence consisting of the amino acid sequence of SEQ ID NO:11. In specific embodiments, the ActRIIB signaling inhibitor is a polypeptide consisting of the amino acid sequence set forth in SEQ ID NO:11. In a preferred embodiment, the ActRIIB signaling inhibitor is luspatercept.

In certain embodiments in which the subject is red blood cell transfusion dependent, the dose of an ActRIIB signaling inhibitor administered to a subject according to the methods provided herein is sufficient to result in red blood cell transfusion independence in the subject over any consecutive 84-day period after the subject has been administered a first dose of the ActRIIB signaling inhibitor. In certain embodiments, a subject is red blood cell transfusion-independent if the subject has received 0 red blood cell units during any consecutive 60-day, 61-day, 62-day, 63-day, 64-day, 65-day, 66-day, 67-day, 68-day, 69-day, 70-day, 71-day, 72-day, 73-day, 74-day, 75-day, 76-day, 77-day, 78-day, 79-day, 80-day, 81-day, 82-day, 83-day, 84-day, 85-day, 86-day, 87-day, 88-day, 89-day, or 90-day period. In certain embodiments, a subject is red blood cell transfusion-dependent if the subject has received an average red blood cell transfusion frequency of 2 to 4 red blood cell units per 28 days during a period of time of at least 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90 days prior to a first administration of the ActRIIB signaling inhibitor to the subject. In certain embodiments, the dose of an ActRIIB signaling inhibitor administered to a subject according to the methods provided herein is sufficient to result in red blood cell transfusion independence in the subject for at least 3, at least 4, at least 5, at least 6, at least 12, at least 18, at least 24 or at least 48 months after administration of the ActRIIB signaling inhibitor. In certain embodiments, the dose of an ActRIIB signaling inhibitor administered to a subject according to the methods provided herein is sufficient to result in red blood cell transfusion independence in the subject indefinitely after administration of the ActRIIB signaling inhibitor. In certain embodiments, the dose of the ActRIIB signaling inhibitor is between 0.33 mg/kg and 2.0 mg/kg. In a specific embodiment, the dose of the ActRIIB signaling inhibitor is 0.33 mg/kg. In a specific embodiment, the dose of the ActRIIB signaling inhibitor is 0.45 mg/kg. In a specific embodiment, the dose of the ActRIIB signaling inhibitor is 0.6 mg/kg. In a specific embodiment, the dose of the ActRIIB signaling inhibitor is 0.8 mg/kg. In a specific embodiment, the dose of the ActRIIB signaling inhibitor is 1.0 mg/kg. In a specific embodiment, the dose of the ActRIIB signaling inhibitor is 1.33 mg/kg. In a specific embodiment, the dose of the ActRIIB signaling inhibitor is 1.75 mg/kg. In a specific embodiment, the dose of the ActRIIB signaling inhibitor is 2.00 mg/kg. In certain embodiments, the ActRIIB signaling inhibitor is an ActRIIB signaling inhibitor described in Section 5.4. In specific embodiments, the ActRIIB signaling inhibitor is a polypeptide comprising the amino acid sequence of SEQ ID NO: 11. In specific embodiments, the ActRIIB signaling inhibitor is a polypeptide comprising an amino acid sequence consisting of the amino acid sequence of SEQ ID NO: 11. In specific embodiments, the ActRIIB signaling inhibitor is a polypeptide consisting of the amino acid sequence set forth in SEQ ID NO:11. In a preferred embodiment, the ActRIIB signaling inhibitor is luspatercept.

In certain embodiments, the dose of an ActRIIB signaling inhibitor administered to a subject according to the methods provided herein is sufficient to decrease the frequency of red blood cell transfusion in the subject as compared to the frequency of red blood cell transfusion in the subject at baseline. In the context of evaluating the decrease in the frequency of red blood cell transfusion in the subject, "baseline" refers to the time immediately prior to the first administration of the ActRIIB signaling inhibitor to the subject. To evaluate the decrease in the frequency of red blood cell transfusion in the subject as compared to the frequency of red blood cell transfusion in the subject at baseline, the mean number of red blood cell units transfused in the subject per a four week period after the subject has been administered a first dose of the ActRIIB signaling inhibitor is compared to the mean number of red blood cell units transfused in the subject per a four week period at baseline. In certain embodiments, the dose of an ActRIIB signaling inhibitor administered to a subject treated according to the methods provided herein is sufficient to reduce the mean number of red blood cell units transfused in the subject per a four week period after the subject has been administered a first dose of the ActRIIB signaling inhibitor by at least 10%, at least 20%, at least 30%, at least 40%, at least 50%, at least 60%, at least 70%, at least 80%, at least 90%, or by at least 100% as compared to the mean number of red blood cell units transfused in the subject per a four week period at baseline. In certain embodiments, the dose of an ActRIIB signaling inhibitor administered to a subject treated according to the methods provided herein is sufficient to reduce the mean number of red blood cell units transfused in the subject per a four week period after the subject has been administered a first dose of the ActRIIB signaling inhibitor by at most 10%, at most 20%, at most 30%, at most 40%, at most 50%, at most 60%, at most 70%, at most 80%, at most 90%, or by at most 100% as compared to the mean number of red blood cell units transfused in the subject per a four week period at baseline. In certain embodiments, the dose of an ActRIIB signaling inhibitor administered to a subject treated according to the methods provided herein is sufficient to reduce the mean number of red blood cell units transfused in the subject per a four week period after the subject has been administered a first dose of the ActRIIB signaling inhibitor by at least 1, at least 2, at least 3, at least 4, at least 5, at least 6, at least 7, or at least 8 units as compared to the mean number of red blood cell units transfused in the subject per a four week period at baseline. In certain embodiments, the dose of an ActRIIB signaling inhibitor administered to a subject treated according to the methods provided herein is sufficient to reduce the mean number of red blood cell units transfused in the subject per a four week period after the subject has been administered a first dose of the ActRIIB signaling inhibitor by at most 1, at most 2, at most 3, at most 4, at most 5, at most 6, at most 7, or at most 8 units as compared to the mean number of red blood cell units transfused in the subject per a four week period at baseline. In certain embodiments, one RBC unit refers to about 150 mL, 200 mL, 250 mL, 300 mL, 350 mL, 100-200 mL, 150-250 mL, 200-300 mL, 250-300 mL, or 250-350 mL of RBCs. In certain embodiments, the dose of the ActRIIB signaling inhibitor is between 0.33 mg/kg and 2.0 mg/kg. In a specific embodiment, the dose of the ActRIIB signaling inhibitor is 0.33 mg/kg. In a specific embodiment, the dose of the ActRIIB signaling inhibitor is 0.45 mg/kg. In a specific embodiment, the dose of the ActRIIB signaling inhibitor is 0.6 mg/kg. In a specific embodiment, the dose of the ActRIIB signaling inhibitor is 0.8 mg/kg. In a specific embodiment, the dose of the ActRIIB signaling inhibitor is 1.0 mg/kg. In a specific embodiment, the dose of the ActRIIB signaling inhibitor is 1.33 mg/kg. In a specific embodiment, the dose of the ActRIIB signaling inhibitor is 1.75 mg/kg. In a specific embodiment, the dose of the ActRIIB signaling inhibitor is 2.00 mg/kg. In certain embodiments, the ActRIIB signaling inhibitor is an ActRIIB signaling inhibitor described in Section 5.4. In specific embodiments, the ActRIIB signaling inhibitor is a polypeptide comprising the amino acid sequence of SEQ ID NO:11. In specific embodiments, the ActRIIB signaling inhibitor is a polypeptide comprising an amino acid sequence consisting of the amino acid sequence of SEQ ID NO:11. In specific embodiments, the ActRIIB signaling inhibitor is a polypeptide consisting of the amino acid sequence set forth in SEQ ID NO:11. In a preferred embodiment, the ActRIIB signaling inhibitor is luspatercept.

In certain embodiments, the dose of an ActRIIB signaling inhibitor administered to a subject according to the methods provided herein is sufficient to decrease transfusion burden in the subject by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100% over any period of time after the subject has been administered a first dose of the ActRIIB signaling inhibitor as compared to the transfusion burden in the subject at baseline. In the context of evaluating the decrease in transfusion burden in the subject, "baseline" refers to the time immediately prior to the first administration of the ActRIIB signaling inhibitor to the subject. In certain embodiments, the dose of an ActRIIB signaling inhibitor administered to a subject according to the methods provided herein is sufficient to decrease transfusion burden in the subject by at most 5%, at most 10%, at most 15%, at most 20%, at most 25%, at most 30%, at most 35%, at most 40%, at most 45%, at most 50%, at most 55%, at most 60%, at most 65%, at most 70%, at most 75%, at most 80%, at most 85%, at most 90%, at most 95%, or at most 100% over any period of time after the subject has been administered a first dose of the ActRIIB signaling inhibitor as compared to the transfusion burden in the subject at baseline. In certain embodiments, the period of time over which the transfusion burden is decreased in the subject is at least 4, at least 5, at least 6, at least 7, at least 8, at least 9, at least 10, at least 11, or at least 12 weeks. In certain embodiments, the period of time over which the transfusion burden is decreased in the subject is 12 weeks (i.e., 84 days). In certain embodiments, the dose of an ActRIIB signaling inhibitor administered to a subject according to the methods provided herein is sufficient to decrease transfusion burden in the subject by at least 50% over any consecutive 84-day period after the subject has been administered a first dose of the ActRIIB signaling inhibitor as compared to the transfusion burden in the subject at baseline. In certain embodiments, the dose of an ActRIIB signaling inhibitor administered to a subject according to the methods provided herein is sufficient to decrease the transfusion burden in the subject for at least 3, at least 4, at least 5, at least 6, at least 12, at least 18, at least 24 or at least 48 months after administration of the ActRIIB signaling inhibitor. In certain embodiments, the dose of an ActRIIB signaling inhibitor administered to a subject according to the methods provided herein is sufficient to decrease the transfusion burden in the subject indefinitely after administration of the ActRIIB signaling inhibitor. In certain embodiments, the dose of the ActRIIB signaling inhibitor is between 0.33 mg/kg and 2.0 mg/kg. In a specific embodiment, the dose of the ActRIIB signaling inhibitor is 0.33 mg/kg. In a specific embodiment, the dose of the ActRIIB signaling inhibitor is 0.45 mg/kg. In a specific embodiment, the dose of the ActRIIB signaling inhibitor is 0.6 mg/kg. In a specific embodiment, the dose of the ActRIIB signaling inhibitor is 0.8 mg/kg. In a specific embodiment, the dose of the ActRIIB signaling inhibitor is 1.0 mg/kg. In a specific embodiment, the dose of the ActRIIB signaling inhibitor is 1.33 mg/kg. In a specific embodiment, the dose of the ActRIIB signaling inhibitor is 1.75 mg/kg. In a specific embodiment, the dose of the ActRIIB signaling inhibitor is 2.00 mg/kg. In certain embodiments, the ActRIIB signaling inhibitor is an ActRIIB signaling inhibitor described in Section 5.4. In specific embodiments, the ActRIIB signaling inhibitor is a polypeptide comprising the amino acid sequence of SEQ ID NO:11. In specific embodiments, the ActRIIB signaling inhibitor is a polypeptide comprising an amino acid sequence consisting of the amino acid sequence of SEQ ID NO:11. In specific embodiments, the ActRIIB signaling inhibitor is a polypeptide consisting of the amino acid sequence set forth in SEQ ID NO:11. In a preferred embodiment, the ActRIIB signaling inhibitor is luspatercept.

In certain embodiments, anemia response is measured. In some embodiments anemia response is measured as it relates to hemoglobin (Hgb) increase, *e*.*g*., proportion of subjects achieving ≥ 1.5 g/dL hemoglobin increase from baseline over any consecutive 84-day period without an RBC transfusion (*e*.*g*., in subjects receiving luspatercept). In some embodiments anemia response is measured as it relates to increased red blood cell (RBC)-transfusion independence, *e*.*g*., proportion of subjects who become RBC-transfusion free over any consecutive 84-day period (*e.g.,* in subjects receiving luspatercept). In some embodiments anemia response is measured as it relates to time to anemia response, *e*.*g*., time from first luspatercept dose to first onset of anemia response (*e*.*g*., in subjects receiving luspatercept). In some embodiments anemia response is measured as it relates to duration of anemia response, *e.g.,* the maximum duration of anemia response in subjects (*e.g.,* in subjects receiving luspatercept). In some embodiments anemia response is measured as it relates to frequency of RBC transfusions, *e*.*g*., the mean number of RBC units transfused per subject per four weeks (*e.g.,* in subjects receiving luspatercept). In some embodiments anemia response is measured as it relates to frequency of RBC transfusion dependence, *e*.*g*., proportion of RBC transfusion dependent subjects who reduce their transfusion burden by ≥ 50% from baseline over any consecutive 84-day period (*e.g.,* in subjects receiving luspatercept). In some embodiments anemia response is measured as it relates to proportion of subjects who achieve a reduction in fatigue symptom as recorded and assessed via the modified Myeloproliferative Neoplasm Symptom Assessment Form (MPN-SAF), *(see* Emanual et "Myeloproliferative Neoplasm (MPN) Symptom Assessment Form Total Symptom Score: Prospective International Assessment of an Abbreviated Symptom Burden Scoring System Among Patients With MPNs," J. Clin. Oncol. 30(33):4098-4103 (2012)), *e.g.,* measuring fatigue, night sweats, itchiness, abdominal discomfort, pain under the ribs on the left side, early satiety, and bone pain (*e.g.,* in subjects receiving luspatercept; e.g., in subjects who achieve ≥ 50% reduction in fatigue symptom as measured by the MPN-SAF. In some embodiments anemia response is measured as it relates to the proportion of subjects who achieve ≥ 50% reduction in total symptom score (TSS) as recorded and assessed via the modified Myeloproliferative Neoplasm Symptom Assessment Form (MPN-SAF), (see Emanual et αl., "Myeloproliferative Neoplasm (MPN) Symptom Assessment Form Total Symptom Score: Prospective International Assessment of an Abbreviated Symptom Burden Scoring System Among Patients With MPNs," J. Clin. Oncol. 30(33):4098-4103 (2012), e.g., measuring fatigue, night sweats, itchiness, abdominal discomfort, pain under the ribs on the left side, early satiety, and bone pain *(e.g.,* in subjects receiving luspatercept). In some embodiments anemia response is measured as it relates to Health-related quality of life (HRQoL) measures, e.g., measure mean changes in HRQoL questionnaire domain scores compared to baseline scores *(e.g.,* in subjects receiving luspatercept). In some embodiments anemia response is measured as it relates to EQ-5D-5L questionnaires scores (EuroQol, Rotterdam, The Netherlands), e.g., measuring mean changes in EQ-5D-5L questionnaire domain scores compared to baseline scores *(e.g.,* in subjects receiving luspatercept). In some embodiments anemia response is measured as it relates to adverse events (AEs) *e.g.,* the type, frequency, and severity, of adverse events (*e.g.,* in subjects receiving luspatercept). In some embodiments anemia response is measured using antidrug antibodies (ADA) (*e*.*g*., frequency of antidrug antibodies and effects on efficacy, safety, or pharmacokinetics in subjects receiving luspatercept). In some embodiments anemia response is measured using pharmacokinetic parameters, *e.g*., plasma concentration-time curve, area under the curve (AUC), Cmax (*e*.*g*., for subjects receiving luspatercept).

In certain embodiments, the dose of the ActRIIB signaling inhibitor is administered via injection. In certain embodiments, the dose of the ActRIIB signaling inhibitor is administered subcutaneously. In certain embodiments, the dose of the ActRIIB signaling inhibitor is administered once every 3 weeks. In specific embodiments, the ActRIIB signaling inhibitor is a polypeptide comprising the amino acid sequence of SEQ ID NO:11. In specific embodiments, the ActRIIB signaling inhibitor is a polypeptide comprising an amino acid sequence consisting of the amino acid sequence of SEQ ID NO:11. In specific embodiments, the ActRIIB signaling inhibitor is a polypeptide consisting of the amino acid sequence set forth in SEQ ID NO:11. In a preferred embodiment, the ActRIIB signaling inhibitor is luspatercept. Assays

Various ActRIIB polypeptide variants, or soluble ActRIIB polypeptide variants, may be tested for their ability to inhibit ActRIIB signaling. In addition, compounds can be tested for their ability to inhibit ActRIIB signaling. Once activity of inhibitors of ActRIIB signaling is confirmed, these compounds can be used with the methods provided herein. The assays below are described for ActRIIA but can be performed analogously for ActRIIB.

For example, the effect of an ActRIIA polypeptide variant on the expression of genes involved in bone production or bone destruction may be assessed. This may, as needed, be performed in the presence of one or more recombinant ActRIIA ligand proteins (e.g., activin), and cells may be transfected so as to produce an ActRIIA polypeptide and/or variants thereof, and optionally, an ActRIIA ligand. Likewise, an ActRIIA polypeptide may be administered to a mouse or other animal, and one or more bone properties, such as density or volume may be assessed. The healing rate for bone fractures may also be evaluated. Dual-energy x-ray absorptiometry (DEXA) is a well-established, non-invasive, quantitative technique for assessing bone density in an animal. In humans central DEXA systems may be used to evaluate bone density in the spine and pelvis. These are the best predictors of overall bone density. Peripheral DEXA systems may be used to evaluate bone density in peripheral bones, including, for example, the bones of the hand, wrist, ankle and foot. Traditional x-ray imaging systems, including CAT scans, may be used to evaluate bone growth and fracture healing. In addition, bone density can be measured using quantitative computed tomography (qCT). The mechanical strength of bone may also be evaluated.

In certain aspects, provided herein is the use of ActRIIA polypeptides (e.g., soluble ActRIIA polypeptides) and activin polypeptides to identify compounds (agents) which are agonist or antagonists of the activin-ActRIIA signaling pathway. Compounds identified through this screening can be tested to assess their ability to modulate bone growth or mineralization in vitro. Optionally, these compounds can further be tested in animal models to assess their ability to modulate tissue growth in vivo.

There are numerous approaches to screening for therapeutic agents for modulating tissue growth by targeting activin and ActRIIA polypeptides. In certain embodiments, high-throughput screening of compounds can be carried out to identify agents that perturb activin or ActRIIA-mediated effects on bone. In certain embodiments, the assay is carried out to screen and identify compounds that specifically inhibit or reduce binding of an ActRIIA polypeptide to activin. Alternatively, the assay can be used to identify compounds that enhance binding of an ActRIIA polypeptide to activin. In a further embodiment, the compounds can be identified by their ability to interact with an activin or ActRIIA polypeptide.

A variety of assay formats will suffice and, in light of the present disclosure, those not expressly described herein will nevertheless be comprehended by one of ordinary skill in the art. As described herein, the test compounds (agents) used herein may be created by any combinatorial chemical method. Alternatively, the subject compounds may be naturally occurring biomolecules synthesized in vivo or in vitro. Compounds (agents) to be tested for their ability to act as modulators of tissue growth can be produced, for example, by bacteria, yeast, plants or other organisms *(e.g.,* natural products), produced chemically (e.g., small molecules, including peptidomimetics), or produced recombinantly. Test compounds contemplated herein include non-peptidyl organic molecules, peptides, polypeptides, peptidomimetics, sugars, hormones, and nucleic acid molecules. In a specific embodiment, the test agent is a small organic molecule having a molecular weight of less than about 2,000 daltons.

The test compounds can be provided as single, discrete entities, or provided in libraries of greater complexity, such as made by combinatorial chemistry. These libraries can comprise, for example, alcohols, alkyl halides, amines, amides, esters, aldehydes, ethers and other classes of organic compounds. Presentation of test compounds to the test system can be in either an isolated form or as mixtures of compounds, especially in initial screening steps. Optionally, the compounds may be derivatized with other compounds and have derivatizing groups that facilitate isolation of the compounds. Non-limiting examples of derivatizing groups include biotin, fluorescein, digoxygenin, green fluorescent protein, isotopes, polyhistidine, magnetic beads, glutathione S transferase (GST), photoactivatible crosslinkers or any combinations thereof

In many drug screening programs which test libraries of compounds and natural extracts, high throughput assays are desirable in order to maximize the number of compounds surveyed in a given period of time. Assays which are performed in cell-free systems, such as may be derived with purified or semi-purified proteins, are often preferred as "primary" screens in that they can be generated to permit rapid development and relatively easy detection of an alteration in a molecular target which is mediated by a test compound. Moreover, the effects of cellular toxicity or bioavailability of the test compound can be generally ignored in the in vitro system, the assay instead being focused primarily on the effect of the drug on the molecular target as may be manifest in an alteration of binding affinity between an ActRIIA polypeptide and activin.

Merely to illustrate, in an exemplary screening assay, the compound of interest is contacted with an isolated and purified ActRIIA polypeptide which is ordinarily capable of binding to activin. To the mixture of the compound and ActRIIA polypeptide is then added a composition containing an ActRIIA ligand. Detection and quantification of ActRIIA/activin complexes provides a means for determining the compound's efficacy at inhibiting (or potentiating) complex formation between the ActRIIA polypeptide and activin. The efficacy of the compound can be assessed by generating dose response curves from data obtained using various concentrations of the test compound. Moreover, a control assay can also be performed to provide a baseline for comparison. For example, in a control assay, isolated and purified activin is added to a composition containing the ActRIIA polypeptide, and the formation of ActRIIA/activin complex is quantitated in the absence of the test compound. It will be understood that, in general, the order in which the reactants may be admixed can be varied, and can be admixed simultaneously. Moreover, in place of purified proteins, cellular extracts and lysates may be used to render a suitable cell-free assay system.

Complex formation between the ActRIIA polypeptide and activin may be detected by a variety of techniques. For instance, modulation of the formation of complexes can be quantitated using, for example, detectably labeled proteins such as radiolabeled (*e*.*g*., 32P, 35S, 14C or 3H), fluorescently labeled (*e.g.,* FITC), or enzymatically labeled ActRIIA polypeptide or activin, by immunoassay, or by chromatographic detection.

In certain cases, contemplated herein is the use of fluorescence polarization assays and fluorescence resonance energy transfer (FRET) assays in measuring, either directly or indirectly, the degree of interaction between an ActRIIA polypeptide and its binding protein. Further, other modes of detection, such as those based on optical waveguides (PCT Publication WO 96/26432 and U.S. Pat. No. 5,677,196), surface plasmon resonance (SPR), surface charge sensors, and surface force sensors, are compatible with many embodiments described herein.

Moreover, an interaction trap assay, also known as the "two hybrid assay," can be used for identifying agents that disrupt or potentiate interaction between an ActRIIA polypeptide and its binding protein. See for example, U.S. Pat. No. 5,283,317; Zervos et al. (1993) Cell 72:223-232; Madura et al. (1993) J Biol Chem 268:12046-12054; Bartel et al. (1993) Biotechniques 14:920-924; and Iwabuchi et al. (1993) Oncogene 8:1693-1696). In a specific embodiment, contemplated herein is the use of reverse two hybrid systems to identify compounds (*e*.*g*., small molecules or peptides) that dissociate interactions between an ActRIIA polypeptide and its binding protein. See for example, Vidal and Legrain, (1999) Nucleic Acids Res 27:919-29; Vidal and Legrain, (1999) Trends Biotechnol 17:374-81; and U.S. Pat. Nos. 5,525,490; 5,955,280; and 5,965,368.

In certain cases, the subject compounds are identified by their ability to interact with an ActRIIA or activin polypeptide. The interaction between the compound and the ActRIIA or activin polypeptide may be covalent or non-covalent. For example, such interaction can be identified at the protein level using in vitro biochemical methods, including photocrosslinking, radiolabeled ligand binding, and affinity chromatography (Jakoby W B et al., 1974, Methods in Enzymology 46: 1). In certain cases, the compounds may be screened in a mechanism based assay, such as an assay to detect compounds which bind to an activin or ActRIIA polypeptide. This may include a solid phase or fluid phase binding event. Alternatively, the gene encoding an activin or ActRIIA polypeptide can be transfected with a reporter system (*e*.*g*., β-galactosidase, luciferase, or green fluorescent protein) into a cell and screened against the library preferably by a high throughput screening or with individual members of the library. Other mechanism based binding assays may be used, for example, binding assays which detect changes in free energy. Binding assays can be performed with the target fixed to a well, bead or chip or captured by an immobilized antibody or resolved by capillary electrophoresis. The bound compounds may be detected usually using colorimetric or fluorescence or surface plasmon resonance.

### 6. EXAMPLES

### 6.1 EXAMPLE 1: A PHASE 2, MULTICENTER, OPEN-LABEL STUDY TO EVALUATE THE EFFICACY AND SAFETY OF LUSPATERCEPT IN SUBJECTS WITH MYELOPROLIFERATIVE NEOPLASM-ASSOCIATED MYELOFIBROSIS AND ANEMIA WITH AND WITHOUT RED BLOOD CELL-TRANSFUSION DEPENDENCE

This example relates to a Phase 2, multicenter, open-label study to evaluate luspatercept, an ActRIIB signaling inhibitor, in subjects with myeloproliferative neoplasm-associated myelofibrosis who have anemia with and without red blood cell-transfusion dependence. The study is divided into a Screening Period, a Treatment Period (consisting of a Primary Phase, a Day 169 Disease Response Assessment, and an Extension Phase), followed by a Posttreatment Follow-up Period. The purpose of this phase 2 study is to evaluate if there is an efficacy signal with luspatercept in patients with MPN-associated MF who are anemic and may or may not be RBC transfusion dependent by correcting ineffective erythropoiesis and thereby lessening anemia, a condition that develops in nearly all MPN-associated MF patients (of which fatigue is a symptom).

Subjects satisfying the eligibility criteria are assigned to 1 of the following cohorts (which are enrolling in parallel) based on their eligibility: (1) Cohort 1: contains up to 20 subjects with anemia only that are not currently receiving RBC transfusions (these subjects are referred to as "anemia only" throughout this example, defined as having received 0 RBC units/84 days immediately up to the Cycle 1, Day 1 (C1D1) date); (II) Cohort 2: contains up to 20 subjects that are RBC-transfusion dependent (these subjects are referred to as "RBC-transfusion dependent" throughout the example, defined as having received an average RBC transfusion frequency of 2 to 4 RBC units/28 days over at least the 84 days immediately up to the C1D1 date); (III) Cohort 3A: contains ≥ 10 subjects (Cohort 3 contains 30 subjects in total) who meet the eligibility criteria for Cohort 1 (anemia only) while being on a stable dose of ruxolitinib for at least 112 days immediately prior to the enrollment date; and (IV) Cohort 3B: contains ≥ 10 subjects (Cohort 3 contains 30 subjects in total) who meet the eligibility criteria for Cohort 2 (RBC-transfusion dependent) while being on a stable dose of ruxolitinib for at least 112 days immediately prior to the enrollment date. Overall, the study will enroll approximately 70 subjects worldwide.

### 6.1.1 Primary Outcome Measures

The following are primary outcome measures for the Phase 2 trial: (i) anemia response as it relates to hemoglobin (Hgb) increase (proportion of cohorts 1 and 3A subjects achieving ≥ 1.5 g/dL hemoglobin increase from baseline over any consecutive 84-day period without an RBC transfusion; time frame of measurements is approximately up to day 168); (ii) anemia response as it relates to increase red blood cell (RBC)-transfusion independence (proportion of cohorts 2 and 3B subjects who become RBC-transfusion free over any consecutive 84-day period; time frame of measurements is approximately up to day 168); (iii) time to anemia response (time from first luspatercept dose to first onset of anemia response in each of the cohorts; time frame of measurements is approximately up to 2 years). (iv) duration of anemia response (the maximum duration of anemia response in each of the cohorts; time frame of measurements is approximately up to 2 years); (v) frequency of RBC transfusions (the mean number of RBC units transfused per subject per four week; time frame of measurements is approximately up to 2 years); (vi) frequency of RBC transfusion dependence (Proportion of RBC transfusion dependent subjects who reduce their transfusion burden by ≥ 50% from baseline over any consecutive 84-day period; time frame of measurements is approximately up to 2 years); (vii) proportion of subjects who achieve ≥ 50% reduction in fatigue symptom, a common symptom among patients with myeloproliferative neoplasm-associated myelofibrosis who have anemia, as recorded and assessed via the modified Myeloproliferative Neoplasm Symptom Assessment Form (MPN-SAF), *(see* Emanual et al., "Myeloproliferative Neoplasm (MPN) Symptom Assessment Form Total Symptom Score: Prospective International Assessment of an Abbreviated Symptom Burden Scoring System Among Patients With MPNs," J. Clin. Oncol. 30(33):4098-4103 (2012); time frame of measurements is approximately up to 2 years); (viii) the proportion of subjects who achieve ≥ 50% reduction in total symptom score (TSS). Myeloproliferative neoplasm (MPN)-associated myelofibrosis-related symptoms (fatigue, night sweats, itchiness, abdominal discomfort, pain under the ribs on the left side, early satiety, and bone pain) as recorded and assessed via the modified Myeloproliferative Neoplasm Symptom Assessment Form (MPN-SAF), (*see* Emanual et al., "Myeloproliferative Neoplasm (MPN) Symptom Assessment Form Total Symptom Score: Prospective International Assessment of an Abbreviated Symptom Burden Scoring System Among Patients With MPNs," J. Clin. Oncol. 30(33):4098-4103 (2012); (ix) Health-related quality of life (HRQoL): measurement of mean changes in HRQoL questionnaire domain scores over the study are compared to baseline scores (time frame of measurements is approximately up to 2 years); (x) EQ-5D-5L questionnaires (EuroQol, Rotterdam, The Netherlands): measure mean changes in EQ-5D-5L questionnaire domain scores over the study are compared to baseline scores (time frame of measurements is approximately up to 2 years); (xi) adverse events (AEs) (including the type, frequency, and severity, are evaluated; time frame of measurements is approximately up to 2 years ); (xii) antidrug antibodies (ADA) (frequency of antidrug antibodies and effects on efficacy, safety, or pharmacokinetics; time frame of measurements is approximately up to 2 years); (xiii) pharmacokinetic (area under the curve (AUC) and Cmax; time frame of measurements is approximately up to 2 years); and (xiv) Functional Assessment of Cancer Therapy - Anemia (FACT-An): measurement of mean changes in FACT-An questionnaire domain scores over the study are compared to baseline scores (time frame of measurements is approximately up to 2 years).

### 6.1.2 Inclusion Criteria

Subjects must satisfy the criteria in this section to be enrolled in the study (with the enrollment date defined as the date in which the subject is assigned a cohort in Integrated Response Technology [IRT]) and receive their first dose of luspatercept.

The subject must be ≥18 years of age at the time of signing the informed consent form (ICF).

The subject has MPN-associated myelofibrosis (PMF, post-PV MF, and/or post-ET MF) as confirmed from the local pathology report.

The subject has anemia defined as: (I) Cohorts 1 and 3A: (a) subject has ≥ 3 Hgb levels of ≤ 9.5 g/dL recorded on ≥ 3 different days, including the day of dosing, in the 84-day period immediately up to the C1D1 date; no subjects with an interval ≥ 42 days between hemoglobin measurements are enrolled; and (b) there must not be any RBC transfusions within the 84-day period immediately up to the C1D1 date; and (11) Cohorts 2 and 3B: (a) subject has an average RBC-transfusion frequency of 2 to 4 RBC units/28 days over at least the 84 days immediately up to the C1D1 date; there must be no interval > 42 days without ≥ 1 RBC-transfusion; (b) subjects must have a Hgb value of ≤ 13 g/dL on C1D1 prior to luspatercept administration; (c) only RBC transfusions given when the Hgb ≤ 9.5 g/dL are scored in determining eligibility; and (d) RBC transfusions given because of bleeding, infection, or chemotherapyinduced anemia are not scored in determining eligibility.

The subject has an Eastern Cooperative Oncology Group (ECOG) performance score of ≤ 2. *See,* e.g., Oken et al. (1982). "Toxicity and response criteria of the Eastern Cooperative Oncology Group". Am. J. Clin. Oncol. 5 (6): 649-55, regarding the ECOG performance score. A subject having an ECOG performance score of 0 is asymptomatic (i.e., fully active, able to carry on all pre-disease activities without restriction). A subject having an ECOG performance score of 1 is symptomatic but completely ambulatory (i.e., restricted in physically strenuous activity but ambulatory and able to carry out work of a light or sedentary nature, for example, light housework, office work). A subject having an ECOG performance score of 2 is symptomatic, <50% in bed during the day (i.e., ambulatory and capable of all self care but unable to carry out any work activities; up and about more than 50% of waking hours). A subject having an ECOG performance score of 3 is symptomatic, >50% in bed, but not bedbound (i.e., capable of only limited self-care, confined to bed or chair 50% or more of waking hours). A subject having an ECOG performance score of 4 is bedbound (i.e., completely disabled; cannot carry on any self-care; totally confined to bed or chair). A subject having an ECOG performance score of 5 is dead.

The subject is not anticipated during the 6 months from the C1D1 date to receive a hematopoietic cell transplant.

A female of childbearing potential (FCBP) for this study is defined as a female who: (1) has achieved menarche at some point; and (2) has not undergone a hysterectomy or bilateral therapy (does not rule out childbearing potential) for at least 24 consecutive months (i.e., has had menses at any time in the preceding 24 consecutive months). FCBP participating in the study must: (a) have 2 negative pregnancy tests as verified by the Investigator prior to starting study therapy; she must agree to ongoing pregnancy testing during the course of the study, and after end of study treatment, this applies even if the subject practices true abstinence from heterosexual contact; and (b) either commit to true abstinence from heterosexual contact (which must be reviewed on a monthly basis and source documented) or agree to use, and be able to comply with, effective contraception without interruption, 28 days prior to starting investigational product, during the study therapy (including dose interruptions), and for 12 weeks (approximately 5 times the mean terminal halflife of luspatercept based on multiple-dose pharmacokinetics [PK] data) after discontinuation of study therapy.

Male subjects must: (a) practice true abstinence (which must be reviewed on a monthly basis) or agree to use a condom during sexual contact with a pregnant female or a female of childbearing potential while participating in the study, during dose interruptions and for at least 12 weeks (approximately 5 times the mean terminal half-life of luspatercept based on multiple-dose PK data) following investigational product discontinuation, even if he has undergone a successful vasectomy.

The subject must understand and voluntarily sign an informed consent form prior to any study-related assessments/procedures being conducted.

The subject is willing and able to adhere to the study visit schedule and other protocol requirements.

### 6.1.3 Method of Treatment

The dose of luspatercept is 1.75 mg/kg.

Luspatercept is administered to the subject subcutaneously once every 21 days for up to 2 years.

### 6.2 EXAMPLE 2

### 6.2.1 Background

Myeloproliferative neoplasm (MPN)-associated myelofibrosis (MF) is a clonal myeloid neoplasm characterized by bone marrow fibrosis, defective bone marrow function, extramedullary hematopoiesis, a propensity for transformation to blast phase, and inflammation (Mesa RA, et al. Leuk Res. 2011;35(1):12-13). Anemia and red blood cell (RBC) transfusion dependence (TD) are independent adverse prognostic and predictive variables for survival among these patients (Passamonti F, et al. Blood. 2010;115(9): 1703-1708; Elena C, et al. Haematologica. 2011;96(1): 167-170).

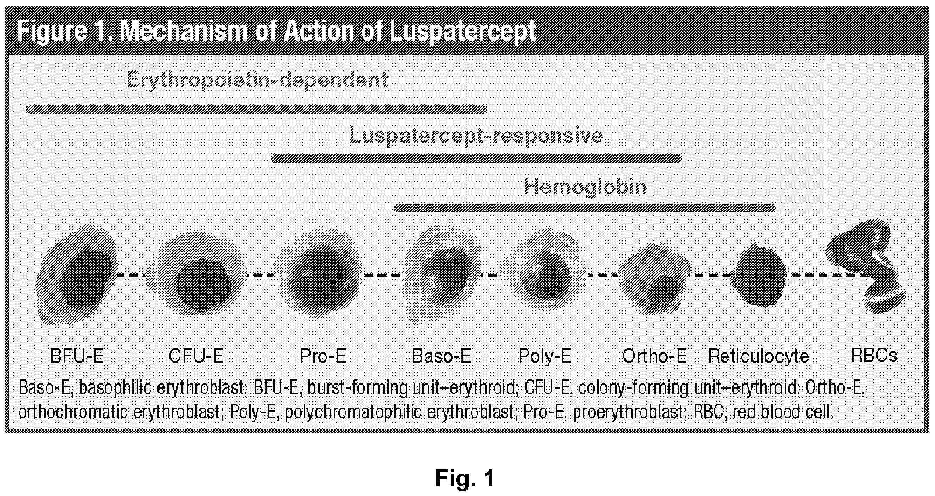

Luspatercept is a recombinant fusion protein consisting of a modified activin receptor type IIB linked to the Fc domain of human immunoglobulin G1 (IgG1) (Figure 1) (Attie KM, et al. Am J Hematol. 2014;89(7):766-770; Suragani RN, et al. Nat Med. 2014;20(4):408-414). Luspatercept acts as an erythroid maturation agent by binding specific transforming growth factor-β (TGF-β) superfamily ligands such as growth differentiation factor-11 (GDF11), blocking their inhibitory effect, and leading to increased RBC production (Suragani RN, et al. Nat Med. 2014;20(4):408-414). In a recent phase 2 study, luspatercept was shown to be effective and well tolerated for the treatment of anemia in patients with lower-risk myelodysplastic syndromes (Platzbecker U, et al. Lancet Oncol. 2017;18(10):1338-1347).

The objective of this clinical trial is to evaluate the efficacy and safety of luspatercept for the treatment of anemia in patients with MPN-associated MF with or without RBC-TD.

### 6.2.2 Study Details

This example relates to an ongoing, multicenter, open-label phase 2 study.

### 6.2.3 Study Population

The inclusion criteria for this study is: (i) age ≥ 18 years; (ii) MPN-associated MF (primary MF, post-polycythemia vera MF, or post-essential thrombocythemia MF); (iii) Anemia, defined as: (a) Cohorts 1 and 3a: ≥ 3 hemoglobin (Hb) levels ≤ 9.5 g/dL on ≥ 3 days (including day of dosing), with no RBC transfusions in the 84 days prior to cycle 1 day 1 (C1D1); ≥ 42 days between measurements will be excluded; (b) Cohorts 2 and 3b: average RBC transfusion frequency of 2-4 RBC units/28 days over ≥ 84 days prior to C1D1, with no interval > 42 days without ≥ 1 RBC transfusion; Hb < 13 g/dL on C1D1 prior to luspatercept administration; and (iv) Eastern Cooperative Oncology Group performance status ≤ 2.

### 6.2.4 Study Design and Treatment:

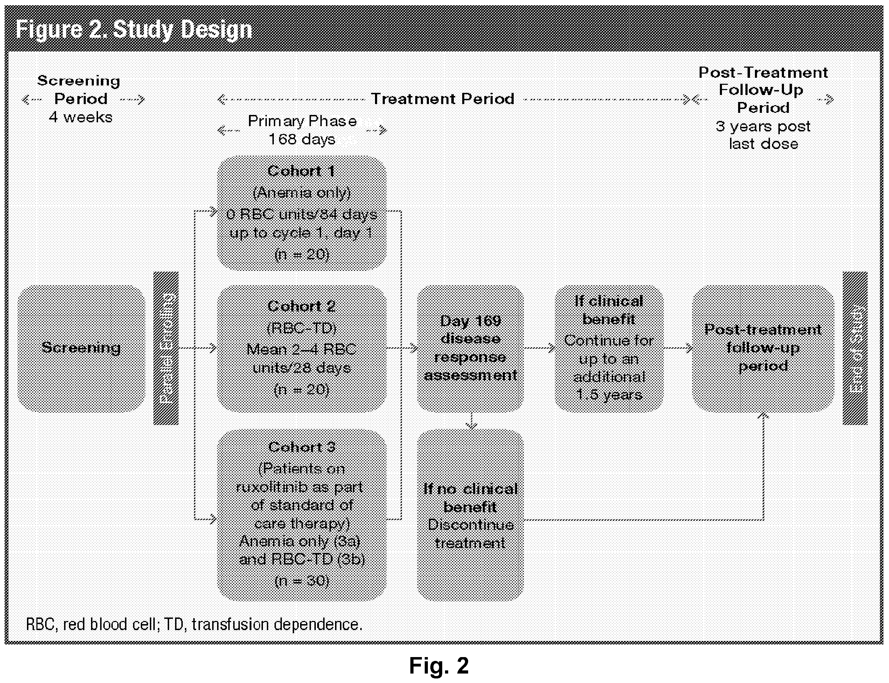

The study comprises 3 periods (Figure 2): (i) Screening period; (ii) Treatment period (primary phase, disease response assessment at day 169, and extension phase); and (iii) Post-treatment follow-up period.

### (a) Screening Period

All screening procedures are conducted ≤ 28 days prior to enrollment.

### (b) Treatment Period

All patients will receive luspatercept 1 mg/kg subcutaneously on day 1 of each 21-day cycle. Patients will be enrolled in cohorts according to RBC transfusion requirement: (i) Cohorts 1 and 3a (anemia only); and (ii) Cohorts 2 and 3b (RBC-TD). Best supportive care may be used in combination with study treatment. Disease response assessment should be completed at day 169 following the first dose of study treatment. Responders may continue treatment for up to an additional 1.5 years. Non-responders will discontinue treatment.

### (c) Post-Treatment Follow-Up Period

After treatment discontinuation, follow-up safety data will be collected up to 42 days after the last dose of study treatment. Safety data will then be collected every 3 months for up to 3 years after the last dose of study treatment or until death, consent withdrawal, or loss to follow-up.

### 6.2.5 Endpoints

Primary and secondary endpoints are listed in the Table 1 (below). Exploratory endpoints include treatment exposure-response, biomarkers, and mutational analyses. All efficacy analyses will be performed primarily on the intent-to-treat population, defined as all patients enrolled. Confirmatory efficacy analyses will be performed on the efficacy-evaluable population, defined as all patients who: (i) received ≥ 3 cycles of study treatment and remain on study for ≥ 21 days after the third study dose; or (ii) achieved Hb > 13 g/dL in < 3 cycles. Safety analyses will be performed on all patients receiving ≥ 1 study treatment dose. Adverse events and laboratory abnormalities are classified according to the NCI-CTCAE version 4.03. Pharmacokinetic analyses will be based on all patients who have evaluable concentration data to determine the pharmacokinetic parameters.

### 6.2.6 Statistical Analyses

Descriptive statistical analyses will primarily be used. The Kaplan-Meier method may be used to estimate duration of anemia response. No inferential comparisons between the cohorts will be performed.

### 6.2.7 Study Status

Enrollment began in November 2017. Target enrollment is 70 patients with MPN-associated MF and anemia, with or without RBC-TD. As of April 9, 2018 across 24 clinical sites, 12 patients have been enrolled into the study.

**Table 1. Study Endpoints**

| **Endpoints** | **Cohorts 1 and 3a (Anemia Only)** | **Cohorts 2 and 3b (RBC-TD)** |
|---|---|---|
| Primary | ≥ 1.5 g/dL Hb increase from baseline over any consecutive 84-day period without an RBC transfusion | RBC-TI over any consecutive 84-day period |
| Secondary | • Time to anemia response | • Time to anemia response |
| | • Duration of anemia response | • Duration of anemia response |
| | • Symptom response improvement (defined as ≥ 50% reduction in fatigue symptoms or ≥ 50% reduction in total symptom score by MF-SAF or MPN-SAF) | • Frequency of RBC transfusions (mean RBC units/4 weeks) |
| | | • Frequency of RBC-TD (defined as reduction in transfusion burden by ≥ 50% from baseline over any consecutive 84-day period) |
| | • HRQoL improvement | |
| | • Safety | |
| | • Pharmacokinetics | • Symptom response improvement (defined as ≥ 50% reduction in fatigue symptoms or ≥ 50% reduction in total symptom score by MF-SAF or MPN-SAF) |
| | • Antidrug antibodies | |
| | | • HRQoL improvement |
| | | • Safety |
| | | • Pharmacokinetics |
| | | • Antidrug antibodies |
| Hb, hemoglobin; HRQoL, health-related quality of life; MF-SAF, Myelofibrosis Symptom Assessment Form; MPN-SAF, Myeloproliferative Neoplasm Symptom Assessment Form; RBC, red blood cell; TD, transfusion dependence; TI, transfusion independence. | | |

### 7. SEQUENCE INFORMATION

**Table 2.**

| SEQ ID NO: | Description | Sequence |
|---|---|---|
| 1 | Extracellular domain of human ActRIIA fused to a human Fc domain | ILGRSETQECLFFNANWEKDRTNQTGVEPCYGDK DKRRHCFATWKNISGSIEIVKQGCWLDDINCYDR TDCVEKKDSPEVYFCCCEGNMCNEKFSYFPEMEV TQPTSNPVTPKPPTGGGTHTCPPCPAPELLGGPSV FLFPPKPKDTLMISRTPEVTCVVVDVSHEDPEVKF NWYVDGVEVHNAKTKPREEQYNSTYRVVSVLTV LHQDWLNGKEYKCKVSNKALPVPIEKTISKAKGQ PREPQVYTLPPSREEMTKNQVSLTCLVKGFYPSDI AVEWESNGQPENNYKTTPPVLDSDGSFFLYSKLT VDKSRWQQGNVFSCSVMHEALHNHYTQKSLSLS PGK |
| 2 | Leader sequence of Honey bee mellitin (HBML) | MKFLVNVALVFMVVYISYIYA |
| 3 | human ActRIIB precursor protein sequence (A64) | MTAPWVALALLWGSLWPGSGRGEAETRECIYYN ANWELERTNQSGLERCEGEQDKRLHCYASWANS SGTIELVKKGCWLDDFNCYDRQECVATEENPQV YFCCCEGNFCNERFTHLPEAGGPEVTYEPPPTAPT LLTVLAYSLLPIGGLSLIVLLAFWMYRHRKPPYGH VDIHEDPGPPPPSPLVGLKPLQLLEIKARGRFGCV WKAQLMNDFVAVKIFPLQDKQSWQSEREIFSTPG MKHENLLQFIAAEKRGSNLEVELWLITAFHDKGS LTDYLKGNIITWNELCHVAETMSRGLSYLHEDVP WCRGEGHKPSIAHRDFKSKNVLLKSDLTAVLADF GLAVRFEPGKPPGDTHGQVGTRRYMAPEVLEGAI NFQRDAFLRIDMYAMGLVLWELVSRCKAADGPV DEYMLPFEEEIGQHPSLEELQEVVVHKKMRPTIKD HWLKHPGLAQLCVTIEECWDHDAEARLSAGCVE ERVSLIRRSVNGTTSDCLVSLVTSVTNVDLPPKES SI |
| 4 | human ActRIIB soluble (extracellular), processed polypeptide sequence (amino acids 19-134 of SEQ ID NO:3) | SGRGEAETRECIYYNANWELERTNQSGLERCEGE QDKRLHCYASWANSSGTIELVKKGCWLDDFNCY DRQECVATEENPQVYFCCCEGNFCNERFTHLPEA GGPEVTYEPPPTAPT |
| 5 | human ActRIIB soluble (extracellular), processed polypeptide sequence with | SGRGEAETRECIYYNANWELERTNQSGLERCEGE QDKRLHCYASWANSSGTIELVKKGCWLDDFNCY |
| | the C-terminal 15 amino acids deleted (amino acids 19-119 of SEQ ID NO:3) | DRQECVATEENPQVYFCCCEGNFCNERFTHLPEA |
| 6 | nucleic acid sequence encoding a human ActRIIB (A64) precursor protein | ATGACGGCGCCCTGGGTGGCCCTCGCCCTCCTC TGGGGATCGCTGTGGCCCGGCTCTGGGCGTGGG GAGGCTGAGACACGGGAGTGCATCTACTACAA CGCCAACTGGGAGCTGGAGCGCACCAACCAGA GCGGCCTGGAGCGCTGCGAAGGCGAGCAGGAC AAGCGGCTGCACTGCTACGCCTCCTGGGCCAAC AGCTCTGGCACCATCGAGCTCGTGAAGAAGGG CTGCTGGCTAGATGACTTCAACTGCTACGATAG GCAGGAGTGTGTGGCCACTGAGGAGAACCCCC AGGTGTACTTCTGCTGCTGTGAAGGCAACTTCT GCAACGAGCGCTTCACTCATTTGCCAGAGGCTG GGGGCCCGGAAGTCACGTACGAGCCACCCCCG ACAGCCCCCACCCTGCTCACGGTGCTGGCCTAC TCACTGCTGCCCATCGGGGGCCTTTCCCTCATC GTCCTGCTGGCCTTTTGGATGTACCGGCATCGC AAGCCCCCCTACGGTCATGTGGACATCCATGAG GACCCTGGGCCTCCACCACCATCCCCTCTGGTG GGCCTGAAGCCACTGCAGCTGCTGGAGATCAA GGCTCGGGGGCGCTTTGGCTGTGTCTGGAAGGC CCAGCTCATGAATGACTTTGTAGCTGTCAAGAT CTTCCCACTCCAGGACAAGCAGTCGTGGCAGAG TGAACGGGAGATCTTCAGCACACCTGGCATGAA GCACGAGAACCTGCTACAGTTCATTGCTGCCGA GAAGCGAGGCTCCAACCTCGAAGTAGAGCTGT GGCTCATCACGGCCTTCCATGACAAGGGCTCCC TCACGGATTACCTCAAGGGGAACATCATCACAT GGAACGAACTGTGTCATGTAGCAGAGACGATG TCACGAGGCCTCTCATACCTGCATGAGGATGTG CCCTGGTGCCGTGGCGAGGGCCACAAGCCGTCT ATTGCCCACAGGGACTTTAAAAGTAAGAATGTA TTGCTGAAGAGCGACCTCACAGCCGTGCTGGCT GACTTTGGCTTGGCTGTTCGATTTGAGCCAGGG AAACCTCCAGGGGACACCCACGGACAGGTAGG CACGAGACGGTACATGGCTCCTGAGGTGCTCGA GGGAGCCATCAACTTCCAGAGAGATGCCTTCCT GCGCATTGACATGTATGCCATGGGGTTGGTGCT GTGGGAGCTTGTGTCTCGCTGCAAGGCTGCAGA CGGACCCGTGGATGAGTACATGCTGCCCTTTGA GGAAGAGATTGGCCAGCACCCTTCGTTGGAGG |
| | | AGCTGCAGGAGGTGGTGCACAAGAAGATG AGGCCCACCATTAAAGATCACTGGTTGAAACAC CCGGGCCTGGCCCAGCTTTGTGTGACCATCGAG GAGTGCTGGGACCATGATGCAGAGGCTCGCTTG TCCGCGGGCTGTGTGGAGGAGCGGGTGTCCCTG ATTCGGAGGTCGGTCAACGGCACTACCTCGGAC TGTCTCGTTTCCCTGGTGACCTCTGTCACCAATG TGGACCTGCCCCCTAAAGAGTCAAGCATCTAA |
| 7 | fusion protein comprising a soluble extracellular domain of ActRIIB (A64; SEQ ID NO: 17) fused to an Fc domain | SGRGEAETRECIYYNANWELERTNQSGLERCEGE QDKRLHCYASWANSSGTIELVKKGCWLDDFNCY DRQECVATEENPQVYFCCCEGNFCNERFTHLPEA GGPEVTYEPPPTAPTGGGTHTCPPCPAPELLGGPS VFLFPPKPKDTLMISRTPEVTCVVVDVSHEDPEVK FNWYVDGVEVHNAKTKPREEQYNSTYRVVSVLT VLHQDWLNGKEYKCKVSNKALPVPIEKTISKAKG QPREPQVYTLPPSREEMTKNQVSLTCLVKGFYPS DIAVEWESNGQPENNYKTTPPVLDSDGSFFLYSKL TVDKSRWQQGNVFSCSVMHEALHNHYTQKSLSL SPGK |
| 8 | fusion protein comprising a soluble extracellular domain of ActRIIB (A64) with the C-terminal 15 amino acids deleted (SEQ ID NO: 5) fused to an Fc domain | SGRGEAETRECIYYNANWELERTNQSGLERCEGE QDKRLHCYASWANSSGTIELVKKGCWLDDFNCY DRQECVATEENPQVYFCCCEGNFCNERFTHLPEA GGGTHTCPPCPAPELLGGPSVFLFPPKPKDTLMIS RTPEVTCVVVDVSHEDPEVKFNWYVDGVEVHNA KTKPREEQYNSTYRVVSVLTVLHQDWLNGKEYK CKVSNKALPVPIEKTISKAKGQPREPQVYTLPPSR EEMTKNQVSLTCLVKGFYPSDIAVEWESNGQPEN NYKTTPPVLDSDGSFFLYSKLTVDKSRWQQGNVF SCSVMHEALHNHYTQKSLSLSPGK |
| 9 | human ActRIIB soluble (extracellular), processed polypeptide sequence with the N-terminal 6 amino acids of the EC domain deleted and the C-terminal 3 amino acids of the EC domain deleted (amino acids 25-131 of SEQ ID NO:14) and with an L79D mutation | ETRECIYYNANWELERTNQSGLERCEGEQDKRLH CYASWRNSSGTIELVKKGCWDDDFNCYDRQECV ATEENPQVYFCCCEGNFCNERFTHLPEAGGPEVT YEPPPT |
| 10 | Unprocessed ActRIIB-Fc | MDAMKRGLCCVLLLCGAVFVSPGAAETRECIYY |
| | fusion protein with the N-terminal 6 amino acids of the EC domain deleted and the C-terminal 3 amino acids of the EC domain deleted (amino acids 25-131 of SEQ ID NO:14) and with an L79D mutation and with TPA leader sequence | NANWELERTNQSGLERCEGEQDKRLHCYASWRN SSGTIELVKKGCWDDDFNCYDRQECVATEENPQV YFCCCEGNFCNERFTHLPEAGGPEVTYEPPPTGGG THTCPPCPAPELLGGPSVFLFPPKPKDTLMISRTPE VTCVVVDVSHEDPEVKFNWYVDGVEVHNAKTK PREEQYNSTYRVVSVLTVLHQDWLNGKEYKCKV SNKALPAPIEKTISKAKGQPREPQVYTLPPSREEM TKNQVSLTCLVKGFYPSDIAVEWESNGQPENNYK TTPPVLDSDGSFFLYSKLTVDKSRWQQGNVFSCS VMHEALHNHYTQKSLSLSPGK* |
| 11 | Processed ActRIIB-Fc fusion protein with the N-terminal 6 amino acids of the EC domain deleted and the C-terminal 3 amino acids of the EC domain deleted (amino acids 25-131 of SEQ ID NO:14) and with an L79D mutation (*i.e.,* luspatercept) | ETRECIYYNANWELERTNQSGLERCEGEQDKRLH CYASWRNSSGTIELVKKGCWDDDFNCYDRQECV ATEENPQVYFCCCEGNFCNERFTHLPEAGGPEVT YEPPPTGGGTHTCPPCPAPELLGGPSVFLFPPKPKD TLMISRTPEVTCVVVDVSHEDPEVKFNWYVDGVE VHNAKTKPREEQYNSTYRVVSVLTVLHQDWLNG KEYKCKVSNKALPAPIEKTISKAKGQPREPQVYTL PPSREEMTKNQVSLTCLVKGFYPSDIAVEWESNG QPENNYKTTPPVLDSDGSFFLYSKLTVDKSRWQQ GNVFSCSVMHEALHNHYTQKSLSLSPGK* |
| 12 | human ActRIIB soluble (extracellular), processed polypeptide sequence (amino acids 20-134 of SEQ ID NO:3) | GRGEAETRECIYYNANWELERTNQSGLERCEGEQ DKRLHCYASWANSSGTIELVKKGCWLDDFNCYD RQECVATEENPQVYFCCCEGNFCNERFTHLPEAG GPEVTYEPPPTAPT |
| 13 | human ActRIIB soluble (extracellular), processed polypeptide sequence with the C-terminal 15 amino acids deleted (amino acids 20-119 of SEQ ID NO:3) | GRGEAETRECIYYNANWELERTNQSGLERCEGEQ DKRLHCYASWANSSGTIELVKKGCWLDDFNCYD RQECVATEENPQVYFCCCEGNFCNERFTHLPE A |
| 14 | human ActRIIB precursor protein sequence (R64) | MTAPWVALALLWGSLWPGSGRGEAETRECIYYN ANWELERTNQSGLERCEGEQDKRLHCYASWRNS SGTIELVKKGCWLDDFNCYDRQECVATEENPQV YFCCCEGNFCNERFTHLPEAGGPEVTYEPPPTAPT LLTVLAYSLLPIGGLSLIVLLAFWMYRHRKPPYGH VDIHEDPGPPPPSPLVGLKPLQLLEIKARGRFGCV WKAQLMNDFVAVKIFPLQDKQSWQSEREIFSTPG MKHENLLQFIAAEKRGSNLEVELWLITAFHDKGS LTDYLKGNIITWNELCHVAETMSRGLSYLHEDVP WCRGEGHKPSIAHRDFKSKNVLLKSDLTAVLADF |
| | | GLAVRFEPGKPPGDTHGQVGTRRYMAPEVLEGAI NFQRDAFLRIDMYAMGLVLWELVSRCKAADGPV DEYMLPFEEEIGQHPSLEELQEVVVHKKMRPTIKD HWLKHPGLAQLCVTIEECWDHDAEARLSAGCVE ERVSLIRRSVNGTTSDCLVSLVTSVTNVDLPPKES SI |
| 15 | human ActRIIB soluble (extracellular), processed polypeptide sequence (amino acids 19-134 of SEQ ID NO: 14) | SGRGEAETRECIYYNANWELERTNQSGLERCEGE QDKRLHCYASWRNSSGTIELVKKGCWLDDFNCY DRQECVATEENPQVYFCCCEGNFCNERFTHLPEA GGPEVTYEPPPTAPT |
| 16 | human ActRIIB soluble (extracellular), processed polypeptide sequence with the C-terminal 15 amino acids deleted (amino acids 19-119 of SEQ ID NO:14) | SGRGEAETRECIYYNANWELERTNQSGLERCEGE QDKRLHCYASWRNSSGTIELVKKGCWLDDFNCY DRQECVATEENPQVYFCCCEGNFCNERFTHLPEA |
| 17 | human ActRIIB soluble (extracellular), processed polypeptide sequence (amino acids 20-134 of SEQ ID NO:14) | GRGEAETRECIYYNANWELERTNQSGLERCEGEQ DKRLHCYASWRNSSGTIELVKKGCWLDDFNCYD RQECVATEENPQVYFCCCEGNFCNERFTHLPEAG GPEVTYEPPPTAPT |
| 18 | human ActRIIB soluble (extracellular), processed polypeptide sequence with the C-terminal 15 amino acids deleted (amino acids 20-119 of SEQ ID NO:14) | GRGEAETRECIYYNANWELERTNQSGLERCEGEQ DKRLHCYASWRNSSGTIELVKKGCWLDDFNCYD RQECVATEENPQVYFCCCEGNFCNERFTHLPE A |
| 19 | human ActRIIB soluble (extracellular), processed polypeptide sequence with the N-terminal 6 amino acids of the EC domain deleted and the C-terminal 3 amino acids of the EC domain deleted (amino acids 25-131 of SEQ ID NO:3) and with an L79D mutation | ETRECIYYNANWELERTNQSGLERCEGEQDKRLH CYASWANSSGTIELVKKGCWDDDFNCYDRQECV ATEENPQVYFCCCEGNFCNERFTHLPEAGGPEVT YEPPPT |
| 20 | Unprocessed ActRIIB-Fc fusion protein with the N-terminal 6 amino acids of the EC domain deleted and the C-terminal 3 amino acids of the EC domain deleted (amino acids 25-131 of SEQ ID NO:3) and with an L79D mutation and with TPA leader sequence | MDAMKRGLCCVLLLCGAVFVSPGAAETRECIYY NANWELERTNQSGLERCEGEQDKRLHCYASWAN SSGTIELVKKGCWDDDFNCYDRQECVATEENPQV YFCCCEGNFCNERFTHLPEAGGPEVTYEPPPTGGG THTCPPCPAPELLGGPSVFLFPPKPKDTLMISRTPE VTCVVVDVSHEDPEVKFNWYVDGVEVHNAKTK PREEQYNSTYRVVSVLTVLHQDWLNGKEYKCKV SNKALPAPIEKTISKAKGQPREPQVYTLPPSREEM TKNQVSLTCLVKGFYPSDIAVEWESNGQPENNYK TTPPVLDSDGSFFLYSKLTVDKSRWQQGNVFSCS VMHEALHNHYTQKSLSLSPGK* |
| 21 | Processed ActRIIB-Fc fusion protein with the N-terminal 6 amino acids of the EC domain deleted and the C-terminal 3 amino acids of the EC domain deleted (amino acids 25-131 of SEQ ID NO:3) and with an L79D mutation | ETRECIYYNANWELERTNQSGLERCEGEQDKRLH CYASWANSSGTIELVKKGCWDDDFNCYDRQECV ATEENPQVYFCCCEGNFCNERFTHLPEAGGPEVT YEPPPTGGGTHTCPPCPAPELLGGPSVFLFPPKPKD TLMISRTPEVTCVVVDVSHEDPEVKFNWYVDGVE VHNAKTKPREEQYNSTYRVVSVLTVLHQDWLNG KEYKCKVSNKALPAPIEKTISKAKGQPREPQVYTL PPSREEMTKNQVSLTCLVKGFYPSDIAVEWESNG QPENNYKTTPPVLDSDGSFFLYSKLTVDKSRWQQ GNVFSCSVMHEALHNHYTQKSLSLSPGK* |
| 22 | human ActRIIB soluble (extracellular), processed polypeptide sequence (amino acids 20-134 of SEQ ID NO: 14) with L79D mutation | GRGEAETRECIYYNANWELERTNQSGLERCEGEQ DKRLHCYASWRNSSGTIELVKKGCWDDDFNCYD RQECVATEENPQVYFCCCEGNFCNERFTHLPEAG GPEVTYEPPPTAPT |
| 23 | human ActRIIB soluble (extracellular), processed polypeptide sequence (amino acids 20-134 of SEQ ID NO:3) with L79D mutation | GRGEAETRECIYYNANWELERTNQSGLERCEGEQ DKRLHCYASWANSSGTIELVKKGCWDDDFNCYD RQECVATEENPQVYFCCCEGNFCNERFTHLPEAG GPEVTYEPPPTAPT |
| 24 | human ActRIIB soluble (extracellular), processed polypeptide sequence (amino acids 20-134 of SEQ ID NO: 14) with L79D mutation fused to an Fc domain with a GGG linker | GRGEAETRECIYYNANWELERTNQSGLERCEGEQ DKRLHCYASWRNSSGTIELVKKGCWDDDFNCYD RQECVATEENPQVYFCCCEGNFCNERFTHLPEAG GPEVTYEPPPTAPTGGGTHTCPPCPAPELLGGPSV FLFPPKPKDTLMISRTPEVTCVVVDVSHEDPEVKF NWYVDGVEVHNAKTKPREEQYNSTYRVVSVLTV LHQDWLNGKEYKCKVSNKALPAPIEKTISKAKGQ PREPQVYTLPPSREEMTKNQVSLTCLVKGFYPSDI AVEWESNGQPENNYKTTPPVLDSDGSFFLYSKLT |
| | | VDKSRWQQGNVFSCSVMHEALHNHYTQKSLSLS PGK* |
| 25 | human ActRIIB soluble (extracellular), processed polypeptide sequence (amino acids 20-134 of SEQ ID NO:3) with L79D mutation fused to an Fc domain | GRGEAETRECIYYNANWELERTNQSGLERCEGEQ DKRLHCYASWANSSGTIELVKKGCWDDDFNCYD RQECVATEENPQVYFCCCEGNFCNERFTHLPEAG GPEVTYEPPPTAPTGGGTHTCPPCPAPELLGGPSV FLFPPKPKDTLMISRTPEVTCVVVDVSHEDPEVKF NWYVDGVEVHNAKTKPREEQYNSTYRVVSVLTV LHQDWLNGKEYKCKVSNKALPAPIEKTISKAKGQ PREPQVYTLPPSREEMTKNQVSLTCLVKGFYPSDI AVEWESNGQPENNYKTTPPVLDSDGSFFLYSKLT VDKSRWQQGNVFSCSVMHEALHNHYTQKSLSLS PGK* |
| 26 | human ActRIIB soluble (extracellular), processed polypeptide sequence (amino acids 20-134 of SEQ ID NO: 14) with L79D mutation fused to an Fc domain and with TPA leader sequence | MDAMKRGLCCVLLLCGAVFVSPGASGRGEAETR ECIYYNANWELERTNQSGLERCEGEQDKRLHCY ASWRNSSGTIELVKKGCWDDDFNCYDRQECVAT EENPQVYFCCCEGNFCNERFTHLPEAGGPEVTYEP PPTAPTGGGTHTCPPCPAPELLGGPSVFLFPPKPKD TLMISRTPEVTCVVVDVSHEDPEVKFNWYVDGVE VHNAKTKPREEQYNSTYRVVSVLTVLHQDWLNG KEYKCKVSNKALPAPIEKTISKAKGQPREPQVYTL PPSREEMTKNQVSLTCLVKGFYPSDIAVEWESNG QPENNYKTTPPVLDSDGSFFLYSKLTVDKSRWQQ GNVFSCSVMHEALHNHYTQKSLSLSPGK* |
| 27 | human ActRIIB soluble (extracellular), processed polypeptide sequence (amino acids 20-134 of SEQ ID NO:3) with L79D mutation fused to an Fc domain and with TPA leader sequence | MDAMKRGLCCVLLLCGAVFVSPGASGRGEAETR ECIYYNANWELERTNQSGLERCEGEQDKRLHCY ASWANSSGTIELVKKGCWDDDFNCYDRQECVAT EENPQVYFCCCEGNFCNERFTHLPEAGGPEVTYEP PPTAPTGGGTHTCPPCPAPELLGGPSVFLFPPKPKD TLMISRTPEVTCVVVDVSHEDPEVKFNWYVDGVE VHNAKTKPREEQYNSTYRVVSVLTVLHQDWLNG KEYKCKVSNKALPAPIEKTISKAKGQPREPQVYTL PPSREEMTKNQVSLTCLVKGFYPSDIAVEWESNG QPENNYKTTPPVLDSDGSFFLYSKLTVDKSRWQQ GNVFSCSVMHEALHNHYTQKSLSLSPGK* |
| 28 | human ActRIIB soluble (extracellular), processed polypeptide sequence having a variant C-terminal sequence (disclosed in WO2007/053775) | GRGEAETRECIYYNANWELERTNQSGLERCEGEQ DKRLHCYASWRNSSGTIELVKKGCWLDDFNCYD RQECVATEENPQVYFCCCEGNFCNERFTHLPEAG GPEGPWASTTIPSGGPEATAAAGDQGSGALWLCL EGPAHE |
| 29 | human ActRIIB soluble | GRGEAETRECIYYNANWELERTNQSGLERCEGEQ |
| | (extracellular), processed polypeptide sequence having a variant C-terminal sequence (disclosed in WO2007/053775) having an L79D mutation | DKRLHCYASWRNSSGTIELVKKGCWDDDFNCYD RQECVATEENPQVYFCCCEGNFCNERFTHLPEAG GPEGPWASTTIPSGGPEATAAAGDQGSGALWLCL EGPAHE |
| 30 | human ActRIIB soluble (extracellular), processed polypeptide sequence having a variant C-terminal sequence (disclosed in WO2007/053775) having an L79D mutation fused to an Fc domain with a TGGG linker | GRGEAETRECIYYNANWELERTNQSGLERCEGEQ DKRLHCYASWRNSSGTIELVKKGCWDDDFNCYD RQECVATEENPQVYFCCCEGNFCNERFTHLPEAG GPEGPWASTTIPSGGPEATAAAGDQGSGALWLCL EGPAHETGGGTHTCPPCPAPELLGGPSVFLFPPKP KDTLMISRTPEVTCVVVDVSHEDPEVKFNWYVD GVEVHNAKTKPREEQYNSTYRVVSVLTVLHQDW LNGKEYKCKVSNKALPAPIEKTISKAKGQPREPQV YTLPPSREEMTKNQVSLTCLVKGFYPSDIAVEWES NGQPENNYKTTPPVLDSDGSFFLYSKLTVDKSRW QQGNVFSCSVMHEALHNHYTQKSLSLSPGK* |
| 31 | Nucleic Acid Sequence Encoding SEQ ID NO:10 | ATGGATGCAATGAAGAGAGGGCTCTGCTGTGTG CTGCTGCTGTGTGGAGCAGTCTTCGTTTCGCCC GGCGCCGCCGAAACCCGCGAATGTATTTATTAC AATGCTAATTGGGAACTCGAACGGACGAACCA ATCCGGGCTCGAACGGTGTGAGGGGGAACAGG ATAAACGCCTCCATTGCTATGCGTCGTGGAGGA ACTCCTCCGGGACGATTGAACTGGTCAAGAAAG GGTGCTGGGACGACGATTTCAATTGTTATGACC GCCAGGAATGTGTCGCGACCGAAGAGAATCCG CAGGTCTATTTCTGTTGTTGCGAGGGGAATTTCT GTAATGAACGGTTTACCCACCTCCCCGAAGCCG GCGGGCCCGAGGTGACCTATGAACCCCCGCCC ACCGGTGGTGGAACTCACACATGCCCACCGTGC CCAGCACCTGAACTCCTGGGGGGACCGTCAGTC TTCCTCTTCCCCCCAAAACCCAAGGACACCCTC ATGATCTCCCGGACCCCTGAGGTCACATGCGTG GTGGTGGACGTGAGCCACGAAGACCCTGAGGT CAAGTTCAACTGGTACGTGGACGGCGTGGAGGT GCATAATGCCAAGACAAAGCCGCGGGAGGAGC AGTACAACAGCACGTACCGTGTGGTCAGCGTCC TCACCGTCCTGCACCAGGACTGGCTGAATGGCA AGGAGTACAAGTGCAAGGTCTCCAACAAAGCC CTCCCAGCCCCCATCGAGAAAACCATCTCCAAA GCCAAAGGGCAGCCCCGAGAACCACAGGTGTA CACCCTGCCCCCATCCCGGGAGGAGATGACCA |
| | | AGAACCAGGTCAGCCTGACCTGCCTGGTCAAAG GCTTCTATCCCAGCGACATCGCCGTGGAGTGGG AGAGCAATGGGCAGCCGGAGAACAACTACAAG ACCACGCCTCCCGTGCTGGACTCCGACGGCTCC TTCTTCCTCTATAGCAAGCTCACCGTGGACAAG AGCAGGTGGCAGCAGGGGAACGTCTTCTCATGC TCCGTGATGCATGAGGCTCTGCACAACCACTAC ACGCAGAAGAGCCTCTCCCTGTCCCCGGGTAAA TGA |
| 32 | fusion protein comprising a soluble extracellular domain of ActRIIB (R64; SEQ ID NO: 15) fused to an Fc domain | SGRGEAETRECIYYNANWELERTNQSGLERCEGE QDKRLHCYASWRNSSGTIELVKKGCWLDDFNCY DRQECVATEENPQVYFCCCEGNFCNERFTHLPEA GGPEVTYEPPPTAPTGGGTHTCPPCPAPELLGGPS VFLFPPKPKDTLMISRTPEVTCVVVDVSHEDPEVK FNWYVDGVEVHNAKTKPREEQYNSTYRVVSVLT VLHQDWLNGKEYKCKVSNKALPVPIEKTISKAKG QPREPQVYTLPPSREEMTKNQVSLTCLVKGFYPS DIAVEWESNGQPENNYKTTPPVLDSDGSFFLYSKL TVDKSRWQQGNVFSCSVMHEALHNHYTQKSLSL SPGK |
| 33 | fusion protein comprising a soluble extracellular domain of ActRIIB (R64) with the C-terminal 15 amino acids deleted (SEQ ID NO: 16) fused to an Fc domain | SGRGEAETRECIYYNANWELERTNQSGLERCEGE QDKRLHCYASWRNSSGTIELVKKGCWLDDFNCY DRQECVATEENPQVYFCCCEGNFCNERFTHLPEA GGGTHTCPPCPAPELLGGPSVFLFPPKPKDTLMIS RTPEVTCVVVDVSHEDPEVKFNWYVDGVEVHNA KTKPREEQYNSTYRVVSVLTVLHQDWLNGKEYK CKVSNKALPVPIEKTISKAKGQPREPQVYTLPPSR EEMTKNQVSLTCLVKGFYPSDIAVEWESNGQPEN NYKTTPPVLDSDGSFFLYSKLTVDKSRWQQGNVF SCSVMHEALHNHYTQKSLSLSPGK |
| 34 | Extracellular domain of human ActRIIA fused to a human Fc domain | ILGRSETQECLFFNANWEKDRTNQTGVEPCYGDK DKRRHCFATWKNISGSIEIVKQGCWLDDINCYDR TDCVEKKDSPEVYFCCCEGNMCNEKFSYFPEMEV TQPTSNPVTPKPPTGGGTHTCPPCPAPELLGGPSV FLFPPKPKDTLMISRTPEVTCVVVDVSHEDPEVKF NWYVDGVEVHNAKTKPREEQYNSTYRVVSVLTV LHQDWLNGKEYKCKVSNKALPVPIEKTISKAKGQ PREPQVYTLPPSREEMTKNQVSLTCLVKGFYPSDI AVEWESNGQPENNYKTTPPVLDSDGSFFLYSKLT VDKSRWQQGNVFSCSVMHEALHNHYTQKSLSLS PGK |

## Claims

1. An ActRIIB signaling inhibitor for use in a method for treating anemia in a subject having myeloproliferative neoplasm-associated myelofibrosis, wherein the subject has previously been treated with ruxolitinib, wherein the method comprises administering to the subject a pharmaceutically effective amount of the ActRIIB signaling inhibitor, wherein the ActRIIB signaling inhibitor is a fusion protein comprising: (a) a fragment of the extracellular domain of ActRIIB, wherein the fragment comprises the amino acid sequence of SEQ ID NO: 9; (b) a linker; and (c) an Fc of an IgG.

2. The ActRIIB signaling inhibitor for use of claim 1, wherein the treating reduces or palliates one or more symptoms of the myeloproliferative neoplasm-associated myelofibrosis in the subject.

3. The ActRIIB signaling inhibitor for use of claim 2, wherein the symptom is one or more of fatigue, night sweats, itchiness, abdominal discomfort, pain under the ribs on the left side, early satiety, or bone pain.

4. The ActRIIB signaling inhibitor for use of any one of claims 1 to 3, wherein the subject has been on a stable dose of ruxolitinib for at least 112 days immediately prior to the administering to the subject the ActRIIB signaling inhibitor.

5. The ActRIIB signaling inhibitor for use of any one of claims 1 to 4, wherein the myeloproliferative neoplasm-associated myelofibrosis is
(i) primary myelofibrosis;
(ii) post-polycythemia vera myelofibrosis; or
(iii) post-essential thrombocythemia myelofibrosis.

6. The ActRIIB signaling inhibitor for use of any one of claims 1 to 5, wherein the subject is:
(i) red blood cell transfusion-independent; or
(ii) red blood cell transfusion-dependent.

7. The ActRIIB signaling inhibitor for use of claim 6, wherein:
(i) the subject is red blood cell transfusion-independent if the subject has received 0 red blood cell units during a period of time of 84 days prior to administration of the ActRIIB signaling inhibitor to the subject; or
(ii) the subject is red blood cell transfusion dependent if the subject has received an average red blood cell transfusion frequency of 2 to 4 red blood cell units per 28 days during a period of time of at least 84 days prior to administration of the ActRIIB signaling inhibitor to the subject.

8. The ActRIIB signaling inhibitor for use of any one of claims 1 to 7, wherein the pharmaceutically effective amount is 0.33 mg/kg, 0.45 mg/kg, 0.6 mg/kg, 0.8 mg/kg, 1 mg/kg, 1.33 mg/kg, 1.75 mg/kg, or 2.0 mg/kg, optionally wherein the pharmaceutically effective amount is 1.0 mg/kg.

9. The ActRIIB signaling inhibitor for use of any one of claims 1 to 8, wherein the ActRIIB signaling inhibitor is administered to the subject:
(i) once every 21 days, once every 28 days, or once every 48 days;
(ii) intravenously or subcutaneously; or
(iii) subcutaneously once every 21 days.

10. The ActRIIB signaling inhibitor for use of any one of claims 1 to 9, wherein the IgG is an IgG1.

11. The ActRIIB signaling inhibitor for use of any one of claims 1 to 9, wherein the ActRIIB signaling inhibitor is
(i) a polypeptide comprising the amino acid sequence of SEQ ID NO:11;
(ii) a polypeptide comprising an amino acid sequence consisting of SEQ ID NO:11; or
(iii) a polypeptide consisting of the amino acid sequence set forth in SEQ ID NO:11.

12. The ActRIIB signaling inhibitor for use of any one of claims 1 to 11, wherein the subject is a human.

13. The ActRIIB signaling inhibitor for use of any one of claims 1 to 7, wherein the method comprises administering to the subject a dose of 0.33 mg/kg, 0.45 mg/kg, 0.6 mg/kg, 0.8 mg/kg, 1 mg/kg, 1.33 mg/kg, 1.75 mg/kg, or 2.0 mg/kg of the ActRIIB signaling inhibitor, wherein the ActRIIB signaling inhibitor is administered to the subject subcutaneously once every 21 days, and wherein the ActRIIB signaling inhibitor:
(i) comprises the amino acid sequence of SEQ ID NO:11;
(ii) comprises an amino acid sequence consisting of the amino acid sequence of SEQ ID NO:11; or
(iii) consists of the amino acid sequence of SEQ ID NO:11.

14. The ActRIIB signaling inhibitor for use of any one of claims 1 to 13, wherein the ActRIIB signaling inhibitor is formulated with a pharmaceutically acceptable carrier as a pharmaceutical composition.

15. The ActRIIB signaling inhibitor for use of any one of claims 1 to 14, wherein the fragment consists of the amino acid sequence of SEQ ID NO: 9.

## Patentansprüche

1. Inhibitor der ActRIIB-Signalgebung zur Verwendung bei einem Verfahren zur Behandlung von Anämie bei einem Individuum, das an Myelofibrose in Verbindung mit myeloproliferativen Neoplasien leidet, wobei das Individuum zuvor mit Ruxolitinib behandelt wurde, wobei das Verfahren Verabreichen einer pharmazeutisch wirksamen Menge des Inhibitors der ActRIIB-Signalgebung an das Individuum umfasst, wobei es sich bei dem Inhibitor der ActRIIB-Signalgebung um ein Fusionsprotein handelt, das Folgendes umfasst: (a) ein Fragment der extrazellulären Domäne von ActRIIB, wobei das Fragment die Aminosäuresequenz unter SEQ ID NO: 9 umfasst; (b) einen Linker; und (c) ein Fc eines IgG.

2. Inhibitor der ActRIIB-Signalgebung zur Verwendung nach Anspruch 1, wobei durch die Behandlung ein oder mehrere Symptome der Myelofibrose in Verbindung mit myeloproliferativen Neoplasien bei dem Individuum reduziert oder gelindert werden.

3. Inhibitor der ActRIIB-Signalgebung zur Verwendung nach Anspruch 2, wobei es sich bei dem Symptom um eines oder mehrere von Müdigkeit, nächtlichen Schweißausbrüchen, Juckreiz, Bauchbeschwerden, Schmerzen unter den Rippen auf der linken Seite, frühzeitiger Sättigung oder Knochenschmerzen handelt.

4. Inhibitor der ActRIIB-Signalgebung zur Verwendung nach einem der Ansprüche 1 bis 3, wobei das Individuum unmittelbar vor dem Verabreichen des Inhibitors der ActRIIB-Signalgebung an das Individuum über wenigstens 112 Tage eine stabile Dosis von Ruxolitinib erhalten hat.

5. Inhibitor der ActRIIB-Signalgebung zur Verwendung nach einem der Ansprüche 1 bis 4, wobei es sich bei der Myelofibrose in Verbindung mit myeloproliferativen Neoplasien um
(i) primäre Myelofibrose,
(ii) Myelofibrose nach Polycythaemia vera oder
(iii) Myelofibrose nach essentieller Thrombozythämie handelt.

6. Inhibitor der ActRIIB-Signalgebung zur Verwendung nach einem der Ansprüche 1 bis 5, wobei das Individuum:
(i) nicht von einer Transfusion roter Blutkörperchen abhängig ist; oder
(ii) von einer Transfusion roter Blutkörperchen abhängig ist.

7. Inhibitor der ActRIIB-Signalgebung zur Verwendung nach Anspruch 6, wobei:
(i) das Individuum nicht von einer Transfusion roter Blutkörperchen abhängig ist, falls das Individuum 0 Rote-Blutkörperchen-Einheiten während eines Zeitraums von 84 Tagen vor Verabreichung des Inhibitors der ActRIIB-Signalgebung an das Individuum erhalten hat; oder
(ii) das Individuum von einer Transfusion roter Blutkörperchen abhängig ist, falls das Individuum eine Transfusion roter Blutkörperchen mit einer mittleren Häufigkeit von 2 bis 4 Rote-Blutkörperchen-Einheiten alle 28 Tage während eines Zeitraums von wenigstens 84 Tagen vor Verabreichung des Inhibitors der ActRIIB-Signalgebung an das Individuum erhalten hat.

8. Inhibitor der ActRIIB-Signalgebung zur Verwendung nach einem der Ansprüche 1 bis 7, wobei die pharmazeutisch wirksame Menge 0,33 mg/kg, 0,45 mg/kg, 0,6 mg/kg, 0,8 mg/kg, 1 mg/kg, 1,33 mg/kg, 1,75 mg/kg oder 2,0 mg/kg beträgt, gegebenenfalls wobei die pharmazeutisch wirksame Menge 1,0 mg/kg beträgt.

9. Inhibitor der ActRIIB-Signalgebung zur Verwendung nach einem der Ansprüche 1 bis 8, wobei dem Individuum der Inhibitor der ActRIIB-Signalgebung:
(i) einmal alle 21 Tage, einmal alle 28 Tage oder einmal alle 48 Tage verabreicht wird;
(ii) intravenös oder subkutan verabreicht wird; oder
(iii) alle 21 Tage subkutan verabreicht wird.

10. Inhibitor der ActRIIB-Signalgebung zur Verwendung nach einem der Ansprüche 1 bis 9, wobei es sich bei dem IgG um ein IgG1 handelt.

11. Inhibitor der ActRIIB-Signalgebung zur Verwendung nach einem der Ansprüche 1 bis 9, wobei es sich bei dem Inhibitor der ActRIIB-Signalgebung um
(i) ein Polypeptid, das die Aminosäuresequenz unter SEQ ID NO:11 umfasst,
(ii) ein Polypeptid, das eine Aminosäuresequenz bestehend aus SEQ ID NO:11 umfasst, oder
(iii) ein Polypeptid, das aus der Aminosäuresequenz gemäß SEQ ID NO:11 besteht, handelt.

12. Inhibitor der ActRIIB-Signalgebung zur Verwendung nach einem der Ansprüche 1 bis 11, wobei es sich bei dem Individuum um einen Menschen handelt.

13. Inhibitor der ActRIIB-Signalgebung zur Verwendung nach einem der Ansprüche 1 bis 7, wobei das Verfahren Verabreichen einer Dosis von 0,33 mg/kg, 0,45 mg/kg, 0,6 mg/kg, 0,8 mg/kg, 1 mg/kg, 1,33 mg/kg, 1,75 mg/kg oder 2,0 mg/kg des Inhibitors der ActRIIB-Signalgebung an das Individuum umfasst, wobei dem Individuum der Inhibitor der ActRIIB-Signalgebung einmal alle 21 Tage subkutan verabreicht wird und wobei der Inhibitor der ActRIIB-Signalgebung:
(i) die Aminosäuresequenz unter SEQ ID NO:11 umfasst;
(ii) eine aus der Aminosäuresequenz unter SEQ ID NO:11 bestehende Aminosäuresequenz umfasst; oder
(iii) aus der Aminosäuresequenz unter SEQ ID NO:11 besteht.

14. Inhibitor der ActRIIB-Signalgebung zur Verwendung nach einem der Ansprüche 1 bis 13, wobei der Inhibitor der ActRIIB-Signalgebung mit einem pharmazeutisch unbedenklichen Träger als eine pharmazeutische Zusammensetzung formuliert ist.

15. Inhibitor der ActRIIB-Signalgebung zur Verwendung nach einem der Ansprüche 1 bis 14, wobei das Fragment aus der Aminosäuresequenz unter SEQ ID NO: 9 besteht.

## Revendications

1. Inhibiteur de signalisation d'ActRIIB pour une utilisation dans un procédé pour le traitement de l'anémie chez un sujet atteint d'une myélofibrose associée à un néoplasme myéloprolifératif, le sujet ayant été précédemment traité par du ruxolitinib, le procédé comprenant une administration au sujet d'une quantité pharmaceutiquement efficace de l'inhibiteur de signalisation d'ActRIIB, l'inhibiteur de signalisation d'ActRIIB étant une protéine de fusion comprenant : (a) un fragment du domaine extracellulaire d'ActRIIB, le fragment comprenant la séquence d'acides aminés de la SEQ ID NO: 9 ; (b) un lieur ; et (c) un Fc d'une IgG.

2. Inhibiteur de signalisation d'ActRIIB pour une utilisation selon la revendication 1, le traitement réduisant ou palliant un ou plusieurs symptômes de la myélofibrose associée à un néoplasme myéloprolifératif chez le sujet.

3. Inhibiteur de signalisation d'ActRIIB pour une utilisation selon la revendication 2, le symptôme étant l'un ou plusieurs parmi la fatigue, des suées nocturnes, des démangeaisons, un inconfort abdominal, une douleur sous les côtes du côté gauche, une satiété précoce ou une douleur osseuse.

4. Inhibiteur de signalisation d'ActRIIB pour une utilisation selon l'une quelconque des revendications 1 à 3, le sujet ayant été traité par une dose stable de ruxolitinib pendant au moins 112 jours immédiatement avant l'administration au sujet de l'inhibiteur de signalisation d'ActRIIB.

5. Inhibiteur de signalisation d'ActRIIB pour une utilisation selon l'une quelconque des revendications 1 à 4, la myélofibrose associée à un néoplasme myéloprolifératif étant
(i) une myélofibrose primaire ;
(ii) une myélofibrose post-polyglobulie essentielle ; ou
(iii) une myélofibrose post-thrombocytémie essentielle.

6. Inhibiteur de signalisation d'ActRIIB pour une utilisation selon l'une quelconque des revendications 1 à 5, le sujet étant :
(i) indépendant d'une transfusion de globules rouges ; ou
(ii) dépendant d'une transfusion de globules rouges.

7. Inhibiteur de signalisation d'ActRIIB pour une utilisation selon la revendication 6,
(i) le sujet étant indépendant d'une transfusion de globules rouges si le sujet a reçu 0 unité de globules rouges pendant une période de temps de 84 jours avant administration de l'inhibiteur de signalisation d'ActRIIB au sujet ; ou
(ii) le sujet étant dépendant d'une transfusion de globules rouges si le sujet a reçu une fréquence moyenne de transfusion de globules rouges de 2 à 4 unités de globules rouges par 28 jours pendant une période de temps d'au moins 84 jours avant administration de l'inhibiteur de signalisation d'ActRIIB au sujet.

8. Inhibiteur de signalisation d'ActRIIB pour une utilisation selon l'une quelconque des revendications 1 à 7, la quantité pharmaceutiquement efficace étant de 0,33 mg/kg, 0,45 mg/kg, 0,6 mg/kg, 0,8 mg/kg, 1 mg/kg, 1,33 mg/kg, 1,75 mg/kg ou 2,0 mg/kg, éventuellement la quantité pharmaceutiquement efficace étant de 1,0 mg/kg.

9. Inhibiteur de signalisation d'ActRIIB pour une utilisation selon l'une quelconque des revendications 1 à 8, l'inhibiteur de signalisation d'ActRIIB étant administré au sujet :
(i) une fois tous les 21 jours, une fois tous les 28 jours ou une fois tous les 48 jours ;
(ii) de manière intraveineuse ou de manière sous-cutanée ; ou
(iii) de manière sous-cutanée une fois tous les 21 jours.

10. Inhibiteur de signalisation d'ActRIIB pour une utilisation selon l'une quelconque des revendications 1 à 9, l'IgG étant une IgG1.

11. Inhibiteur de signalisation d'ActRIIB pour une utilisation selon l'une quelconque des revendications 1 à 9, l'inhibiteur de signalisation d'ActRIIB étant
(i) un polypeptide comprenant la séquence d'acides aminés de la SEQ ID NO: 11 ;
(ii) un polypeptide comprenant une séquence d'acides aminés constituée de la SEQ ID NO: 11 ; ou
(iii) un polypeptide constitué de la séquence d'acides aminés indiquée dans la SEQ ID NO: 11.

12. Inhibiteur de signalisation d'ActRIIB pour une utilisation selon l'une quelconque des revendications 1 à 11, le sujet étant un humain.

13. Inhibiteur de signalisation d'ActRIIB pour une utilisation selon l'une quelconque des revendications 1 à 7, le procédé comprenant une administration au sujet d'une dose de 0,33 mg/kg, 0,45 mg/kg, 0,6 mg/kg, 0,8 mg/kg, 1 mg/kg, 1,33 mg/kg, 1,75 mg/kg ou 2,0 mg/kg de l'inhibiteur de signalisation d'ActRIIB, l'inhibiteur de signalisation d'ActRIIB étant administré au sujet de manière sous-cutanée une fois tous les 21 jours, et l'inhibiteur de signalisation d'ActRIIB :
(i) comprenant la séquence d'acides aminés de la SEQ ID NO: 11 ;
(ii) comprenant une séquence d'acides aminés constituée de la séquence d'acides aminés de la SEQ ID NO: 11 ; ou
(iii) étant constitué de la séquence d'acides aminés de la SEQ ID NO: 11.

14. Inhibiteur de signalisation d'ActRIIB pour une utilisation selon l'une quelconque des revendications 1 à 13, l'inhibiteur de signalisation d'ActRIIB étant formulé avec un support pharmaceutiquement acceptable comme une composition pharmaceutique.

15. Inhibiteur de signalisation d'ActRIIB pour une utilisation selon l'une quelconque des revendications 1 à 14, le fragment étant constitué de la séquence d'acides aminés de la SEQ ID NO: 9.
